(19) Europäisches Patentamt
European Patent Office
Office européen des brevets

(11) EP 3 344 225 B1

(12) # EUROPEAN PATENT SPECIFICATION

(45) Date of publication and mention of the grant of the patent:
**17.06.2020 Bulletin 2020/25**

(21) Application number: **15759765.9**

(22) Date of filing: **03.09.2015**

(51) Int Cl.:
***A61K 8/37*** (2006.01) ***A61Q 1/08*** (2006.01)
***A61Q 1/10*** (2006.01) ***A61K 8/04*** (2006.01)
***A61K 8/11*** (2006.01) ***A61K 8/20*** (2006.01)

(86) International application number:
**PCT/EP2015/070177**

(87) International publication number:
**WO 2017/036540 (09.03.2017 Gazette 2017/10)**

(54) **AQUEOUS GEL FOR CARING FOR AND/OR MAKING UP KERATIN MATERIALS COMPRISING MICROCAPSULES ENCAPSULATING AN OILY DISPERSION OF AT LEAST ONE REFLECTIVE AGENT**

WÄSSRIGES GEL ZUR PFLEGE UND/ODER KOSMETISCHEN BEHANDLUNG VON KERATINMATERIALIEN MIT MIKROKAPSELN ZUR VERKAPSELUNG EINER ÖLIGEN DISPERSION VON MINDESTENS EINEM REFLEKTIERENDEN MITTEL

GEL AQUEUX POUR LE SOIN ET/OU LE MAQUILLAGE DE MATIÈRES KÉRATINIQUES COMPRENANT DES MICROCAPSULES ENCAPSULANT UNE DISPERSION HUILEUSE D'AU MOINS UN AGENT RÉFLÉCHISSANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**11.07.2018 Bulletin 2018/28**

(73) Proprietor: **L'OREAL**
**75008 Paris (FR)**

(72) Inventors:
- **RICARD, Audrey**
**94210 La Varenne Saint Hilaire (FR)**
- **GOLDSTEIN, Danny**
**12235 Northern Galilee (IL)**

(74) Representative: **L'Oreal**
**Service D.I.P.I.**
**9, rue Pierre Dreyfus**
**92110 Clichy (FR)**

(56) References cited:
**US-A1- 2002 159 957** **US-A1- 2005 048 014**
**US-A1- 2008 274 148** **US-A1- 2012 269 752**
**US-A1- 2014 356 403** **US-B1- 6 932 984**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 344 225 B1

## Description

The present invention concerns a composition as laid out in the claims which is in the form of aqueous gel for caring for and/or making up keratin materials comprising, in a physiologically acceptable medium,

a) one aqueous phase ; and
b) at least one hydrophilic gelling agent, and
c) at least microcapsules having a mean size from 10 μm to 400 μm and comprising:

- an inner core comprising at least a dispersion of bismuth oxychloride in at least one oil chosen from 2-ethylhexyl hydroxystearate, ethylhexyl ethylhexanoate, castor oil, or any combination thereof, and more particularly is 2-ethylhexyl hydroxystearate and
- at least one outer shell formed of a wall-forming polymeric material surrounding the said core, the said outer shell comprising

i) at least one wall-forming polymer, and
ii) optionally at least one plasticizer and/or at least one opaque substance and/:or at least one fatty acid salt.

The present invention concerns also a cosmetic process for caring for and/or making up keratinic materials, comprising application on said keratinic materials in particular on the skin of a composition as above defined.

Consumers are looking for new cosmetic products to improve the appearance of keratin materials and especially the skin, in particular the surface appearance (visible and/or tactile) and/or skin tone including brightness or a light effect to the skin with a natural glow and advantageously a healthy glow.

By 'light' or 'light effect' is meant according to the invention the reflection characteristic of light, diffuse reflection and continues on the skin. Indeed, the skin naturally reflects part of the incident light. The "light effect" according to the invention can increase this refelction, which provides the makeup rendering brighter, more sparkle.

For 'healthy glow' a natural skin coloring means, with an improvement of dull complexion (desaturating or chromatic effect and anti-dull completion).

The use of reflective agents as bismuth oxychloride (CI 77163) in cosmetic formulations is well-known and notably described in WO 2004/041234 US703361 and US 2014/356403.

The use of reflective agents as bismuth oxychloride (CI 77163) as a powder or agglomerates is known in foundations as a charge to bring some sensory (soft). This compound can also bring a punctual and discontinuous satin effect, which in products such as fluids, or even compact powders can be perceived as a pearly shine. However, because of its capacity to absorb moisture and oils, bismuth oxychloride under powder form or agglomerate has a tendency to render white and to affect the final visual rendering of the product after application on the skin and the sensory properties such as freshness. In addition, the powder form requires industrial constraints of grinding to a finer particle size in order to obtain more bright, more sparkle.

To remedy these problems of whitening of final visual rendering and of particle size industrial constraint, it has been proposed to use bismuth oxychloride (CI 77163) as a bismuth oxychloride dispersion in a polar oil as for instance, in U.S. Patent Application having Publication No. 2012/0269752.

However, this oily dispersion used in aqueous gels as a tendency to modify and alter the rheology and the gelly texture by the presence of the oil, to reduce the lightness, to alter the sensorial properties during and/or after application as the play time, the freshness, smoothness, a non greasy feeling, the homogeneity of the make-up.

In the prior art, in particular in the documents US2010095868, US7622132, WO09079135, EP1518903B1, it has been proposed to use a reflective agent in microcapsule as nacres pigments containing bismuth oxychloride or bismuth oxychloride.

In cosmetic formulations it is generally highly desirable to retain a cosmetically active agent that provides a visual effect within capsules before application thereof. Encapsulation of such agent is thought for in order to maintain a long term visual effect of the cosmetic formulation; to protect the encapsulated agent from interacting with other agents in the formulation; to mask the visual effect of the active agent before application; to maintain the stability of the active agent in a formulation and/or to release the encapsulated active agent only upon application. The effectiveness of protection/masking by single-layer microencapsulation depends on the chemical structure, molecular weight and physical properties of the microencapsulated ingredient.

Microparticles encapsulating a variety of cosmetically active agents, including colorants and/or pigments and other agents that provide a visual effect, have been described in the art.

U.S. Patent Nos. 5,320,835 and 5,382,433 disclose "activable" dormant colored particles or pigments and cosmetic formulations comprising them and further comprising a colored base phase, and colorant entrapping substrate particles dispersed in said base phase. The encapsulated colorants are said to be released into the base phase when

mechanical action is applied to the cosmetic formulation, and produce an intense shade in the color of the base phase, whereas the colorant entrapping substrate particles entrap the released colorants and produce a subtle shade in the color of the base phase. The encapsulated pigments are made by a coacervation method.

WO 98/5002 discloses similar color-sustainable base cosmetic formulations, further including volatile solvents to minimize the gritty feel of the microencapsulated material. The color obtained from the released encapsulated pigments is exactly the same as the color of the composition itself. Releasing provides renewed intensity of the original base color.

U.S. Patent No. 5,380,485 discloses colored cosmetic compositions, comprising particulate fillers coated with polymer that is combined with colorants, and their application in decorative cosmetics.

U.S. Patent Application having Publication Nos. 2005/0031558 and 2005/0276774 disclose a personal care or cosmetic composition containing microparticles comprising a shatter resistant blend of distinct colorants microencapsulated within a polymer matrix, preferably a cross-linked polymer matrix that does not allow any of the entrapped colorant to be released even under prolonged use. The matrix polymer is preferably transparent or translucent such that the blend of encapsulated colorants provides the coloring of the cosmetic product itself and of the skin upon application of the cosmetic composition. The microparticles disclosed in U.S. Patent Application having Publication No. 2005/0276774 further contain secondary particles (i.e. hydrophobic polymers different from those of the matrix polymer) that are distributed throughout the matrix.

U.S. Patent No. 4,756,906 discloses decorative cosmetic compositions containing a first colorant and microcapsules containing a solvated second colorant, different from the first colorant. Upon rupture of the microcapsules, the coloration of the encapsulated pigment is added into the composition thereby altering its color characteristics.

WO 2004/075679 discloses rigid, non-rupturable microcapsules containing a blend of at least two coloring agents and compositions comprising them, which do not change their color upon application onto the skin. The microcapsules are non-rupturable due to the use of cross-linked polymeric matrix comprising polymers that have a glass transition temperature (Tg) higher than 80 °C.

U.S. Patent No. 6,932,984 discloses single- and double-layer microcapsules and a method for microencapsulation of substances by the solvent removal method using non-chlorinated solvents. The method is based on physical processes which do not cause any change of original physical and/or chemical properties, biological activity, and safety of raw materials during the process.

U.S. Patent No. 7,838,037 discloses double-layer and/or triple-layer microcapsules, designed to rupture by a slight mechanical action such as rubbing or pressing on the skin, and thereby immediately release their encapsulated content. These microcapsules are prepared by the solvent removal method using non-chlorinated solvents. This method affords physical stability to the microcapsules, high ability to entrap the active agents, protection of the active agents inside the microcapsules, and prevention of the diffusion of the microencapsulated active agents to the external water phase in a water-based preparation.

WO 2009/138978 discloses cosmetic compositions for dermal/topical application comprising double-layer, rupturable microcapsules which contain one or more microencapsulated colorants. When applied to the skin, such compositions produce an immediate color change effect indicating the delivery to the skin of the active substances contained in said compositions.

There is thus a need to find new compositions in the form of aqueous gels based on a reflective agent which do not have the drawbacks as mentioned above.

There is also the need to use in the said compositions of a reflective agent in an appropriate encapsulated form which may efficiently mask the visual effect of the reflective agent before application and/or release the encapsulated active agent only upon application.

The applicant has surprisingly discovered that this objective can be achieved with an aqueous gel for caring for and/or making up keratin materials comprising, in a physiologically acceptable medium,

a) one aqueous phase ; and
b) at least one hydrophilic gelling agent, and
c) at least microcapsules having a mean size from 10 μm to 400 μm and comprising:

- an inner core comprising at least a dispersion of bismuth oxychloride in at least one oil chosen from 2-ethylhexylhydroxy stearate, ethylhexyl ethylhexanoate, castor oil, or any combination thereof, and more particularly is 2-ethylhexyl hydroxystearate and
- at least one outer shell formed of a wall-forming polymeric material surrounding the said core, the said outer shell comprising

  i) at least one wall-forming polymer, and
  ii) optionally at least one plasticizer and/or at least one opaque substance and/:or at least one fatty acid salt.

The aqueous gels according to the invention allow to give a high brightness and a high freshness.

The microcapsules of the invention as described below, contain at least a dispersion of a reflective agent which is bismuth oxychloride in at least one oil, and allow to encapsulate the said dispersion in high load (e.g., higher than 50 %, 60 % and even higher than 70 %, of the total weight of the microcapsule. The said microcapsules, are stable during the manufacturing and storage processes, maintain the encapsulated oily dispersion of reflective agent inside the capsules with minimal or nullified leakage, and are rupturable under mild shear forces, thus enabling an immediate release of the encapsulated agent upon application of the microcapsules to the skin. The obtained microcapsules provided herewith may further provide a masking effect of the light reflectance of the reflective agent before rupture, if desired.

This discovery is the basis of the invention.

The present invention concerns a composition under the form of aqueous gel for caring for and/or making up keratin materials comprising, in a physiologically acceptable medium,

a) one aqueous phase ; and
b) at least one hydrophilic gelling agent, and
c) at least microcapsules having a mean size from 10 μm to 400 μm and comprising:

- an inner core comprising at least a dispersion of bismuth oxychloride in at least one oil chosen from 2-ethylhexyl hydroxystearate, ethylhexyl ethylhexanoate, castor oil or any combination thereof, and more particularly is 2-ethylhexyl hydroxystearate, and
- at least one outer shell formed of a wall-forming polymeric material surrounding the said core, the said outer shell comprising

    i) at least one wall-forming polymer, and
    ii) optionally at least one plasticizer and/or at least one opaque substance and/:or at least one fatty acid salt.

The present invention concerns also a cosmetic process for caring for and/or making up keratinic materials, comprising application on said keratinic materials in particular on the skin of a composition as above defined.

## Definitions

"Physiologically acceptable medium" means any medium compatible with the keratin materials, which has a color, a smell and a pleasant feel and which does not generate unacceptable discomfort (stinging, tautness or redness) liable to put the consumer from using this composition.

In the context of the present invention, the term "keratin materials" means the skin and especially areas like the face, cheeks, hands, body, legs, around the eyes, the eyelids and the lips.

The term "aqueous gel" means a composition comprising a continuous aqueous phase containing a viscoelastic mass formed from colloidal suspensions. The viscosity of a gel of the invention is measured at 25 ° C with a Rheomat RM180 machine (moving 2 or 3) the company Rheometric Scientific and its value is generally at least 60 mobile UD 2 (Units Deviation)

## MICROCAPSULES

The microcapsules provided by the present embodiments are particles (e.g., generally spherical particles), which are generally closed structures containing at least an encapsulated (enveloped, entrapped) oily dispersion of a reflective agent, in particular bismuth oxychloride. The microcapsules generally have a core-shell structural feature, namely each microcapsule is comprised of a polymeric shell and a core that comprises at least one oily dispersion of reflective agent enveloped by the shell.

The shell of the microcapsule is typically applied as a wall-forming material and serves as a membrane for the encapsulated substance. In some embodiments, the outer shell exhibits some opacity, or otherwise a masking effect of the reflective agent, by virtue of inclusion of an opaque substance in the shell, optionally in combination with a fatty acid salt.

The outer shell may further comprise a plasticizer to control its hardness, and is designed such that the microcapsules are rupturable upon rubbing or pressing on the skin.

In some of any of the embodiments described herein, the microcapsules are single-layer microcapsules, comprising a single outer shell enveloping the inner core.

In some other embodiments, the microcapsules are double-layer, or triple-layer, or multi-layer microcapsules, comprising additional one or more layers enveloping the shell that envelopes the inner core.

A multi-layer microcapsule is featured as comprising an inner core microcapsule comprising a core which comprises an oily dispersion of bismuth oxychloride, as described herein, being enveloped by a first shell comprised of

a first wall-forming material, and at least one additional shell comprised of a second wall forming material enveloping said first shell, which can be regarded as enveloping a single-layer microcapsule as described herein (comprising the reflective agent-containing inner core and a first shell of a first wall-forming material).

Each shell in the multi-layered microcapsules is typically and independently applied as a wall-forming material (e.g., a first, second, third and so forth wall-forming materials forming the first, second, third, and so forth, outer shells, respectively), and serves as a membrane for the encapsulated substance. In some embodiments, one or more, or each, of the outer shells in the multi-layered microcapsules according to these embodiments is optionally opaque by virtue of an opaque substance comprised therein, and/or further contains a fatty acid salt, as described herein.

The microcapsules of the present embodiments, among other uses, are suitable for inclusion in topical, e.g., cosmetic, cosmeceutical and pharmaceutical (e.g., dermatological), applications. When applied to the skin, the microcapsules are capable of being ruptured upon application of shear forces such as rubbing and pressing on the skin, but they remain intact in the formulation itself before application. The microcapsules are hard enough to avoid destruction of the shells and realization of the content during production processes such as isolation/filtration, drying, sieving, etc., and/or during storage.

In some embodiments, the microcapsules encapsulating an oily dispersion of bismuth oxychloride as described herein are prepared by a solvent removal method, as described hereinunder and exemplified in the Examples section that follows.

In some embodiments, a mean size of the microcapsules as described herein is more preferably from 50 μm to 350 μm, more particularly from 50 μm to 250 μm, advantageously from 90 μm to 250 μm, more advantageously from 100 μm to 200 μm.

Herein throughout, a "mean" size means an average size of the microcapsules. The size of the microcapsules may be measured by a Laser distribution size method and particularly by measuring the values D[50] and D[90].

D50 means the size of which 50 % of the microcapsules do not exceed, and D90 means the size of which 90 % of the microcapsules do not exceed.

In some of any of the embodiments described herein, the outer shell comprises, in addition to the wall-forming material, a fatty acid salt, and an opaque substance, as described herein.

According to some of any of the embodiments of the present invention, the microcapsules described herein exhibit masking of the luminous effect of the oily dispersion of bismuth oxychloride, as reflected by a positive shift (delta) of the lightness value (L*) determined in X-rite measurements.

According to some of any of the embodiments of the invention, a microcapsule as described herein is rupturable or breakable when applied to the skin; that is, a microcapsule as described herein remains intact in a formulation containing same and during industrial processes, but readily breaks when pressed of rubbed on the skin. The non-breakability of the microcapsules before topical application thereof is routinely assessed by monitoring (e.g., using a light microscope) the ability of the microcapsules in a basic cream or lotion to sustain their size and shape when subjected to low shear mixing at e.g., 40-600 (or 80-100) rpm for 5-10 minutes at room temperature and at 40 °C. A change of less than 10 % in the microcapsule size is indicative of the non-breakability of the microcapsules upon routine industrial processes.

## THE INNER CORE:

The inner core in the microcapsules described herein comprises at least a dispersion of at least one reflective agent in at least one oil.

### a) Reflective agent(s)

As used herein, a "reflective agent" describes an agent which increases the diffuse light reflection of a substrate onto which it is applied. A reflective agent as described herein is typically intended to increase the light reflection of keratinous substrates, particularly the skin, and more particularly facial skin.

According to the invention, the reflective agent is bismuth oxychloride.

### a) Oil(s) used in the dispersion

According to the present invention, the term "oil" means a water-immiscible nonaqueous compound that is liquid at room temperature (25°C) and at atmospheric pressure (760 mmHg).

According to a preferred embodiment, the oil is a polar oil.

The term "polar oil", as used herein, refers to any oil having, at 25°C, a solubility parameter $\delta_d$ characteristic of dispersive interactions of greater than 16 and a solubility parameter $\delta_p$ characteristic of polar interactions strictly greater than 0. The solubility parameters $\delta_d$ and $\delta_p$ are defined according to the Hansen classification. For example,

these polar oils may be chosen from esters, triglycerides and ethers.

The definition and calculation of the solubility parameters in the Hansen three-dimensional solubility space are described in the paper by C.M. Hansen: "The three dimensional solubility parameters", J. Paint Technol. 39, 105 (1967).

According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{½}$.

The parameters $\delta_p$, $\delta_h$, $\delta_D$ and $\delta_a$ are expressed in $(J/cm^3)^{½}$.

The polar oils will preferably be chosen from oils having $\delta_a > 6$.

These polar oils may be of plant, mineral or synthetic origin.

The polar oils will preferably be chosen from non-volatile polar hydrocarbon-based oils.

The term "polar hydrocarbon-based oil" means a polar oil formed essentially from, or even constituted by, carbon and hydrogen atoms, and optionally oxygen and nitrogen atoms, and not containing any silicon or fluorine atoms. It may contain alcohol, ester, ether, carboxylic acid, amine and/or amide groups.

The term "non-volatile oil" means an oil that remains on the skin or the keratin fiber at room temperature and atmospheric pressure for at least several hours, and that especially has a vapour pressure of less than $10^{-3}$ mmHg (0.13 Pa).

The non-volatile polar hydrocarbon-based oil may be chosen especially from the following oils:

- hydrocarbon-based polar oils such triglycerides consisting of fatty acid esters of glycerol, in particular the fatty acids of which may have chain lengths ranging from $C_4$ to $C_{36}$, and especially from $C_{18}$ to $C_{36}$, these oils possibly being linear or branched, and saturated or unsaturated; these oils may especially be heptanoic or octanoic triglycerides, wheatgerm oil, sunflower oil, grapeseed oil, sesame seed oil (820.6 g/mol), corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passionflower oil or musk rose oil; or alternatively caprylic/capric acid triglycerides, for instance those sold by the company Stéarineries Dubois or those sold under the names Miglyol 810®, 812® and 818® by the company Dynamit Nobel;
- synthetic ethers containing from 10 to 40 carbon atoms, such as dicaprylyl ether;
- hydrocarbon-based esters of formula RCOOR' in which RCOO represents a carboxylic acid residue comprising from 2 to 40 carbon atoms, and R' represents a hydrocarbon-based chain containing from 1 to 40 carbon atoms, such as cetostearyl octanoate, isopropyl alcohol esters, such as isopropyl myristate or isopropyl palmitate, ethyl palmitate, 2-ethylhexyl palmitate, isopropyl stearate or isostearate, isostearyl isostearate, octyl stearate, 2-ethylhexylhydroxysterate, diisopropyl adipate, heptanoates, and especially isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate and palmitate, polyethylene glycol diheptanoate, propylene glycol 2-diethyl hexanoate, hexyl laurate, neopentanoic acid esters, for instance isodecyl neopentanoate, isotridecyl neopentanoate, isostearyl neopentanoate and 2-octyldodecyl neopentanoate, isononanoic acid esters, for instance isononyl isononanoate, isotridecyl isononanoate and octyl isononanoate, oleyl erucate, isopropyl lauroyl sarcosinate, diisopropyl sebacate, isocetyl stearate, isodecyl neopentanoate, isostearyl behenate, and myristyl myristate;

- fatty alcohols containing from 12 to 26 carbon atoms, for instance octyldodecanol, 2-butyloctanol, 2-hexyldecanol, 2-undecylpentadecanol and oleyl alcohol;
- higher $C_{12}$-$C_{22}$ fatty acids, such as oleic acid, linoleic acid and linolenic acid, and mixtures thereof;
- fatty acids containing from 12 to 26 carbon atoms, for instance oleic acid;
- dialkyl carbonates, the two alkyl chains possibly being identical or different, such as dicaprylyl carbonate sold under the name Cetiol CC® by Cognis; and
- aromatic esters such as tridecyl trimellitate, $C_{12}$-$C_{15}$ alcohol benzoate, 2-phenylethyl benzoate, and butyloctyl salicylate,
- hydroxylated esters such as polyglycerol-2 triisostearate,
- esters of $C_{24}$-$C_{28}$ branched fatty acids or fatty alcohols such as those described in patent application EP-A-0 955 039, and especially triisoarachidyl citrate, pentaerythrityl tetraisononanoate, glyceryl triisostearate, glyceryl tris(2-decyl)tetradecanoate, pentaerythrityl tetraisostearate, polyglyceryl-2 tetraisostearate or pentaerythrityl tetrakis(2-decyl)tetradecanoate,

- esters and polyesters of dimer diol and of monocarboxylic or dicarboxylic acid, such as esters of dimer diol and of fatty acid and esters of dimer diol and of dimer dicarboxylic acid, such as Lusplan DD-DA5® and Lusplan DD-DA7® sold by the company Nippon Fine Chemical and described in patent application US 2004-175 338,
- and mixtures thereof.

According to the invention the oil is selected from 2-ethylhexyl hydroxystearate, (or octyl hydroxystearate), ethylhexyl ethylhexanoate, castor oil, or any combination thereof, and more particularly is 2-ethylhexyl hydroxystearate, (or octyl hydroxystearate).

In a preferred embodiment for the oily dispersion of reflective agent used for making the inner core of the microcapsule, the amount of reflective agent in the dispersion ranges from 50 % to 90 % by weight, more preferably from 60 % to 80 % by weight, more particularly from 65 % to 75 % by weight of the total weight of the dispersion. The amount of the oil is therefore in the range of from 10 % to 50 % by weight, more preferably from 20 % to 40 % by weight, more particularly, from 25 % to 35 % by weight relative to the total weight of the dispersion, respectively.

In a preferred embodiment, the weight ratio of the reflective agent particles to the oil(s) ranges from 1.5/1 to 5/1, more preferably from 1.5/1 to 3/1, particularly from 2/1 to 4/1, and more particularly from 2/1 to 3/1.

According to a particular form of the invention, the oily dispersion dispersion of reflective agent is a dispersion of bismuth oxychloride in ethylhexyl hydroxystearate, more particularly a dispersion containing from 68% to 72% by weight of bismuth oxychloride in 28% to 32% by weight of 2-ethylhexyl hydroxystearate relative to the total weight of the dispersion,

Such a dispersion is particularly sold under the commercial name Biron Liquid Silver® or Timiron® Liquid Silver, by the company MERCK.

According to a preferred embodiement of the present invention, the amount of the inner core of the microcapsules constituted by the oily dispersion of reflective agent, is within a range of from 20 % to 90 %%, by weight., more preferably from 30 % to 90 %%, by weight, in particular from 40 % to 90 % by weight, more particularly from 50 % to 90 % by weight, more better from 60 % to 90%, by weight, more advantageously from 70 % to 90 %%, by weight., more advantageously from 70 % to 80 %, by weight, more particularly advantageously from 60 % to 80 %, by weight relative to the total weight of the microcapsule.

In some of any of the embodiments described herein, the microcapsule contains only one type of a reflective agent or a mixture of two or more reflective agents, either encapsulated individually, and/or one or more blends of reflective agents may be encapsulated within the inner core of the microcapsules. A person skilled in the art will know how to choose reflective agent and combinations of reflective agents to produce a desired effect on the skin.

## THE WALL-FORMING MATERIALS

### a) Wall-forming polymer

The wall-forming material forms the outer shell(s) of the microcapsules of the present embodiments, and serves as a membrane for the encapsulated substance (the reflective agent). According to embodiments of the present invention, the wall forming material forming the outer shell(s) comprises a wall-forming polymer or copolymer. In some of any of the embodiments of the present invention, one or more of the outer shells further comprise at least one opaque substance and/or at least one fatty acid salt, and may optionally further comprise at least one plasticizer.

The phrase "wall-forming polymer", which is also referred to herein as "wall-forming polymeric material" refers to a polymeric material (e.g., a polymer or copolymer) or a combination of two or more different polymeric materials, as defined herein, which form a component of the external wall or layer or shell of single-layer microcapsules, or, in the case of multi-layer microcapsules, additionally of the one or more intermediate shells between the inner core and the external (outer most) layer. In the context of single-layer microcapsules, the term "polymer shell" refers to a polymer layer comprised of the wall-forming polymer(s), which envelopes the inner core. In the context of multi-layer microcapsules, the term "polymer shell" refers to any of the polymer layers which envelopes the inner core, or which envelopes the preceding polymer layer.

In some embodiments, the wall-forming polymer is selected so as to sustain shear forces applied while being compounded in industrial processes, but, nevertheless, so as to provide microcapsule which are rupturable when applied (e.g., rubbed or pressed) on the skin.

In some embodiments, the wall-forming polymeric material comprises a polymer containing a sufficient amount of functional groups which are capable of forming hydrogen bonds.

In some embodiments, the polymeric material forming the one or more outer shells independently comprises hydrogen bond-forming functional groups featuring 4-40 weight percents of total polymer weight. Hydrogen bond-forming functional groups include, but are not limited to, functional groups which comprise one or more electron-donating atom(s) such as oxygen, sulfur and/or nitrogen.

In some embodiments, the hydrogen bond-forming groups include carboxylic acid, carboxylate, hydroxy, or any combination thereof.

In some embodiments, one or more, or each, of the wall-forming polymeric materials forming the outer shell(s) comprises a polyacrylate, a polymethacrylate, a cellulose ether or ester, or any combination thereof.

Exemplary wall-forming polymeric materials include, but are not limited to, polyacrylates, polymethacrylates, low molecular weight poly(methyl methacrylate)-co-(methacrylic acid) (e.g., 1:0.16), poly(ethyl acrylate)-co-(methyl methacrylate)-co-(trimethylammmonium-ethyl methacrylate chloride) (e.g., 1/2/0.1) (also known as Eudragit® RSPO), poly(butyl methacrylate)-co-(2-dimethylaminoethyl methacrylate)-co-(methyl methacrylate) (e.g., 1/2/1), poly(styrene)-co-(maleic anhydride), copolymer of octylacrylamide, cellulose ethers, cellulose esters, poly(ethylene glycol)-block-poly(propylene glycol)-block-poly(ethylene glycol), PLA (poly(lactic acid), PGA (poly(glycolide), PLGA (poly(lactide)-co-poly(glycolide) or any combination thereof.

Any combination of polymers and co-polymers as described herein is contemplated for a wall-forming material, as described herein.

In some embodiments, the wall-forming polymeric material of an outer shell comprises a cellulose ether or ester such as, but not limited to, methyl cellulose, ethyl cellulose, hydroxypropyl methyl cellulose, hydroxypropyl cellulose, cellulose acetate, cellulose acetate phthalate, cellulose acetate butyrate and hydroxypropyl methyl cellulose acetate phthalate. When a cellulose ether or ester is used in the polymeric material, it preferably contains 4-20 % hydroxyl groups which are free to form hydrogen bonds (e.g., hydroxyl groups which are not alkylated or acylated).

In some of any of the embodiments described herein, the wall-forming material of an outer shell comprises an acrylate/ammonium methacrylate copolymer such as, for example, Eudragit® RSPO. In some of any of the other embodiments of the present invention, the wall-forming material of an outer shell comprises a combination of the above-mentioned polymers such as, but not limited to, combinations of acrylate/ammonium methacrylate copolymer (e.g., Eudragit® RSPO) with either poly(methyl methacrylate), poly(methacrylate), poly(methyl methacrylate)-co-(methacrylic acid) or cellulose acetate.

When two polymeric materials are used as a wall-forming material, a weight ratio therebetween can range from 10/1 to 1/1, and can be, for example, 5/1, 4/1, 3/1, 2/1, or 3/2.

In some of any of the embodiments described herein, the wall forming material is or comprises poly(methyl methacrylate (PMMA).

In some of any of the embodiments described herein, the wall forming material is or comprises a poly(methyl methacrylate)-co-(methacrylic acid) (PMMA/MA).

In some of any of the embodiments described herein, the wall forming material is or comprises an acrylate/ammonium methacrylate copolymer (e.g., Eudragit® RSPO). In some of any of the embodiments described herein, the wall forming material is or comprises cellulose acetate.

The amount (weight/weight) of the wall-forming polymer(s) of the outer shell relative to the total microcapsule weight can be within a range of from 5 % to 30 % by weight, more preferably from about 5 % to 20 % by weight, particularly from about 5 % to about 15 % by weight, more particularly from about 5 % to about 10 % by weight.

In some embodiments, when the wall-forming material is a cellulose ester such as cellulose acetate, and the outer shell may not comprise a fatty acid salt, as described herein. In some such embodiments, the outer shell comprises an opaque substance, such as $TiO_2$, in an amount higher than 10 % by weight, for example, in an amount ranging from 20 % to 40 % by weight, more preferably from 30 % to 40 % by weight relative to the total weight of the microcapsule.

In embodiments when the wall-forming material is cellulose acetate, the amount of the cellulose acetate can be, for example, from 5 % to 10 % by weight, more preferably from 5 % to 8 % by weight, and in particular 5 % by weight relative to the total weight of the composition.

In embodiments related to multi-layer microcapsules, the wall-forming material in each of the outer shells in the microcapsules described herein (e.g., a first wall-forming material of the inner core, a second wall-forming material of a first outer shell enveloping the inner core, and optionally a third wall-forming material of a second outer shell enveloping the first outer shell, and so forth) can be the same or different.

**b) Opaque substance:**

The outer shell of the single-layer microcapsules described herein can be opaque, semi-opaque or non-opaque (transparent). In some embodiments, the outer shell is opaque, and thus masks the light reflectance imparted by the reflective agent.

In some embodiments, one or more of the outer shells of multi-layer microcapsules as described herein can be opaque, semi-opaque or non-opaque (transparent). In some embodiments, one or more of the outer shells (e.g., the most outer shell) is opaque, and thus masks the light reflectance imparted by the reflective agent.

In some embodiments of the present invention, opacity of the outer shell of the microcapsules is obtained by an inclusion of an opaque substance.

As used herein, an "opaque substance" is a substance which is non-transparent and blocks at least 70 % of the light passing therethrough.

Thus, an opaque outer shell blocks 70 % to 100 % of the light. Semi-opaque outer shell blocks up to 50 % of the light. Non-opaque or transparent outer shell blocks no more than 30 % of the light passing therethrough.

The terms "opacity" and "opaque" refer to herein to UV-vis light, such as, for example, daylight.

Exemplary opaque substances include, but are not limited to, $TiO_2$, zinc oxide, alumina, boron nitride, talc, mica and any combination thereof.

The total amount of opaque substance(s) in the outer shell is within a range of from 1 % to 50 % by weight, more preferably from 1 % to 40 % by weight, more particularly from 10 % to 40% by weight, relative to the total weight of the microcapsule.

In some of any of the embodiments described herein, the opaque substance is, or comprises, $TiO_2$, and in some embodiments, an amount of $TiO_2$ is within a range of from 1 % to about 30 % by weight, preferably from 10 % to 40 %, by weight, of the total weight of the microcapsule.

In some of any of the embodiments described herein, the opaque substance is, or comprises, $TiO_2$, and in some embodiments, an amount of $TiO_2$ is about 10 % by weight relative to the total weight of the microcapsule.

In some of any of the embodiments described herein, the opaque substance is, or comprises, $TiO_2$, and in some embodiments, an amount of $TiO_2$ is about 35 % by weight relative to the total weight of the microcapsule.

In some embodiments, the outer shell does not comprise an opaque substance as described herein.

**c) Fatty acid salt:**

In some of any of the embodiments described herein, an outer shell optionally comprises an opaque substance as described herein in any one of the respective embodiments, and/or alternatively, or in addition, further comprises a fatty acid salt as described herein in any one of the respective embodiments.

A fatty acid salt comprises a long hydrophobic hydrocarbon chain (e.g., of 4 to 30 carbon atoms in length) carboxylate anion (a fatty acyl) and a cation, as depicted in the following formula:

$$(R\text{-}C(=O)\text{-}O\text{-})_n M^{(n+)}$$

wherein R is a substituted or unsubstituted, liner or branched hydrocarbon chain of 4 to 30 carbon atoms, $M^+$ is a cation, preferably a metal cation, and n is an integer representing the number of fatty acyls that interact with the cation, and also represents the charge number of the cation (e.g., 1, 2, 3, etc.).

The fatty acid salts that are usable in some of any of the embodiments of the present invention may contain 1 to 3 fatty acyl chains, each chain, independently, comprising 4 to 30 or 8 to 24 carbon atoms (C8-C24) in length. Thus, the fatty acid salt can be a salt of a monovalent, divalent or trivalent metal ion or a salt of an organic cation.

A monovalent metal ion can be, for example, $Na^+$, $K^+$, $Cs^+$, $Li^+$; a divalent metal ion is selected from $Mg^{2+}$, $Ca^{2+}$, Fe (II), $Co^{2+}$, $Ni^{2+}$, $Cu^{2+}$, $Mn^{2+}$, $Cd^{2+}$, $Sr^{2+}$ or $Zn^{2+}$; a thrivalent metal ion can be, for example, Fe(III), $La^{3+}$, $Eu^{3+}$ or $Gd^{3+}$; an organic cation can be, for example, ammonium, sulfonium, phosphonium or arsonium.

The fatty acyl can be derived from fatty acids such as, but not limited to, stearic acid, arachidic acid, palmitoleic acid, oleic acid, linoleic acid, linolaidic acid, arachidonic acid, myristoleic acid and erucic acid. Other fatty acids are also contemplated.

Exemplary fatty acid salts include, but are not limited to, magnesium stearate, magnesium oleate, calcium stearate, calcium linoleate, sodium stearate, magnesium arachidnoate, magnesium palmitate, magnesium linoleate, calcium arachidonoate, calcium myristoleate, sodium linoleate, calcium linoleate, sodium stearate, potassium stearate, sodium laurate, sodium myristate, sodium palmitate, potassium laurate, potassium myristate, potassium palmitate, calcium laurate, calcium myristate, calcium palmitate, zinc laurate, zinc myristate, zinc palmitate, zinc stearate, magnesium laurate, and magnesium myristate.

In a preferred embodiment, the fatty acid salt is magnesium stearate.

The fatty acid salt is usually in an amount within a range of from 0.05 % to 5 % by weight, more preferably from 0.1 % to 4.5 % by weight, particularly from 0.2 % to 4 % by weight, more particularly from 0.5 % to 4 %, advantageously from 0.5 % to 3.0 % by weight, more advantageously from 0.75 % to 3.0 % by weight, particularly more advantageously from 1.0 % to 3.0 % by weight, more better from 1.0 % to 2.0 % by weight, and in particular is 1.0 %, by weight relative to the total microcapsule's weight.

Without being bound by any particular theory, it is assumed that the cation of the fatty acid salt attracts the particles of an opaque substance and optionally the free carboxylic and/or hydroxyl groups of the wall-forming polymer, resulting in a better adhesion of both the opaque substance and the polymeric material to the inner core, thereby providing efficient masking of the oily dispersion of oxychloride bismuth present in the inner core.

Fatty acid salts may be used in the preparation of single-layer microcapsules while being added to the organic

phase together with the encapsulated material, and the wall-forming polymer, with or without the opaque substance. Upon contacting the organic phase with an aqueous phase, the fatty chains will spontaneously wrap around the encapsulated substance and their polar/ionic heads will interact with the oppositely charged opaque substance as well as with oppositely charged groups on the polymer, thereby enhancing the formation of an opaque polymeric envelope surrounding a core comprising the encapsulated material.

**d) Plasticizer:**

In some embodiments of any of the embodiments of the present invention, an outer shell of the microcapsules further comprises a plasticizer.

Herein and in the art, a "plasticizer" describes a substance which increases the plasticity or fluidity of a composition. In the context of the present embodiments, a plasticizer is added to the wall-forming material in order to control the physical properties and level of elasticity of the microcapsule's outer shells.

Exemplary plasticizers include, but are not limited to, triethyl citrate, tricaprylin, trilaurin, tripalmitin, triacetin, acetyltriethyl citrate, paraffin oil, and any combination thereof. In exemplary embodiments, the plasticizer is triethyl citrate.

The amount of the plasticizer can be within a range of from 0.5 % to about 30 % by weight, preferably from 0.5 % to 20 % by weight, more preferably from 1.0 % to 20 % by weight, particularly from 5 % to 15 % by weight, more particularly from 5 % to 10 % by weight, advantageously is 10 % by weight relative to the total weight of the microcapsule.

**Exemplary compositions of microcapsules:**

In a most preferred embodiment of the present invention, the microcapsules as described herein comprise, as the inner core, bismuth oxychloride dispersed in 2-ethylhexyl hydroxystearate. In some of these embodiments, the amount of the inner core is at least 50 % by weight, more preferably from 60 to 80% by weight (for example, 60 %, or 70 %, or 79 %, or 80 %) relative to the total weight of the microcapsule.

In particular, the microcapsules are single-layer microcapsules, and the outer shell comprises magnesium stearate in an amount within a range of from 1.0 % to 2.0 % by weight, and $TiO_2$ in an amount within a range of from 5 % to 15 %, by weight relative to the total weight of the microcapsule.

In some of these embodiments, the amount of the wall-forming polymer(s) ranges from 5 % to 15 % by weight relative to the total weight of the microcapsule.

In some of these embodiments, the wall-forming polymer is selected from a poly (methyl methacrylate) or a copolymer of methyl methacrylic acid and acrylic acid or acrylate/ammonium methacrylate copolymer.

In some exemplary embodiments of the present invention, the microcapsules are single-layer microcapsules, and the outer shell comprises $TiO_2$ in an amount within a range of from 30 % to 40 %, by weight relative to the total weight of the microcapsule, and does not comprise a fatty acid salt. In some of these embodiments, the wall-forming polymer is a cellulose ester such as cellulose acetate.

In some exemplary embodiments, a microcapsule as described herein is a single-layer microcapsule and comprises a reflective agent as described herein in an amount of about 60-80 % by weight, a wall-forming polymer or copolymer in an amount of 5-10 % by weight, magnesium stearate in an amount of 0-1 % by weight, and $TiO_2$ in an amount of 0-35% by weight relative to the total weight of the microcapsule.

In a preferred embodiment of the invention, the microcapsules are single-layer microcapsules.

In a preferred embodiment of the invention, the microcapsules are single-layer microcapsules comprising the inner core constituted by bismuth oxychloride dispersed in 2-ethylhexyl hydroxystearate in an amount from 60 to 80% by weight, the outer shell comprises magnesium stearate in an amount within a range of from 1.0 % to 2.0 % by weight, $TiO_2$ in an amount within a range of from 1 % to 20 % by weight, more preferably from 5 % to 15 % by weight, more particularly in an amount of 10 %, by weight, and, as a wall-forming polymer, PMMA, in an amount within a range of from 5 % to 20 % by weight, more preferably in an amount of 10 %, by weight, relative to the total weight of the microcapsule. An exemplary such composition is presented in Example 1 hereinafter. The amount of the raw material (oily dispersion of bismuth oxychloride) used to prepare the microcapsules is 79 % by weight of the total weight of the microcapsule.

In another preferred embodiment of the invention, the microcapsules are single-layer microcapsules comprising the inner core constituted by bismuth oxychloride dispersed in 2-ethylhexyl hydroxystearate in an amount of from 60 to 80% by weight, the outer shell does not comprise magnesium stearate, and comprises $TiO_2$ in an amount within a range of 10 % to 50 % by weight, more preferably from 10 % to 40 %, more particularly from 20 % to 40 % by weight, advantageously from 30 % to 40 % by weight more advantageously in an amount of 25 % by weight, and, as a wall-forming polymer, ethyl cellulose, in an amount within a range of 1 % to 10 % by weight, more preferably in an amount of 5 %, by weight relative the total weight of the microcapsule. An exemplary such composition is presented in Example 2 hereinafter. The amount of the raw material (oily dispersion of reflective agent) used to prepare the microcapsules is

60 % by weight of the total weight of the microcapsule.

In another preferred embodiment of the invention, the microcapsules are single-layer microcapsules comprising the inner core constituted by bismuth oxychloride dispersed in 2-ethylhexyl hydroxystearate in an amount of from 60 to 80% by weight, and the outer shell comprises magnesium stearate in an amount within a range of from 1.0 % to 2.0 %, by weight, $TiO_2$ in an amount within a range of 1 % to 20 % by weight, preferably from 5 % to 15 % by weight, more preferably in an amount of 10 % by weight, and, as a wall-forming polymer EUDRAGIT® RS PO (Poly(ethyl acrylate-co-methyl methacrylate-co-trimethyl ammonium ethyl methacrylate chloride), in an amount within a range of 5 % to 20 % by weight, more preferably in an amount of 10 %, by weight relative the total weight of the microcapsule. An exemplary such composition is presented in Example 3 hereinafter. The amount of the raw material (oily dispersion of bismuth oxychloride) used to prepare the microcapsules is 79 % by weight relative to the total weight of the microcapsule.

In another preferred embodiment of the invention, the microcapsules are single-layer microcapsules.comprising the inner core constituted by bismuth oxychloride dispersed in 2-ethylhexyl hydroxystearate in an amount of from 60 to 80% by weight, the outer shell comprises magnesium stearate in an amount within a range of from 1.0 % to 2.0 % by weight, $TiO_2$ in an amount within a range of 1 % to 20 % by weight, more preferably from 5 % to 15 % by weight, more particularly in an amount of 10 % by weight, and, as a wall-forming polymer, PMMA/MA, in an amount within a range of 5 % to 20 % by weight, more preferably in an amount of 10 % by weight relative to the total weight of the microcapsule. An exemplary such composition is presented in Example 4 hereinafter. The amount of the raw material (oily dispersion of bismuth oxychloride) used to prepare the microcapsules is 79 % by weight of the total weight of the microcapsule.

In another preferred embodiment of the invention, the microcapsules are single-layer microcapsules comprising the inner core constituted by bismuth oxychloride dispersed in 2-ethylhexyl hydroxystearate in an amount of from 60 to 80% by weight, the outer shell does not comprise magnesium stearate nor $TiO_2$, and comprises a plasticizer, in an amount within a range of 1 % to 20 % by weight, preferably from 5 % to 15 % by weight, more preferably, in an amount of 10 % by weight, and, as a wall-forming material, EUDRAGIT® RS PO (Poly(ethyl acrylate-co-methyl methacrylate-co-trimethyl ammonium ethyl methacrylate chloride), in an amount within a range of 5 % to 20 % by weight, more preferably in an amount of 10 % by weight relative to the total weight of the microcapsule. An exemplary such composition is presented in Example 5 hereinafter. The amount of the raw material (oily dispersion of bismuth oxychloride) used to prepare the microcapsules is 80 % by weight of the total weight of the microcapsule.

**The process for the preparation of the microcapsules:**

The process used for the preparation of the microcapsules according to embodiments of the present invention is a modification of the microencapsulation solvent removal method disclosed, for example, in U.S. Patent Nos. 6,932,984 and 7,838,037 and WO 2012/156965, which are incorporated by reference as if fully set forth herein. According to this technology, the active ingredient is found in the core of the microcapsule. This technique seals each micro-capped ingredient from chemical and cross-link reactions, degradation, color change or loss of potency during production, and for extended periods in storage.

The solvent removal process is based on four main steps as follows:

(i) preparing a homogeneous organic solution comprising the encapsulated oily dispersion of reflective agent, and a wall-forming polymeric material, and optionally an opaque substance and/or a fatty acid salt, and an organic solvent that is partially miscible in water;
(ii) preparing an emulsion of an aqueous continuous phase containing an emulsifier and saturated with the same organic solvent of the organic solution, and optionally comprising the opaque substance;
(iii) mixing the homogeneous organic solution with the aqueous emulsion, under high shear stirring to thereby form an emulsion; and
(iv) extracting the organic solvent by adding to the emulsion formed in step (iii) an amount of water which initiates extraction of the organic solvent from the emulsion, thereby obtaining the microcapsules.

For multi-layer (e.g., double-layer and triple-layer) microcapsules, the microcapsules are formed by first modifying the surface of the single-layer microcapsules formed according to steps (i)-(iv) and then subjecting the surface-modified inner core microcapsules to one or more cycles of steps (i)-(iv), when the inner core microcapsules are dispersed in the organic solution together with the wall-forming material.
In some embodiments, the microcapsules according to the present embodiments can be prepared a modified solvent removal method comprising the following steps:

(a) contacting an organic phase comprising an oily dispersion of reflective agent, and a wall-forming polymer or copolymer, optionally a fatty acid salt, and optionally an opaque substance and/or a plasticizer, and a first partially water-miscible organic solvent, with an aqueous solution saturated with said organic solvent and comprising an

emulsifier, to thereby obtain an emulsion; and

(b) adding to the formed emulsion an amount of water which initiates extraction of the organic solvent from the emulsion, thereby obtaining the microcapsules.

In further steps, the microcapsules are isolated following step (b), dried and sifted to thereby obtain a free flowing powder of the microcapsules.

These steps are further detailed as follows:

The homogenous solution prepared in step (a) is obtained by preparing an organic solution or dispersion of a wall-forming polymeric material as described in any one of the respective embodiments described herein, in an organic solvent that is partially miscible in water and is capable of dissolving or dispersing the wall-forming polymer. In exemplary embodiments, the organic solvent is an organic solvent approved for topical applications, such as, but not limited to, ethyl acetate, ethanol, ethyl formate, or any combination thereof. In some embodiments, the organic solvent is ethyl acetate.

The fatty acid salt is as described in any one of the respective embodiments described herein. The opaque substance is as described in any one of the respective embodiments described herein. In preferred embodiments, the opaque substance is $TiO_2$.

When a plasticizer is used, it is usually selected from tricaprylin, trilaurin, tripalmitin, triacetin, triethyl citrate, acetyltriethyl citrate, paraffin oil, or any combination thereof. The components of the organic solution are mixed/stirred until a homogeneous, optionally transparent, solution or dispersion is obtained.

The aqueous continuous phase is saturated with the organic solvent that forms the organic solution, and typically comprises an emulsifier, and optionally the opaque substance (if included in the microcapsule and not included in the organic phase).

The organic solution or dispersion and the aqueous continuous phase are mixed under low sheer stirring to thereby form an emulsion.

In step (b), an amount of water is added to the emulsion prepared in (a), thereby extracting the organic solvent and allowing the r microcapsules to form.

In the context of embodiments of the invention, the term "low sheer stirring" refers to a mixing at about 100-800 rpm, preferably at about 300-600 rpm.

In some embodiments, when the microcapsules are multi-layer microcapsules, the process further comprises:

(c) optionally repeating steps (a) and (b), using a second, third, and so on, organic phases and aqueous continuous phases, thereby obtaining multi-layered microcapsules.

## HYDROPHILIC GELLING AGENT

The term "hydrophilic gelling agent" within the meaning of the present invention, a compound capable of gelling the aqueous phase of the compositions according to the invention.

The gelling agent is hydrophilic and is thus present in the aqueous phase of the composition.

The gelling agent may be water soluble or water-dispersible.

As stated above, the aqueous phase of a composition according to the invention is gelled with at least one hydrophilic gelling agent.

The hydrophilic gelling agent may be chosen from synthetic polymeric gelling agents, natural or polymeric gelling agents of natural origin, mixed silicates and pyrogenic silicas, and mixtures thereof.

These hydrophilic gelling agents can be cationic, anionic, amphoteric or nonionic.

Preferably, the hydrophilic gelling agent may be chosen from synthetic polymeric gelling agents.

The synthetic polymeric hydrophilic gelling considered according to the invention may be particulate or not.

Within the meaning of the invention, the term means that the particulate polymer is in the form of particles, preferably spherical.

### Polymeric gelling agent natural or of natural origin

The polymeric hydrophilic gelling agents suitable for the invention may be natural or of natural origin.

Within the meaning of the invention, the term "naturally occurring" meant polymeric gelling agents obtained by modification of natural polymeric gelling agents.

These gelling agents can be particulate or non-particulate.

Specifically, these gelling under the category of polysaccharides.

In general, the polysaccharides can be distinguished into several categories.

And polysaccharides suitable for the invention can be homopolysaccharides the picture glucans, mannans and galactans or heteropolysaccharides in the image of hemicellulose.

Similarly, it may be linear or branched polysaccharides in the image of the gum arabic and amylopectin, and composite image of the starch.

In particular, polysaccharides suitable for the invention can be distinguished depending on whether they are starch or not.

A. Starch Polysaccharides

As representatives of this category can be particularly cited, native starches, modified starches and particulate starches.

Native starches

The starches used in the present invention are more particularly macromolecules form of polymers consisting of elementary units which are anhydroglucose units (dextrose), linked by $\alpha$(1,4) bounds, of chemical formula $(C_6H_{10}O_5)_n$. The number of these units and their assembly distinguish amylose molecule formed from about 600 to 1,000 glucose molecules chained linearly, and amylopectin, branched polymer every 25 glucose residues approximately ($\alpha$ bond (1.6). The total chain can be between 10 000 and 100 000 glucose residues.

Starches are described in particular in "Kirk-Othmer Encyclopedia of Chemical TECHNOLOGY, 3rd edition, volume 21, pages 492-507, Wiley Interscience, 1983'.

The relative proportions of amylose and amylopectin and their degree of polymerization, vary in function of the botanical origin of the starches. On average, a sample of native starch comprises about 25% amylose and 75% amylopectin.

Sometimes there is presence of phytoglycogen (between 0% and 20% of the starch), an analog of the branched amylopectin but every 10-15 glucose residues.

The starch can be in the form of semi-crystalline granules: amylopectin is organized in sheets, amylose forms a less organized amorphous region between the different layers.

Amyloidosis is organized in a right propeller to six glucose per turn. It dissociates into assimilated glucose by the action of enzymes, amylases, especially easily if it is in the form of amylopectin. Indeed, the helical formation does not promote accessibility of starch with enzymes.

Starches are generally in the form of a white powder insoluble in cold water, the elementary particle size is from 3 to 100 microns.

By treating it with hot water, the starch is obtained. It is operated in the industry for its gelling and thickening properties.

The starch molecules used in the present invention may have as botanical cereals or tubers. Thus, the starches are for example selected from starches of corn, rice, manioc, tapioca, barley, potato, wheat, sorghum, peas.

Native starches are exemplified by the products sold under the names C * AmilogelTM, Cargill GelTM, C * GelTM, Cargill GumTM, DryGelTM, C* Pharm GelTM by Cargill under the name Amidon but by Roquette, and under the name Pure Tapioca by National Starch.

Modified starches

The modified starches used in the composition of the invention may be modified by one or more of the following reactions: pregelatinization, degradation (acid hydrolysis, oxidation, dextrinization), substitution (esterification, etherification), crosslinking (esterification), bleaching.

More particularly, these reactions can be performed as follows:

- Pregelatinization by splitting the starch granules (for example drying and cooking in a drying drum);
- Acid hydrolysis leading to a very fast cooling demotion;
- Dextrinization at high temperature acidic medium (hydrolysis then repolymerization) ;
- Crosslinking with functional agents capable of reacting with the hydroxyl groups of the starch molecules, which will thus be linked together (for example with glyceryl and / or phosphate);
  Alkaline medium esterification for grafting functional groups, especially C1-C6 acyl (acetyl), C1-C6 hydroxyalkyl (hydroxyethyl, hydroxypropyl) carboxymethyl, octenyl. One can in particular be obtained by crosslinking with phosphorus compounds, monostarch phosphates (of the type $A_m$-O-PO-$(OX)_2$), the distarch phosphates (of the type $A_m$-O-PO-(OX)-O-$A_m$) or even triamidon (type $A_m$-O-PO-$(O$-$A_m)_2$) or mixtures thereof.

X denotes in particular the alkali metal (e.g. sodium or potassium), alkaline earth metals (e.g. calcium, magnesium), ammonium salts, amine salts such as monoethanolamine, diethanolamine, triethanolamine, the 3-amino 1,2-propanediol, ammoniums resulting from basic amino acids such as lysine, arginine, sarcosine, ornithine or citrulline.

Phosphorus compounds can be for example sodium tripolyphosphate, sodium orthophosphate, phosphorus oxychloride or sodium trimetaphosphate.

The starch molecules may be derived from any vegetable sources such as starch including corn, potato, oats, rice, tapioca, sorghum, barley or wheat. May also be used hydrolyzates of starches mentioned above.

Modified starches are exemplified by the products sold under the designations C *Tex-Instant (pregelatinized adipate), C *StabiTex-Instant (pregelatinised phosphate), C*PolarTex-Instant (hydroxypropylated pregelatinised) *Set C (acid hydrolysis, oxidation), C * size (oxidation), C * BatterCrisp (oxidation), C * DRYset (dextrinization), C*TexTM (acetylated distarch adipate), C*PolarTexTM (hydroxypropyl distarch phosphate) , C * StabiTexTM (distarch phosphate, acetylated distarch phosphate) by Cargill, for distarch phosphates or compounds rich in distarch phosphate as the product provided under the PREJEL references VA-70-T AGGL (distarch phosphate gelatinized hydroxypropylated cassava) or PREJEL TK1 (cassava distarch phosphate gelatinized) or PREJEL 200 (cassava gelatinized acetylated distarch phosphate) by the company Avebe, or STRUCTURE ZEA from National Starch (gelatinized corn distarch phosphate).

Gelatinized corn distarch phosphate will be preferably used, in particular STRUCTURE ZEA from National Starch.

B. Non-starch polysaccharides

In general, non-starch polysaccharides may be selected from polysaccharides produced by microorganisms; isolated from algae polysaccharides, polysaccharides of higher plants, such as homogeneous polysaccharides, in particular cellulose and its derivatives, heterogeneous polysaccharides such as gum arabic, galactomannans, glucomannan, and derivatives thereof; and mixtures thereof.

In particular, the polysaccharides can be chosen from glucans, amylose, amylopectin, glycogen, celluloses and their derivatives, especially methyl celluloses, hydroxyalkylcelluloses, ethylhydroxyethylcelluloses, and, mannans, xylans, lignins, the arabans, galactans, galacturonans,, chitin, chitosans, glucoronoxylanes, arabinoxylans, xyloglucans, glucomannans, arabinogalactans, the, the, the glycosaminoglycans, arabic gums, tragacanth gums, gum ghatti , the karaya gums, carob gums, galactomannans such as guar gums and their nonionic derivatives, in particular hydroxypropyl guar, and ionic, biopolysaccharide gums of microbial origin, in particular scleroglucan gum or of, mucopolysaccharides and particularly chondroitin sulfate and mixtures thereof.

These polysaccharides can be chemically modified, including urea group, urethane, or by hydrolysis reaction, oxidation, esterification, etherification, sulfation, phosphation, amination, amidation of alkylation or more of these changes.

Such a gelling agent may be implemented in an amount of 0.1% to 8% by weight in solids relative to the total weight of the aqueous phase, particularly from 0.1% to 6% by weight.

More specifically, these polysaccharides suitable for the invention can be distinguished depending on whether they are from microorganisms, algae and higher plants, and are detailed below.

Polysaccharides produced by microorganisms

Succinoglycan

Succinoglycan is an extracellular polymer produced by bacterial fermentation, high molecular weight and consisting of repeating units of octasaccharides (repeat eight sugars). Succinoglycans are for example marketed under the name Rheozan by Rhodia.

Scleroglucan

Scleroglucan is a nonionic branched homopolysaccharide, consisting of units β-D glucan. Molecules consist of a linear main chain made of D-glucose units linked by β bonds (1,3) and including a three is linked to a side D-glucose unit by a bond β (1,6).

A more complete description of scleroglucans and their preparation can be found in US 3,301,848.

Scleroglucan is for example sold under the name Amigum by Alban Muller Company, or under the name CS ™ Actigum by Cargill.

Polysaccharides isolated from algae

Galactannes

The polysaccharide of the invention may be chosen especially from a galactan agar.
gar-type galactannes are polysaccharides galactose containedd in the cell wall of certain species of red algae (Rhodophyceae). They are formed from a polymer group whose basic skeleton is a β chain(1,3)D-galactopyranose and α(1,4)3-6-anhydrogalactose, these units repeating regularly and alternately. The differences within the family of agars are due to the presence or absence of methylated or solvated carboxyethylated groups. These hybrid structures are usually present in varying percentages, depending on the algae species and the harvest season.
Agar-agar is a mixture of polysaccharides (agarose and agaropectin) of high molecular weight between 40000 to 300 000 $g.mol^{-1}$. It is obtained by manufacturing algae extraction juice, usually by autoclaving, and by treating these juices which include about 2% agar-agar in order to extract the latter.
Agar is such product by the group B & V Agar Producers, under the name Gold Hagar Agarite and Grand Agar by Hispanagar society, and under the Agar-Agar names, QSA (Quick Soluble Agar) and Puragar by Setexam company.

Furcellaran

Furcellaran is commercially obtained from red algae Furcellaria fasztigiata. Furcellaran is for example produced by the East-Agar society.

Polysaccharides of superior plants

This category of polysaccharides can be divided into homogeneous polysaccharides (one species of monosaccharides) and heterogeneous compounds of several kinds of monosaccharides.

a) Homogeneous polysaccharides and their derivatives

The polysaccharide according to the invention may be selected from celluloses and derivatives or fructans.

Cellulose and derivatives

The polysaccharide according to the invention may also be a cellulose or a derivative thereof in particular cellulose esters or ethers (eg methylcellulose, carboxymethylcellulose, hydroxymethylcellulose, hydroxyethylcellulose, hydroxypropyl cellulose, hydroxymethyl propyl cellulose, cellulose acetate, cellulose nitrate, nitrocellulose).
The invention may also contain a cellulose associative polymer. By cellulose compound means any compound of the invention polysaccharide containing, in its structure, linear chains of residues anhydroglucopyranose (AGU) which are united by glycosidic β(1,4) bounds. The repeating unit is cellobiose dimer. The AGU are under chair conformation and have 3 hydroxyl groups : 2 secondary alcohols (in position 2 and 3) and a primary alcohol (6-position). The polymers thus formed are associated with each other by intermolecular bonds such as hydrogen bonding, thereby imparting structure to fibrillar cellulose (about 1500 molecules per fiber).
The degree of polymerization differs greatly depending on the origin of cellulose; its value can vary from hundreds to tens of thousands.
The cellulose has the following chemical structure:

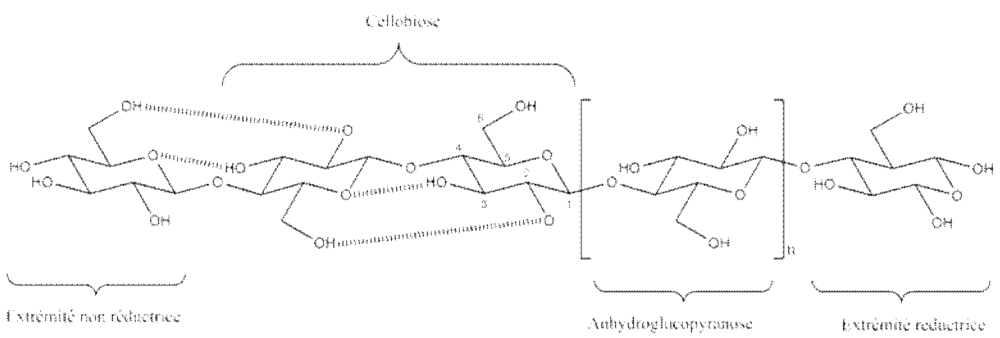

The hydroxyl groups of cellulose may react with partially or totally different chemical reagents to provide cellulose

derivatives with its own properties. Cellulose derivatives can be anionic, cationic, amphoteric or nonionic. Among these derivatives, there are the cellulose ethers, celluloses esters and ethers of cellulose esters.

Among the nonionic cellulose ethers include alkyl celluloses, such as methylcelluloses and ethylcelluloses; hydroxyalkyl, such as hydroxymethyl, hydroxyethyl and hydroxypropyl; mixed cellulose hydroxyalkyl alkylcelluloses, such as hydroxypropylmethylcellulose, the hydroxyethylmethylcelluloses, the hydroxyethylethylcelluloses and hydroxybutyl methyl celluloses.

Among the anionic cellulose ethers include carboxyalkylcelluloses and their salts. For example, there may be mentioned carboxymethyl celluloses, carboxymethylmethylcelluloses and carboxymethylhydroxyethylcelluloses and their sodium salts.

Among the cationic cellulose ethers include hydroxyethyl celluloses, crosslinked or non-quaternized.

The quaternizing may especially glycidyltrimethylammonium chloride or a fatty amine, such as laurylamine or stearylamine. As other cationic cellulose ether may be mentioned hydroxyethylcellulosehydroxypropyltrimethylammonium.

The quaternized cellulose derivatives are, in particular:

- Quaternized celluloses modified with groups comprising at least one fatty chain such as alkyl, arylalkyl or alkylaryl groups containing at least 8 carbon atoms, or mixtures thereof;
- Quaternized hydroxyethylcelluloses modified with groups comprising at least one fatty chain such as alkyl, arylalkyl or alkylaryl groups containing at least 8 carbon atoms, or mixtures thereof.

The alkyl radicals borne by the quaternized celluloses or hydroxyethylcelluloses above preferably contain from 8 to 30 carbon atoms.

The aryl radicals preferably denote phenyl, benzyl, naphthyl or anthryl.

It may be mentioned as examples of quaternized alkylhydroxyethylcelluloses fatty chains C8-C30 products Quatrisoft LM 200, LM-X 529-18 QUATRISOFT-A, LM-X QUATRISOFT 529-18B alkyl (C12) and QUATRISOFT LM-X 529-8 (C 18 alkyl) sold by the company Amerchol and the products Crodacel QM, CRODACEL QL alkyl (C12) and CRODACEL QS (C 18 alkyl) sold by the company Croda.

Among the cellulose derivatives, may also be mentioned :

- Celluloses modified with groups comprising at least one fatty chain, for example hydroxyethylcelluloses modified with groups comprising at least one fatty chain such as alkyl, in particular C8 -C22 arylalkyl or alkylaryl groups, such as Natrosol Plus Grade330CS (C16alkyls) sold by the company Aqualon, and
- Celluloses modified with polyalkylene glycol groups of alkyl phenol ether, such as the product Amercell Polymer HM-1500 (polyethylene glycol (15) ether of nonylphenol) sold by the company Amerchol.

Among the cellulose esters, there are inorganic cellulose esters (nitrates, sulfates, phosphates or cellulose ...), organic esters of cellulose (monoacetate, triacetate, amidopropionates, acetatebutyrates, acetatepropionates or cellulose acetatetrimellitates ...) and mixed esters organic / inorganic cellulose such as cellulose and acetatebutyratesulfates acetatepropionatesulfates. Among the cellulose ether esters include phthalates, hydroxypropyl methylcellulose and ethyl cellulose sulphates.

Cellulose compounds of the invention may be chosen from unsubstituted celluloses and substituted celluloses.

Celluloses and derivatives are exemplified by the products sold under the names Avicel (microcrystalline cellulose, MCC) by FMC Biopolymers, under the name Cekol (carboxymethylcellulose) by the company Noviant (CP-Kelco), under the name Akucell AF (carboxymethylcellulose sodium) by Akzo Nobel under the name MethocelTM (cellulose ethers) and EthocelTM (ethyl cellulose) by Dow under the Aqualon® names (carboxymethyl cellulose and sodium carboxymethylcellulose), Benecel® (methylcellulose), BlanoseTM (carboxymethylcellulose), Culminal® (methylcellulose, hydroxypropyl methylcellulose), Klucel (hydroxypropyl cellulose), Polysurf® (cetyl hydroxyethyl cellulose) and Natrosol® CS (hydroxyethyl) from Hercules Aqualon.

b) Heterogeneous polysaccharides and their derivatives

The polysaccharides used according to the invention may be gums such as cassia gum, karaya gum, konjac, tragacanth, tara, gum arabic or acacia.

Arabic Gum

Arabic gum is a highly branched acid polysaccharide which is in the form of potassium salts of mixtures of magnesium and calcium. The monomeric components of the free acid (arabic acid) are D-galactose, L-arabinose, L-rhamnose and D-glucuronic acid.

Galactomannans (guar, locust bean, fenugreek, tara gum) and derivatives (phosphate guar, hydroxypropyl guar ...)

Galactomannans are nonionic polysaccharides extracted from the albumen of seeds of leguminous plants which they constitute the glucide reserve.

Galactomannans are macromolecules consisting of a main chain of D-mannopyranose units linked in β(1,4) on the side branches consistuted of a single unit D-galactopyranose linked in α (1,6) to the chain main. Different galactomannans are distinguished firstly by the proportion of α-D-galactopyranose units present in the polymer, and secondly by significant differences in terms of distribution of galactose units along the mannose chain.

The mannose / galactose ratio (M / G) is of the order of 2 to guar gum, tara gum 3 and 4 for the locust bean gum.

Galactomannans have the following chemical structure:

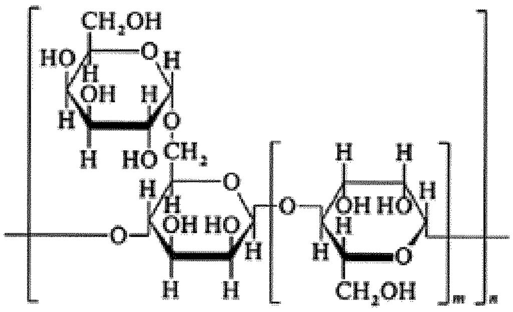

*m* = 3: Locust beam gum
*m* = 1: Guar gum
*m* = 2: Tara gum

<u>Guar</u>

Guar gum is characterized by a ratio mannose: galactose in the range of 2: 1. Galactose group is regularly distributed along the mannose chain.

Guar gums used according to the invention may be nonionic, cationic or anionic. According to the invention, use may be nonionic guar gums chemically modified or unmodified.

The unmodified non-ionic guar gums are for example the products sold under the name Vidogum GH, G and Vidogum Vidocrem Unipektin the company and under the name Jaguar by Rhodia under the name Meypro® Guar by the company Danisco under the name VISCOGUMTM by Cargill, and under the name Supercol® guar gum by Aqualon.

Gums nonionic guar hydrolyzed used according to the invention are for example represented by the products sold under the name Meyprodor® by Danisco.

Nonionic guar gums modified used in the invention are preferably modified with hydroxyalkyl groups C1-C6alkyl, among which may be mentioned hydroxymethyl, hydroxyethyl, hydroxypropyl and hydroxybutyl groups.

Such nonionic guar gums optionally modified with hydroxyalkyl groups are sold, for example under the trade names Jaguar HP 60, HP Jaguar 105 and Jaguar HP 120 (hydroxypropyl guar), by Rhodia, or under the name N-Hance HP ® (hydroxypropyl guar) by Aqualon.

The cationic galactomannan gums preferably have a lower cationic charge density than or equal to 1.5 meq / g and more particularly between 0.1 and 1 meq / g. The charge density can be determined according to the Kjeldahl method. It usually corresponds to a pH of about 3 to 9.

Generally, within the meaning of the present invention, the term "cationic galactomannan gum" means any galactomannan gum containing cationic groups and / or ionized to cationic groups.

Preferred cationic groups are chosen from those comprising primary, secondary, tertiary and / or quaternary.

The cationic galactomannan gums used generally have an average molecular weight between 500 and approximately 5x106, and preferably between $10^3$ and about $3x10^6$. The cationic galactomannan gums used in the present invention are, for example gums comprising cationic tri (C1-C4) ammonium. Preferably, 2% to 30% by number of the hydroxyl functions of these gums bear trialkylammonium cationic groups.

Among these trialkylammonium groups, there may be mentioned most particularly trimethylammonium and triethylammonium groups. Still more preferably, these groups represent from 5% to 20% by weight of the total weight of the modified galactomannan gum.

According to the invention, the cationic galactomannan gum is preferably a guar gum hydroxypropyl trimethylammonium comprising groups, ie a guar gum modified for example with 2,3-epoxypropyl trimethylammonium.

These galactomannan gums in particular guar modified with cationic groups are products already known in themselves and are described for example in US Patent 3,589,578 and US 4 031 307. Such products are also sold under the EXCEL trade names Jaguar, Jaguar C13 S, Jaguar C 15, Jaguar C 17 and Jaguar C162 (guar hydroxypropyltrimonium chloride) by Rhodia under the name Amilan® Guar (guar hydroxypropyltrimonium chloride) by Degussa and under the name N-Hance® 3000 (guar hydroxypropyltrimonium chloride) by Aqualon.

Anionic guar gums used in the invention are polymers comprising groups derived from carboxylic acid, sulfonic acid, sulfenic, phosphoric, phosphonic or pyruvic acid. Preferably, the anionic group is a carboxylic acid group. The anionic group may also be in the form of an acid salt, especially a sodium, calcium, lithium or potassium.

Anionic guar gums used according to the invention are preferably carboxymethylated guar derivatives (carboxymethyl guar or carboxymethyl hydroxypropyl guar).

Carob gums

Locust bean gum is extracted from the seeds of the carob tree (Ceratonia siliqua). The unmodified gum carob used in this invention is sold for example under the name Viscogum ™ by Cargill under the name L Vidogum by Unipektin company under the name Grinsted® LBG by Danisco.

Carob gums chemically modified for use in this invention can be represented for example by cationic locust beans sold under the name Catinal CLB (carob hydroxypropyltrimonium chloride) by Toho Company.

Tara gum

Tara gum used in the context of this invention is sold for example under the name Vidogum SP Unipektin by the company.

Glucomannan (konjac gum)

Glucomannan is a high molecular weight polysaccharide (500 000 <Mglucomannane <2 million), consisting of D-mannose and D-glucose with a branch at about 50 or 60 units. It is found in the woods but it is also the main component of konjac gum. The konjac (Amorphophallus konjac) is a plant of the family Araceae.

The products used according to the invention are for example sold under the name Propol® and Rheolex® by Shimizu society.

Other polysaccharides

Other polysaccharides useful according to the invention, mention may also be made of chitin (poly-N-acetyl-D-glucosamine, β(1,4)-2-acetamido-2-deoxy-D-glucose), chitosan and derivatives (chitosan-beta-glycerophosphate, carboxymethylchitin, etc.) as those sold by the company France-Chitin.

II. Synthetic polymeric gelling agents

Within the meaning of the invention, the term means that the synthetic polymer is not naturally occurring or not derived from a polymer of natural origin.

The synthetic polymeric hydrophilic gelling agent is advantageously chosen from crosslinked acrylic homo- or co-polymers ; associative polymers, especially polyurethane type associative polymers ; polyacrylamides ; crosslinked and/or neutralized polymers and ccopolymers of 2-acrylamido-2-methylpropanesulphonic ; modified or unmodified crosslinked; carboxyvinyl polymers, and mixtures thereof, especially as defined below.

A. Particulate synthetic polymeric gelling agent

They are preferably chosen from the crosslinked polymers.
It can especially be crosslinked acrylic homopolymers or copolymers, preferably partially neutralized or neutralized, which are in particulate form.

According to one embodiment, the particulate gelling agent according to the present invention is selected from crosslinked sodium polyacrylates. Preferably, it has, in a dry or non-hydrated state, an average size not exceeding 100 microns, preferably less than or equal to 50 microns. The average particle size is the mean diameter by mass (D50) measured by laser granulometry or other similar method known to those skilled in the art.

Thus, preferably, the particulate gelling agent according to the present invention is selected from crosslinked sodium polyacrylates, preferably in the form of particles having an average size (mean diameter) less than or equal to

100 microns, more preferably in particle form spherical.

Examples of crosslinked sodium polyacrylate include those sold under the names Octacare X100, X110 and RM100 by Avecia, those marketed under the names Flocare GB300 and Flosorb 500 by SNF, those marketed under the names Luquasorb 1003, Luquasorb 1010, Luquasorb Luquasorb 1280 and 1110 by BASF, those marketed under the names Water Lock G400 and G430 (INCI name: Acrylamide / Sodium acrylate copolymer) by the company Grain Processing.

Also exemplary crosslinked polyacrylate microspheres such as for example those sold under the name SH 10 AQUAKEEP® NF offered by Sumitomo Seika.

Such gelling agents can be implemented in an amount of 0.1% to 5% by weight in solids relative to the total weight of the aqueous phase, in particular 0.3% to 2% by weight and in particular in an amount from about 0.5% to 1.7% by weight relative to the total weight of the aqueous phase.

B. Non particulate synthetic polymeric gelling agents

This gelling family may be detailed in the following sub-families:

1. the associative polymers,
2. the polyacrylamides and polymers and copolymers of 2-acrylamido-2 methylpropanesulfonic acid, crosslinked and/or neutralized, and
3. modified or unmodified carboxyvinylpolymers.

**1. Associative polymers**

For the purposes of the present invention, the term "associative polymer" means any amphiphilic polymer comprising in its structure at least one fatty chain and at least one hydrophilic portion. The associative polymers in accordance with the present invention may be anionic, cationic, nonionic or amphoteric.

B1. Associative anionic polymers

Among the associative anionic polymers that may be mentioned are those comprising at least one hydrophilic unit, and at least one fatty-chain allyl ether unit, more particularly those whose hydrophilic unit is formed by an unsaturated ethylenic anionic monomer, more particularly by a vinylcarboxylic acid and most particularly by an acrylic acid or a methacrylic acid or mixtures thereof, and whose fatty-chain allyl ether unit corresponds to the monomer of formula (I) below:

$$CH_2=C(R')CH_2OB_nR \qquad (I)$$

in which R' denotes H or $CH_3$, B denotes the ethylenoxy radical, n is zero or denotes an integer ranging from 1 to 100, R denotes a hydrocarbon-based radical chosen from alkyl, arylalkyl, aryl, alkylaryl and cycloalkyl radicals, comprising from 8 to 30 carbon atoms, preferably 10 to 24 and even more particularly from 12 to 18 carbon atoms.

Anionic amphiphilic polymers of this type are described and prepared, according to an emulsion polymerization process, in patent EP 0 216 479.

Among the associative anionic polymers that may also be mentioned are maleic anhydride/C30-C38 $\alpha$-olefin/alkyl maleate terpolymers, such as the product (maleic anhydride/C30-C38 $\alpha$-olefin/isopropyl maleate copolymer) sold under the name Performa V 1608 by the company Newphase Technologies.

Among the associative anionic polymers, it is possible, according to a preferred embodiment, to use copolymers comprising among their monomers an $\alpha,\beta$-monoethylenically unsaturated carboxylic acid and an ester of an $\alpha,\beta$-monoethylenically unsaturated carboxylic acid and of an oxyalkylenated fatty alcohol. Preferentially, these compounds also comprise as monomer an ester of an $\alpha,\beta$-monoethylenically unsaturated carboxylic acid and of a C1-C4 alcohol.

Examples of compounds of this type that may be mentioned include Aculyn 22® sold by the company Rohm & Haas, which is a methacrylic acid/ethyl acrylate/oxyalkylenated stearyl methacrylate (comprising 20 OE units) terpolymer or Aculyn 28 (methacrylic acid/ethyl acrylate/oxyethylenated behenyl methacrylate (25 OE) terpolymer).

Examples of associative anionic polymers that may also be mentioned include anionic polymers comprising at least one hydrophilic unit of unsaturated olefinic carboxylic acid type, and at least one hydrophobic unit exclusively of the type such as a (C10-C30) alkyl ester of an unsaturated carboxylic acid. Examples that may be mentioned include the anionic polymers described and prepared according to patents US 3 915 921 and 4 509 949.

Associative anionic polymers that may also be mentioned include anionic terpolymers.

The anionic terpolymer used according to the invention is a linear or branched and/or crosslinked terpolymer,

of at least one monomer (1) bearing an acid function in free form, which is partially or totally salified with a nonionic monomer (2) chosen from N,N-dimethylacrylamide and 2-hydroxyethyl acrylate and at least one polyoxyethylenated alkyl acrylate monomer (3) of formula (I) below:

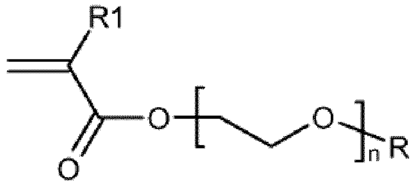

(I)

in which $R_1$ represents a hydrogen atom, R represents a linear or branched C2-C8 alkyl radical and n represents a number ranging from 1 to 10.

The term "branched polymer" denotes a non-linear polymer which bears side chains so as to obtain, when this polymer is dissolved in water, a high degree of entanglement leading to very high viscosities, at a low speed gradient. The term "crosslinked polymer" denotes a non-linear polymer which is in the form of a three-dimensional network that is insoluble in water but swellable in water, leading to the production of a chemical gel.

The acid function of the monomer (1) is especially a sulfonic acid or phosphonic acid function, the said functions being in free or partially or totally salified form.

The monomer (1) may be chosen from styrenesulfonic acid, ethylsulfonic acid and 2-methyl-2-[(1-oxo-2-propenyl)amino]-1-propanesulfonic acid (also known as acryloyldimethyl taurate), in free or partially or totally salified form. It is present in the anionic terpolymer preferably in molar proportions of between 5 mol% and 95 mol% and more particularly between 10 mol% and 90 mol%. The monomer (1) will more particularly be 2-methyl-2-[(1-oxo-2-propenyl)amino]-1-propanesulfonic acid in free or partially or totally salified form.

The acid function in partially or totally salified form will preferably be an alkali metal salt such as a sodium or potassium salt, an ammonium salt, an amino alcohol salt such as a monoethanolamine salt, or an amino acid salt such as a lysine salt.

The monomer (2) is preferably present in the anionic terpolymer in molar proportions of between 4.9 mol% and 90 mol%, more particularly between 9.5 mol% and 85 mol% and even more particularly between 19.5 mol% and 75 mol%.

In formula (I), examples of linear C8-C16 alkyl radicals that may be mentioned include octyl, decyl, undecyl, tridecyl, tetradecyl, pentadecyl and hexadecyl.

In formula (I), examples of branched C8-C16 alkyl radicals that may be mentioned include 2-ethylhexyl, 2-propylheptyl, 2-butyloctyl, 2-pentylnonyl, 2-hexyldecyl, 4-methylpentyl, 5-methylhexyl, 6-methylheptyl, 15-methylpentadecyl, 16-methylheptadecyl and 2-hexyloctyl.

According to a particular form of the invention, in formula (I), R denotes a C12-C16 alkyl radical.

According to a particular form of the invention, in formula (I), n ranges from 3 to 5. Tetraethoxylated lauryl acrylate will more particularly be used as monomer of formula (I).
The monomer (3) of formula (I) is preferably present in the anionic terpolymer in molar proportions of between 0.1 mol% and 10 mol% and more particularly between 0.5 mol% and 5 mol%.

According to a particular mode of the invention, the anionic terpolymer is crosslinked and/or branched with a diethylenic or polyethylenic compound in the proportion expressed relative to the total amount of monomers used, from 0.005 mol% to 1 mol%, preferably from 0.01 mol% to 0.5 mol% and more particularly from 0.01 mol% to 0.25 mol%.

The crosslinking agent and/or branching agent is preferably chosen from ethylene glycol dimethacrylate, diallyloxyacetic acid or a salt thereof, such as sodium diallyloxyacetate, tetraallyloxyethane, ethylene glycol diacrylate, diallylurea, triallylamine, trimethylolpropane triacrylate and methylenebis(acrylamide), or mixtures thereof.

The anionic terpolymer may contain additives such as complexing agents, transfer agents or chain-limiting agents.

Use will be made more particularly of an anionic terpolymer of 2-methyl-2-[(1-oxo-2-propenyl]amino]-1-propanesulfonic acid partially or totally salified in the form of the ammonium salt, N,N-dimethylacrylamide and tetraethoxylated lauryl acrylate crosslinked with trimethylolpropane triacrylate, of INCI name Polyacrylate Crosspolymer-6, such as the product sold under the trade name Sepimax Zen® by the company SEPPIC

Cationic associative polymers

Cationic associative polymers that may be mentioned include polyacrylates bearing amine side groups.

The polyacrylates bearing quaternized or non-quaternized amine side groups contain, for example, hydrophobic

groups of the type such as steareth-20 (polyoxyethylenated (20) stearyl alcohol).
Examples of polyacrylates bearing amino side chains that may be mentioned are the polymers 8781-121B or 9492-103 from the company National Starch.

Nonionic associative polymers

Nonionic associative polymers may be chosen from:

- The copolymers of vinylpyrrolidone and of hydrophobic monomers containing a fatty chain;
- Copolymers of methacrylates or alkyl acrylates, C1-C6 and of amphiphilic monomers comprising at least one fatty chain;
- Copolymers of hydrophilic methacrylates or acrylates and of hydrophobic monomers comprising at least one fatty chain such as for example the copolymer of polyethylene glycol methacrylate / lauryl methacrylate;
- Associative polyurethanes.

Associative polyurethanes are nonionic block copolymers comprising in the chain, both hydrophilic sequences most often of polyoxyethylenated nature (polyurethanes can be called polyether polyurethanes), and hydrophobic sequences which may be aliphatic chains alone and / or cycloaliphatic and / or aromatic sequences.
In particular, these polymers comprise at least two lipophilic hydrocarbon chains, having from C6 to C30 carbon atoms, separated by a hydrophilic sequence, the hydrocarbon chains may be pendent chains or chains at the end of a hydrophilic sequence. In particular, it is possible that one or more pendent chains to be provided. In addition, the polymer may comprise a hydrocarbon chain at one end or both ends of a hydrophilic sequence.
Associative polyurethanes can be sequenced as triblock or multiblock. The hydrophobic blocks may be at each end of the chain (for example: triblock copolymer with hydrophilic central block) or distributed both at the ends and in the chain (multiblock copolymer for example). These polymers may also be graft or star.
Preferably, the associative polyurethanes are triblock copolymers whose hydrophilic sequence is a polyoxyethylenated chain comprising from 50 to 1000 oxyethylene groups. In general, the associative polyurethanes comprise a urethane bond between the hydrophilic blocks, whence arises the name.
In a preferred embodiment, is used as a gelling a nonionic associative polyurethane polymer.
As examples of nonionic fatty-chain polyurethane polyethers used in the invention, one can also use Rheolate® FX 1100 (steareth-100 / PEG 136 / HDI (hexamethyl diisocyanate) copolymer), the Rheolate® 205 urea function, sold by Elementis or the Rheolates® 208, 204 or 212, as Acrysol® RM 184 or RM 2020 Acrysol®.
One can also quote the product Elfacos® T210 alkyl chain C12-C14 and the product Elfacos® T212 C16-18 alkyl chain (PPG-14-Palmeth 60 Hexyl dicarbamate) of Akzo.
The DW product from Rohm & Haas 1206B® to C.sub.20 alkyl chain and a urethane bond, sold at 20% dry matter in water, can also be used.
It is also possible to use solutions or dispersions of these polymers, especially in water or in a hydroalcoholic medium. Examples of such polymers include the Rheolate® 255 Rheolate ® ® 278 and Rheolate 244 sold by Elementis. You can also use the product DW 1206F and DW 1206J sold by the company Rohm & Haas.
Associative polyurethanes used according to the invention are in particular those described in the article by G. Fonnum, J. Bakke and Fk. Hansen, Colloid Polym. Sci, 271, 380-389 (1993).
More particularly, according to the invention can also be used a polyurethane associative capable of being obtained by polycondensation of at least three compounds comprising (i) at least one polyethylene glycol comprising from 150 to 180 moles of ethylene oxide, (ii) stearyl alcohol or decyl alcohol, and (iii) at least one diisocyanate.
Such polyurethane polyethers are sold especially by the company Rohm & Haas under the names of Aculyn® 46 and Aculyn® 44. The Aculyn 46® is a polycondensate of polyethylene glycol 150 or 180 mol of ethylene oxide, of stearyl alcohol and of methylene-bis(4-cyclohexyl isocyanate) (SMDI), at 15% by weight in a matrix of maltodextrin (4%) and water (81%), and Aculyn®44 is a polycondensate of polyethylene glycol containing 150 or 180 mol of ethylene oxide, decyl alcohol and methylenebis (4-cyclohexyl isocyanate) (SMDI), 35% by weight in a mixture of propylene glycol (39%) and water (26%).
It is also possible to use solutions or dispersions of these polymers, especially in water or in a hydroalcoholic medium. Examples of such polymers include SER AD FX1010, SER AD FX1035 and SER AD 1070 from Elementis, the Rheolate® 255, 278 and Rheolate® Rheolate® 244 sold by Elementis . Aculyn® the products can also be used 44 Aculyn® 46, DW 1206F and DW 1206J, and the Acrysol® RM 184 from Rohm & Haas, or even the Borchi Gel LW 44 from Borchers, and mixtures thereof.

**Amphoteric associative polymers**

Among the associative amphoteric polymers of the invention, mention may be made of crosslinked or non-crosslinked, branched or unbranched amphoteric polymers, which may be obtained by copolymerization:

1) of at least one monomer of formula (IVa) or (IVb):

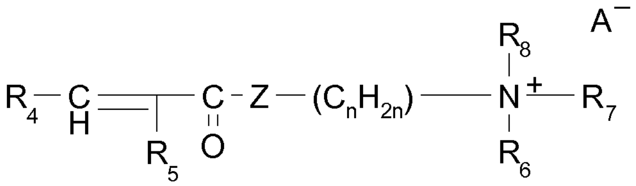
(IVa)

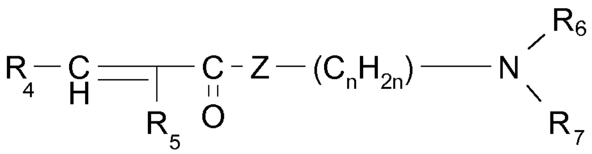
(IVb)

in which $R_4$ and $R_5$, which may be identical or different, represent a hydrogen atom or a methyl radical;
$R_6$, $R_7$ and $R_8$, which may be identical or different, represent a linear or branched alkyl radical containing from 1 to 30 carbon atoms;
Z represents an NH group or an oxygen atom;
n is an integer from 2 to 5;
A- is an anion derived from an organic or mineral acid, such as a methosulfate anion or a halide such as chloride or bromide;

2) of at least one monomer of formula (V):

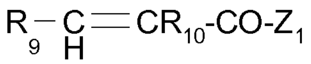
(V)

in which $R_9$ and $R_{10}$, which may be identical or different, represent a hydrogen atom or a methyl radical;
Z1 represents a group OH or a group $NHC(CH_3)_2CH_2SO_3H$;

3) of at least one monomer of formula (VI):

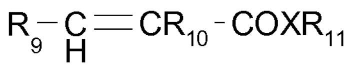
(VI)

in which $R_9$ and $R_{10}$, which may be identical or different, represent a hydrogen atom or a methyl radical, X denotes an oxygen or nitrogen atom and $R_{11}$ denotes a linear or branched alkyl radical containing from 1 to 30 carbon atoms;
4) optionally at least one crosslinking or branching agent; at least one of the monomers of formula (IVa), (IVb) or (VI) comprising at least one fatty chain containing from 8 to 30 carbon atoms and said compounds of the monomers of formulae (IVa), (IVb), (V) and (VI) possibly being quaternized, for example with a C1-C4 alkyl halide or a C1-C4 dialkyl sulfate.

The monomers of formulae (IVa) and (IVb) of the present invention are preferably chosen from the group formed by:

- dimethylaminoethyl methacrylate, dimethylaminoethyl acrylate,
- diethylaminoethyl methacrylate, diethylaminoethyl acrylate,
- dimethylaminopropyl methacrylate, dimethylaminopropyl acrylate,
- dimethylaminopropylmethacrylamide or dimethylaminopropylacrylamide, optionally quaternized, for example with a C1-C4 alkyl halide or a C1-C4 dialkyl sulfate.

More particularly, the monomer of formula (IVa) is chosen from acrylamidopropyltrimethylammonium chloride and methacrylamidopropyltrimethylammonium chloride.

The compounds of formula (V) of the present invention are preferably chosen from the group formed by acrylic acid, methacrylic acid, crotonic acid, 2-methylcrotonic acid, 2-acrylamido-2-methylpropanesulfonic acid and 2-methacrylamido-2-methylpropanesulfonic acid. More particularly, the monomer of formula (V) is acrylic acid.

The monomers of formula (VI) of the present invention are preferably chosen from the group formed by C12-C22 and more particularly C16-C18 alkyl acrylates or methacrylates.

The crosslinking or branching agent is preferably chosen from N,N'-methylenebisacrylamide, triallylmethylammonium chloride, allyl methacrylate, n-methylolacrylamide, polyethylene glycol dimethacrylates, ethylene glycol dimethacrylate, diethylene glycol dimethacrylate, 1,6-hexanediol dimethacrylate and allyl sucrose.

The polymers according to the invention may also contain other monomers such as nonionic monomers and in particular C1-C4 alkyl acrylates or methacrylates.

The ratio of the number of cationic charges/anionic charges in these amphoteric polymers is preferably equal to about 1.

The weight-average molecular weights of the associative amphoteric polymers have a weight-average molecular mass of greater than 500, preferably between 10 000 and 10 000 000 and even more preferentially between 100 000 and 8 000 000.

Preferably, the associative amphoteric polymers of the invention contain from 1 mol% to 99 mol%, more preferentially from 20 mol% to 95 mol% and even more preferentially from 25 mol% to 75 mol% of compound(s) of formula (IVa) or (IVb).

They also preferably contain from 1 mol% to 80 mol%, more preferentially from 5 mol% to 80 mol% and even more preferentially from 25 mol% to 75 mol% of compound(s) of formula (V). The content of compound(s) of formula (VI) is preferably between 0.1 mol% and 70 mol%, more preferentially between 1 mol% and 50 mol% and even more preferentially between 1 mol% and 10 mol%. The crosslinking or branching agent, when it is present, is preferably between 0.0001 mol% and 1 mol% and even more preferentially between 0.0001 mol% and 0.1 mol%.

Preferably, the mole ratio between the compound(s) of formula (IVa) or (IVb) and the compound(s) of formula (V) ranges from 20/80 to 95/5 and more preferentially from 25/75 to 75/25.

The associative amphoteric polymers according to the invention are described, for example, in patent application WO 98/44012.

The amphoteric polymers that are particularly preferred according to the invention are chosen from acrylic acid/acrylamidopropyltrimethylammonium chloride/stearyl methacrylate copolymers.

According to a preferred embodiment, the associative polymer is chosen from nonionic associative polymers and more particularly from associative polyurethanes, such as Steareth-100/PEG-136/HDI Copolymer sold under the name Rheolate FX 1100 by Elementis.

Such an associative polymer is advantageously used in a proportion of from 0.1% to 8% by weight of solids and preferably about 3% by weight, relative to the total weight of the aqueous phase.

2. Polyacrylamides and crosslinked and/or neutralized 2-acrylamido-2-methylpropanesulfonic acid polymers and copolymers

The polymers used that are suitable as aqueous gelling agent for the invention may be crosslinked or non-crosslinked homopolymers or copolymers comprising at least the 2-acrylamidomethylpropanesulfonic acid (AMPS®) monomer, in a form partially or totally neutralized with a mineral base other than aqueous ammonia, such as sodium hydroxide or potassium hydroxide.

They are preferably totally or almost totally neutralized, i.e. at least 90% neutralized.

These AMPS® polymers according to the invention may be crosslinked or non-crosslinked.

When the polymers are crosslinked, the crosslinking agents may be chosen from the polyolefinically unsaturated compounds commonly used for crosslinking polymers obtained by free-radical polymerization.

Examples of crosslinking agents that may be mentioned include divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allyl or vinyl ethers of polyfunctional alcohols, and also the allylic esters of phosphoric and/or vinylphosphonic acid derivatives, or mixtures of these compounds.

According to one preferred embodiment of the invention, the crosslinking agent is chosen from methylenebisacrylamide, allyl methacrylate and trimethylolpropane triacrylate (TMPTA). The degree of crosslinking generally ranges from 0.01 mol% to 10 mol% and more particularly from 0.2 mol% to 2 mol% relative to the polymer.

The AMPS® polymers that are suitable for use in the invention are water-soluble or water-dispersible. They are in this case:

- either "homopolymers" comprising only AMPS monomers and, if they are crosslinked, one or more crosslinking agents such as those defined above;
- or copolymers obtained from AMPS® and from one or more hydrophilic or hydrophobic ethylenically unsaturated monomers and, if they are crosslinked, one or more crosslinking agents such as those defined above.

When said copolymers comprise hydrophobic ethylenically unsaturated monomers, the latter do not comprise a fatty chain and are preferably present in small amounts.
For the purpose of the present invention, the term "fatty chain" is intended to mean any hydrocarbon-based chain containing at least 7 carbon atoms.
The term "water-soluble or water-dispersible" means polymers which, when introduced into an aqueous phase at 25°C, at a mass concentration equal to 1%, make it possible to obtain a macroscopically homogeneous and transparent solution, i.e. a solution with a light maximum transmittance value, at a wavelength equal to 500 nm, through a sample 1 cm thick, of at least 60% and preferably of at least 70%.
The "homopolymers" according to the invention are preferably crosslinked and neutralized, and they may be obtained according to the preparation process comprising the following steps:

(a) the monomer such as AMPS in free form is dispersed or dissolved in a solution of tert-butanol or of water and tert-butanol;
(b) the monomer solution or dispersion obtained in (a) is neutralized with one or more mineral or organic bases, preferably aqueous ammonia NH3, in an amount making it possible to obtain a degree of neutralization of the sulfonic acid functions of the polymer ranging from 90% to 100%;
(c) the crosslinking monomer(s) are added to the solution or dispersion obtained in (b);
(d) a standard free-radical polymerization is performed in the presence of free-radical initiators at a temperature ranging from 10 to 150°C; the polymer precipitates in the tert-butanol-based solution or dispersion.

The water-soluble or water-dispersible AMPS® copolymers according to the invention contain water-soluble ethylenically unsaturated monomers, hydrophobic monomers, or mixtures thereof.
The water-soluble co-monomers may be ionic or nonionic.
Among the ionic water-soluble co-monomers, mention may be made, for example, of the following compounds and salts thereof:

- (meth)acrylic acid,
- styrenesulfonic acid,
- vinylsulfonic acid and (meth)allylsulfonic acid,
- vinylphosphonic acid,
- maleic acid,
- itaconic acid,
- crotonic acid,
- water-soluble vinyl monomers of formula (A) below:

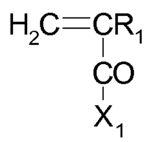 (A)

in which:

- $R_1$ is chosen from H, $-CH_3$, $-C_2H_5$ and $-C_3H_7$;
- $X_1$ is chosen from:
- alkyl oxides of type $-OR_2$ where $R_2$ is a linear or branched, saturated or unsaturated hydrocarbon-based radical containing from 1 to 6 carbon atoms, substituted with at least one sulfonic ($-SO_3-$) and/or sulfate ($-SO_4-$) and/or phosphate ($-PO_4H_2-$) group.

Among the nonionic water-soluble co-monomers, mention may be made, for example, of:

- (meth)acrylamide,
- N-vinylacetamide and N-methyl-N-vinylacetamide,
- N-vinylformamide and N-methyl-N-vinylformamide,
- maleic anhydride,
- vinylamine,
- N-vinyllactams comprising a cyclic alkyl group containing from 4 to 9 carbon atoms, such as N-vinylpyrrolidone, N-butyrolactam and N-vinylcaprolactam,
- vinyl alcohol of formula $CH_2$=CHOH,
- water-soluble vinyl monomers of formula (B) below:

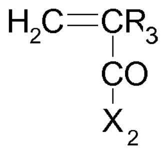

(B)

in which:

- $R_3$ is chosen from H, $-CH_3$, $-C_2H_5$ and $-C_3H_7$;
- $X_2$ is chosen from:
- alkyl oxides of the type -OR4 where R4 is a linear or branched, saturated or unsaturated hydrocarbon-based radical having from 1 to 6 carbon atoms, optionally substituted with a halogen (iodine, bromine, chlorine or fluorine) atom; a hydroxyl (-OH) group; ether.

Mention may be made, for example, of glycidyl (meth)acrylate, hydroxyethyl methacrylate, and (meth)acrylates of ethylene glycol, of diethylene glycol or of polyalkylene glycol.

Among the hydrophobic comonomers without a fatty chain, mention may be made, for example, of:

- styrene and derivatives thereof, such as 4-butylstyrene, $\alpha$-methylstyrene and vinyltoluene;
- vinyl acetate of formula $CH_2$=CH-$OCOCH_3$;
- vinyl ethers of formula $CH_2$=CHOR in which R is a linear or branched, saturated or unsaturated hydrocarbon-based radical containing from 1 to 6 carbons;
- acrylonitrile;
- caprolactone;
- vinyl chloride and vinylidene chloride;
- silicone derivatives, which, after polymerization, result in silicone polymers such as methacryloxypropyltris(trimethylsiloxy)silane and silicone methacrylamides;
- hydrophobic vinyl monomers of formula (C) below:

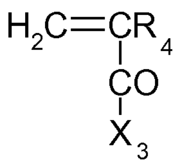

(C)

in which:

- $R_4$ is chosen from H, $-CH_3$, $-C_2H_5$ and $-C_3H_7$;
- $X_3$ is chosen from:
- alkyl oxides of the type $-OR_5$ where R5 is a linear or branched, saturated or unsaturated hydrocarbon-based radical containing from 1 to 6 carbon atoms.

Mention may be made, for example, of methyl methacrylate, ethyl methacrylate, n-butyl (meth)acrylate, tert-butyl (meth)acrylate, cyclohexyl acrylate, isobornyl acrylate and 2-ethylhexyl acrylate.

The water-soluble or water-dispersible AMPS® polymers of the invention preferably have a molar mass ranging from 50 000 g/mol to 10 000 000 g/mol, preferably from 80 000 g/mol to 8 000 000 g/mol, and even more preferably from 100 000 g/mol to 7 000 000 g/mol.

As water-soluble or water-dispersible AMPS homopolymers in accordance with the invention, mention may be made, for example, of crosslinked or non-crosslinked polymers of sodium acrylamido-2-methylpropanesulfonate, such as that used in the commercial product Simulgel 800 (CTFA name: Sodium Polyacryloyldimethyl Taurate), crosslinked ammonium acrylamido-2-methylpropanesulfonate polymers (INCI name: Ammonium polydimethyltauramide) such as those described in patent EP 0 815 928 B1 and such as the product sold under the trade name Hostacerin AMPS® by the company Clariant.

As water-soluble or water-dispersible AMPS copolymers in accordance with the invention, examples that may be mentioned include:

- crosslinked acrylamide/sodium acrylamido-2-methylpropanesulfonate copolymers, such as that used in the commercial product Sepigel 305 (CTFA name: Polyacrylamide/C13-C14 Isoparaffin/ Laureth-7) or that used in the commercial product sold under the name Simulgel 600 (CTFA name: Acrylamide/Sodium Acryloyldimethyltaurate/Isohexadecane/Polysorbate-80) by the company SEPPIC;
- copolymers of AMPS® and of vinylpyrrolidone or vinylformamide, such as that used in the commercial product sold under the name Aristoflex AVC® by the company Clariant (CTFA name: Ammonium acryloyldimethyltaurate/VP copolymer) but neutralized with sodium hydroxide or potassium hydroxide;
- copolymers of AMPS® and of sodium acrylate, for instance the AMPS/sodium acrylate copolymer, such as that used in the commercial product sold under the name Simulgel EG® by the company SEPPIC or under the trade name Sepinov EM (CTFA name: Hydroxyethyl acrylate/sodium acryloyldimethyltaurate copolymer);
- copolymers of AMPS® and of hydroxyethyl acrylate, for instance the AMPS®/hydroxyethyl acrylate copolymer, such as that used in the commercial product sold under the name Simulgel NS® by the company SEPPIC (CTFA name: Hydroxyethyl acrylate/sodium acryloyldimethyltaurate copolymer (and) squalane (and) polysorbate 60), or such as the product sold under the name Sodium acrylamido-2-methylpropanesulfonate/hydroxyethyl acrylate copolymer, such as the commercial product Sepinov EMT 10 (INCI name: Hydroxyethyl acrylate/sodium acryloyldimethyltaurate copolymer).

Preferably, the product sold under the name Sodium acrylamido-2-methylpropanesulfonate/hydroxyethyl acrylate copolymer, such as the commercial product Sepinov EMT 10 (INCI name: Hydroxyethyl acrylate/sodium acryloyldimethyl taurate copolymer) is used as water-soluble or water-dispersible AMPS copolymers in accordance with the invention.

In general, an aqueous phase according to the invention may comprise from 0.1 % to 8 % by weight of solids, preferably 0.2 % to 5 % by weight and more preferentially from 0.7 % to 2.5 % by weight of polyacrylamide(s) and/or of crosslinked and/or neutralized 2-acrylamido-2-methylpropanesulfonic acid polymer(s) and copolymer(s) relative to its total weight.

3. Modified or unmodified carboxyvinyl polymers

The modified or unmodified carboxyvinyl polymers may be copolymers derived from the polymerization of at least one monomer (a) chosen from $\alpha,\beta$-ethylenically unsaturated carboxylic acids or esters thereof, with at least one ethylenically unsaturated monomer (b) comprising a hydrophobic group.

The term "copolymers" means both copolymers obtained from two types of monomer and those obtained from more than two types of monomer, such as terpolymers obtained from three types of monomer.

Their chemical structure more particularly comprises at least one hydrophilic unit and at least one hydrophobic unit. The term "hydrophobic group or unit" means a radical with a saturated or unsaturated, linear or branched hydrocarbon-based chain, comprising at least 8 carbon atoms, preferably from 10 to 30 carbon atoms, in particular from 12 to 30 carbon atoms and more preferentially from 18 to 30 carbon atoms.

Preferably, these copolymers are chosen from copolymers derived from the polymerization:

- of at least one monomer of formula (1) below:

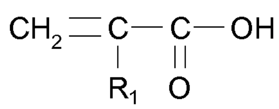

(1)

in which $R_1$ denotes H or $CH_3$ or $C_2H_5$, i.e. acrylic acid, methacrylic acid or ethacrylic acid monomers, and

- of at least one monomer of unsaturated carboxylic acid (C10-C30)alkyl ester type corresponding to the monomer of formula (2) below:

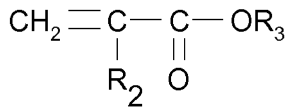

(2)

in which $R_2$ denotes H or $CH_3$ or $C_2H_5$ (i.e. acrylate, methacrylate or ethacrylate units) and preferably H (acrylate units) or $CH_3$ (methacrylate units), $R_3$ denoting a C10-C30 and preferably C12-C22 alkyl radical.

The unsaturated carboxylic acid (C10-C30)alkyl esters are preferably chosen from lauryl acrylate, stearyl acrylate, decyl acrylate, isodecyl acrylate and dodecyl acrylate, and the corresponding methacrylates, such as lauryl methacrylate, stearyl methacrylate, decyl methacrylate, isodecyl methacrylate and dodecyl methacrylate, and mixtures thereof. According to a preferred embodiment, these polymers are crosslinked.

Among the copolymers of this type, use will more particularly be made of polymers derived from the polymerization of a monomer mixture comprising:

- essentially acrylic acid,
- an ester of formula (2) described above in which R2 denotes H or CH3, R3 denoting an alkyl radical containing from 12 to 22 carbon atoms,
  (iii) and a crosslinking agent, which is a well-known copolymerizable polyethylenic unsaturated monomer, such as diallyl phthalate, allyl (meth)acrylate, divinylbenzene, (poly)ethylene glycol dimethacrylate or methylenebisacrylamide.

Among the copolymers of this type, use will more particularly be made of those consisting of from 95% to 60% by weight of acrylic acid (hydrophilic unit), 4% to 40% by weight of C10-C30 alkyl acrylate (hydrophobic unit) and 0% to 6% by weight of crosslinking polymerizable monomer, or alternatively those consisting of from 98% to 96% by weight of acrylic acid (hydrophilic unit), 1% to 4% by weight of C10-C30 alkyl acrylate (hydrophobic unit) and 0.1% to 0.6% by weight of crosslinking polymerizable monomer such as those described previously.

Among the abovementioned polymers, the ones that are most particularly preferred according to the present invention are acrylate/C10-C30-alkyl acrylate copolymers (INCI name: Acrylates/C10-30 Alkyl acrylate Crosspolymer) such as the products sold by the company Lubrizol under the trade names Pemulen TR-1, Pemulen TR-2, Carbopol 1382, Carbopol EDT 2020 and Carbopol Ultrez 20 Polymer, and even more preferentially Pemulen TR-2.

Among the modified or unmodified carboxyvinyl polymers, mention may also be made of sodium polyacrylates such as those sold under the name Cosmedia SP® containing 90% solids and 10% water, or Cosmedia SPL® as an inverse emulsion containing about 60% solids, an oil (hydrogenated polydecene) and a surfactant (PPG-5 Laureth-5), both sold by the company Cognis.

Mention may also be made of partially neutralized sodium polyacrylates that are in the form of an inverse emulsion comprising at least one polar oil, for example the product sold under the name Luvigel® EM by the company BASF.

The modified or unmodified carboxyvinyl polymers may also be chosen from crosslinked (meth)acrylic acid homopolymers.

For the purposes of the present patent application, the term "(meth)acrylic" means "acrylic or methacrylic".

Examples that may be mentioned include the products sold by Lubrizol under the names Carbopol 910, 934, 940, 941, 934 P, 980, 981, 2984, 5984 and Carbopol Ultrez 10 Polymer, or by 3V-Sigma under the name Synthalen® K, Synthalen® L or Synthalen® M.

Among the modified or unmodified carboxyvinyl polymers, mention may be made in particular of Carbopol (CTFA name: carbomer) and Pemulen (CTFA name: Acrylates/C10-30 alkyl acrylate crosspolymer) sold by the company Lubrizol.

The modified or unmodified carboxyvinyl polymers may be present in a proportion of from 0.1% to 5% by weight of solids relative to the weight of the aqueous phase, in particular from 0.3% to 1% by weight and preferably in a proportion of about 1% by weight, relative to the weight of the aqueous phase.

Advantageously, the hydrophilic gelling agent is at least one synthetic polymeric gelling agent chosen from crosslinked acrylic homopolymers or copolymers; polyacrylamides and crosslinked and/or neutralized 2-acrylamido-2-methylpropanesulfonic acid polymers and copolymers; modified or unmodified carboxyvinyl polymers, and mixtures thereof.

More particularly, it is at least a 2-acrylamido-2-methylpropanesulfonic acid polymer or copolymer, an associative polyurethane and/or a crosslinked sodium polyacrylate.

### III. Other hydrophilic gelling agents

These gelling agents are more particularly chosen from mixed silicates and fumed silicas.

### III.A Mixed silicate

For the purposes of the present invention, the term "mixed silicate" means any silicate of natural or synthetic origin containing several (two or more) types of cations chosen from alkali metals (for example Na, Li, K) or alkaline-earth metals (for example Be, Mg, Ca), transition metals and aluminium.

According to a particular embodiment, the mixed silicate(s) are in the form of solid particles containing at least 10% by weight of at least one silicate relative to the total weight of the particles. In the rest of the present description, these particles will be referred to as "silicate particles".

Preferably, the silicate particles contain less than 1% by weight of aluminium relative to the total weight of the particles. Even more preferably, they contain from 0% to 1% by weight of aluminium relative to the total weight of the particles.

Preferably, the silicate particles contain at least 50% by weight and better still at least 70% by weight of the silicate relative to the total weight of the particles. Particles containing at least 90% by weight of silicates, relative to the total weight of the particles, are particularly preferred.

In particular, this is a silicate or a mixture of silicates and of alkali metals or alkaline-earth metals, of aluminium or of iron.

Preferably, it is sodium, magnesium and/or lithium silicate.

To ensure good cosmetic properties, these silicates are generally in finely divided form, and in particular in the form of particles with a mean size ranging from 2 nm to 1 μm (from 2 nm to 1000 nm), preferably from 5 nm to 600 nm and even more preferentially from 20 to 250 nm.

Silicate particles may have any form, for example the form of spheres, flakes, needles, platelets, disks or leaflets, or totally random forms. Preferably, the silicate particles have the form of disks or leaflets.

Also, the term "mean size" of the particles means the number-average size of the largest dimension (length) that it is possible to measure between two diametrically opposite points on an individual particle. The size may be determined, for example, by transmission electron microscopy or by measuring the specific surface area by the BET method or alternatively by means of a laser particle sizer.

When the particles are in the form of disks or leaflets, they generally have a thickness ranging from about 0.5 nm to 5 nm.

The silicate particles may consist of an alloy with metal or metalloid oxides, obtained, for example, via thermal fusion of its various constituents. When the particles also comprise such a metal or metalloid oxide, it is preferably chosen from silicon, boron or aluminium oxide.

According to a particular embodiment of the invention, the silicates are phyllosilicates, i.e. silicates having a structure in which the SiO4 tetrahedra are organized as leaflets between which the metal cations are enclosed.

The mixed silicates that are suitable for use in the invention may be chosen, for example, from montmorillonites, hectorites, bentonites, beidellite and saponites.

According to a preferred embodiment of the invention, the mixed silicates used are more particularly chosen from hectorites and bentonites, and better still from laponites.

A family of silicates that is particularly preferred in the compositions of the present invention is thus that of laponites. Laponites are sodium magnesium silicates also possibly containing lithium, which have a layer structure similar to that of montmorillonites. Laponite is the synthetic form of the natural mineral known as hectorite. The synthetic origin of this family of silicates is of considerable advantage over the natural form, since it allows good control of the composition of the product. In addition, laponites have the advantage of having a particle size that is much smaller than that of the natural minerals hectorite and bentonite.

Laponites that may especially be mentioned include the products sold under the following names: Laponite® XLS, Laponite® XLG, Laponite® RD, Laponite® RDS and Laponite® XL21 (these products are sodium magnesium silicates and sodium lithium magnesium silicates) by the company Rockwood Additives Limited.

Such gelling agents may be used in a proportion of from 0.1% to 8% by weight of solids relative to the total weight of the aqueous phase, especially from 0.1% to 5% by weight and in particular from 0.5% to 3% by weight, relative to the total weight of the aqueous phase.

## III.B Hydrophilic fumed silica

The fumed silicas according to the present invention are hydrophilic.

Hydrophilic fumed silicas are obtained by pyrolysis of silicon tetrachloride ($SiCl_4$) in a continuous flame at 1000°C in the presence of hydrogen and oxygen. Among the fumed silicas of hydrophilic nature that may be used according to the present invention, mention may be made especially of the products sold by the company Degussa or Evonik-Degussa under the trade names Aerosil® 90, 130, 150, 200, 300 and 380 or by the company Cabot under the name Carbosil H5.

Such gelling agents may be used in a proportion of from 0.1% to 10% by weight of solids relative to the total weight of the aqueous phase, especially from 0.1% to 5% by weight and in particular from 0.5% to 3% by weight, relative to the total weight of the aqueous phase.

## COSMETIC COMPOSITIONS

The composition according to the invention, may be aqueous or hydro-alcoholic lotions or serums.

## AQUEOUS PHASE

The aqueous phase of a composition according to the invention comprises water and optionally a water-soluble solvent.

In the present invention, the term "water-soluble solvent" denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition in accordance with the invention, mention may be made especially of lower monoalcohols containing from 2 to 8 carbon atoms, such as ethanol and isopropanol, glycols containing from 2 to 8 carbon atoms, such as ethylene glycol, propylene glycol, 1,3-butylene glycol and dipropylene glycol, C3 and C4 ketones and C2-C4 aldehydes.

The gels according to the present invention comprise an aqueous phase generally in a proportion greater than or equal to 40% by weight and preferably greater than or equal to 50% by weight and more particularly greater than or equal to 55% by weight based on the weight total weight.

According to another embodiment variant, the aqueous phase of a composition according to the invention may comprise at least one C2-C32 polyol.

For the purposes of the present invention, the term "polyol" should be understood as meaning any organic molecule comprising at least two free hydroxyl groups.

Preferably, a polyol in accordance with the present invention is present in liquid form at room temperature.

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols advantageously suitable for the formulation of a composition according to the present invention are those exhibiting in particular from 2 to 32 carbon atoms and preferably from 3 to 16 carbon atoms.

Advantageously, the polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols such as glycerol oligomers, for instance diglycerol, and polyethylene glycols, and mixtures thereof.

According to a preferred embodiment of the invention, the said polyol is chosen from ethylene glycol, pentaerythritol, trimethylolpropane, propylene glycol, glycerol, polyglycerols and polyethylene glycols, and mixtures thereof.

According to a particular embodiment, the composition of the invention may comprise at least propylene glycol.

According to another particular embodiment, the composition of the invention may comprise at least glycerol.

A water suitable for the invention can be a floral water such as cornflower water and / or a mineral water such as water from Vittel, water from Lucas or water from La Roche Posay, and / or thermal water.

## COMPOSITIONS WITH ALCOHOL

According to a particular form of the invention, the composition contains at least one mono-alcohol comprising from 2 to 8 carbon atoms.

According to a particular form of the invention, the composition contains from 0,5 to 7%, and preferably from 1 to 5% by weight relative to the total weight of at least one mono-alcohol comprising from 2 to 8 carbon atoms.

The compositions of the invention comprise at least one mono-alcohol having from 2 to 8 carbon atoms, especially from 2 to 6 carbon atoms, and particularly 2 to 4 carbon atoms.

The compositions of the invention may include one or more mono-alcohol (s).
The monoalcohol may be represented for example by formula RaOH, wherein Ra is an alkyl group, linear or branched, comprising 2 to 8 carbon atoms.

As a monohydric alcohol include ethanol, isopropanol, propanol or butanol.

According to one embodiment, the compositions of the invention include ethanol.

## ADDITIVES

The compositions according to the invention can also contain additional cosmetic ingredients classically used for the formulation of particular galenic forms, generally adapted to the aimed keratinous material. Those additional cosmetic ingredients can be notably chosen film-forming polymers, non-ionic, anionic and cationic surfactants, hydrophilic or lipophilic gellants or thickeners, dispersants, actives, sunscreen agents, preservatives, antioxidants, solvents, perfumes, fillers other than the particles of the invention, bactericides, odour absorbers, coloring agents (pigments, nacres, water-soluble dyestuffs), salts, and their mixtures.

## COLORING AGENTS

According to a particular mode of the invention, the composition contain at least one particulate or non-particulate, water-soluble or water-insoluble coloring agent preferably in a proportion of at least 0.01% by weight relative to the total weight of the composition.

For obvious reasons, this amount is liable to vary significantly with regard to the intensity of the desired colour effect and of the colour intensity afforded by the coloring agents under consideration, and its adjustment clearly falls within the competence of a person skilled in the art.

For the purposes of the invention, the term "water-soluble coloring agent" means any natural or synthetic, generally organic compound, which is soluble in an aqueous phase or in water-miscible solvents, and which is capable of imparting colour.

As water-soluble dyes that are suitable for use in the invention, mention may be made especially of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper chlorophylline, methylene blue, anthocyanins (enocianin, black carrot, hibiscus and elder), caramel and riboflavin.

The water-soluble dyes are, for example, beetroot juice and caramel.

The particulate coloring agents may especially be pigments, nacres and/or particles with metallic tints.

The term "pigments" should be understood as meaning white or coloured, mineral or organic particles that are insoluble in an aqueous solution, which are intended to colour and/or opacify the composition containing them.

The pigments may be white or coloured, and mineral and/or organic.

As mineral pigments that may be used in the invention, mention may be made of titanium oxide, titanium dioxide, zirconium oxide, zirconium dioxide, cerium oxide or cerium dioxide and also zinc oxide, iron oxide or chromium oxide, ferric blue, manganese violet, ultramarine blue and chromium hydrate, and mixtures thereof.
It may also be a pigment having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

They may also be pigments having a structure that may be, for example, of silica microsphere type containing iron oxide. An example of a pigment having this structure is the product sold by the company Miyoshi under the reference PC Ball PC-LL-100 P, this pigment being constituted of silica microspheres containing yellow iron oxide.

Advantageously, the pigments in accordance with the invention are iron oxides and/or titanium dioxides.

The term "nacres" should be understood as meaning iridescent or non-iridescent coloured particles of any shape, especially produced by certain molluscs in their shell or alternatively synthesized, which have a colour effect via optical interference.

A composition according to the invention may comprise from 0% to 15% by weight of nacres relative to the total weight of the said composition.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles at the surface of which are superposed at least two successive layers of metal oxides and/or of organic dyestuffs.

Examples of nacres that may also be mentioned include natural mica coated with titanium oxide, with iron oxide, with natural pigment or with bismuth oxychloride.

Among the nacres available on the market, mention may be made of the nacres Timica, Flamenco and Duochrome (based on mica) sold by the company Engelhard, the Timiron nacres sold by the company Merck, the Prestige

mica-based nacres sold by the company Eckart, and the Sunshine synthetic mica-based nacres sold by the company Sun Chemical.

The nacres may more particularly have a yellow, pink, red, bronze, orangey, brown, gold and/or coppery colour or tint.

Advantageously, the nacres in accordance with the invention are micas coated with titanium dioxide or with iron oxide, and also bismuth oxychloride.

For the purposes of the present invention, the term "particles with a metallic tint" means any compound whose nature, size, structure and surface finish allow it to reflect the incident light, especially in a non-iridescent manner.

The particles with a metallic tint that may be used in the invention are in particular chosen from:

- particles of at least one metal and/or of at least one metal derivative;
- particles comprising a single-material or multi-material organic or mineral substrate, at least partially coated with at least one layer with a metallic tint comprising at least one metal and/or at least one metal derivative; and
- mixtures of the said particles.

Among the metals that may be present in the said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo and Cr, and mixtures or alloys thereof (for example bronzes and brasses) are preferred metals. The term "metal derivatives" denotes compounds derived from metals, especially oxides, fluorides, chlorides and sulfides.

Illustrations of these particles that may be mentioned include aluminum particles, such as those sold under the names Starbrite 1200 EAC® by the company Siberline and Metalure® by the company Eckart and glass particles coated with a metallic layer, especially those described in documents JP-A-09188830, JP-A-10158450, JP-A-10158541, JP-A-07258460 and JP-A-05017710.

Hydrophobic treatment of the coloring agent

The pulverulent dyestuffs as described previously may be totally or partially surface-treated, with a hydrophobic agent, to make them more compatible with the oily phase of the composition of the invention, especially so that they have good wettability with oils. Thus, these treated pigments are well dispersed in the oily phase. Hydrophobic-treated pigments are described especially in document EP-A-1 086 683.

The hydrophobic-treatment agent may be chosen from silicones such as methicones, dimethicones and perfluoroalkylsilanes; fatty acids, for instance stearic acid; metal soaps, for instance aluminium dimyristate, the aluminium salt of hydrogenated tallow glutamate; perfluoroalkyl phosphates; polyhexafluoropropylene oxides; perfluoropolyethers; amino acids; N-acylamino acids or salts thereof; lecithin, isopropyl triisostearyl titanate, isostearyl sebacate, and mixtures thereof.

The term "alkyl" mentioned in the compounds cited above especially denotes an alkyl group containing from 1 to 30 carbon atoms and preferably containing from 5 to 16 carbon atoms.

**FILLERS**

According to a particular mode of the invention, the composition contain at least one filler.

For the purposes of the present invention, the term "fillers" should be understood as meaning colourless or white solid particles of any form, which are in an insoluble and dispersed form in the medium of the composition.

These fillers, of mineral or organic, natural or synthetic nature, give the composition containing them softness and give the makeup result a matt effect and uniformity.

The fillers in the composition according to the present invention may be in lamellar form (or platelet), spherical (or globular), fiber or any other intermediate form between these defined forms.

Spherical Charges

Spherical fillers used according to the invention have the shape or substantially the shape of a sphere and may be hollow or solid. Advantageously, the spherical fillers of the invention have a particle size (number average diameter) of from 0.1 .mu.m to 250 μm, preferably from 1 μm to 150 μm, more preferably from 10 to100 μm.

The spherical fillers may be organic or mineral microspheres. As organic spherical fillers include for example polyamide powders and especially Nylon® powders such as Nylon-12 or Polyamide 12, sold under the names ORGASOL by Arkema; polyethylene powders; polytetrafluoroethylene powders (Teflon *); microspheres based on acrylic copolymers, such as copolymer of ethylene glycol dimethacrylate / lauryl methacrylate copolymer sold by Dow Corning under the name Polytrap; expanded powders such as hollow microspheres and especially the microspheres sold under the

name Expancel by Kemanord Plast or under the name Micropearl F 80 ED by Matsumoto; silicone resin microbeads such as those sold under the name Tospearl by Toshiba Silicone ; polymethyl methacrylate microspheres, sold under the name Microsphere M-100 by Matsumoto or under the name Covabead LH85 by Wacker; ethylene acrylate copolymer powders, such as those sold under the name Flobeads by Sumitomo Seika Chemicals; powders of natural organic materials such as starch powders, especially of corn starch, wheat or rice, crosslinked or otherwise, such as the powders of starch crosslinked with octenyl succinate anhydride, sold under the name Dry -FLO by National Starch; metal soaps derived from organic carboxylic acids having 8 to 22 carbon atoms, preferably from 12 to 18 carbon atoms, for example, zinc stearate, magnesium or lithium, zinc laurate, myristate magnesium, Polyporus the L * 200 (Chemdal Corporation), polyurethane powders, in particular, powders of crosslinked polyurethane comprising a copolymer, said copolymer comprising trimethylol hexyl lactone as the polymer of hexamethylene diisocyanate / trimethylol hexyl lactone, sold under the name Plastic Powder D-400® or Plastic Powder D-800® by the company Toshiki, carnauba microwaxes, such as that sold under the name MicroCare 350® by the company Micro Powders, microwaxes of synthetic wax such as that sold under the name MicroEase 114S® by the company Micro Powders, microwaxes consisting of a mixture of carnauba wax and polyethylene wax, such as those sold under the names Micro Care 300® and 310® by the company Micro Powders, microwaxes consisting of a mixture of carnauba wax and of synthetic wax, such as that sold under the name Micro Care 325® by the company Micro Powders, polyethylene microwaxes such as those sold under the names Micropoly 200®, 220®, and 220L® 250S® by the company Micro Powders.

As spherical inorganic filler, there may be mentioned the hydrophobic aerogel silica particles.

The hydrophobic silica aerogel particles, advantageously, present a specific surface area per unit mass (MS) of from 200 to 1500 $m^2/g$, preferably from 600 to 1200 $m^2/g$ and better still from 600 to 800 $m^2/g$. The surface area per unit mass can be determined by the nitrogen absorption method called BET (Brunauer - Emmet - Teller) method described in "The Journal of the American Chemical Society", Vol. 60, page 309, February 1938 and corresponding to the international standard ISO 5794/1 (Appendix D). The BET surface area is the total surface area of the said silica aerogel particles.

The hydrophobic silica aerogel particles, preferably have a size, expressed as mean diameter (D [0,5]) and measured according to the method previously described, less than 1500 microns and preferably from 1 to 30 $\mu$m, preferably from 5 to 25 $\mu$m, preferably 5 to 20 $\mu$m and more preferably 5 to 15 $\mu$m.

The hydrophobic silica aerogel particles may advantageously have a packed density $\rho$ of 0.04 to 0.10 $g/cm^3$, preferably 0.05 to 0.08 $g/cm^3$.

In the context of the present invention, the packed density $\rho$ can be assessed using the following protocol, said protocol of the packed density:

40g of powder is poured into a measuring cylinder and then the test piece is placed on a device 2003 in STAV STAMPF Volumeter. The specimen is then subjected to a series of 2500 settlements (this operation is repeated until the difference in volume between two successive tests is less than 2%); then the final volume Vf of packed powder is measured directly on the specimen. The packed density is determined by the mass ratio (m)/ Vf, namely 40/Vf (Vf being expressed in $cm^3$ and mg).

According to one embodiment, the hydrophobic silica aerogel particles have a specific surface area per unit volume SV of 5 to 60 $m^2$ / $cm^3$, preferably 10 to 50 $m^2$ /$cm^3$ and more preferably from 15 to 40 $m^2/cm^3$. The specific surface area per unit volume is given by the equation: SV = $\rho$ * SM where $\rho$ is the packed density in $g/cm^3$ and SM is the surface area per unit mass expressed in $m^2/g$, as defined above.

The hydrophobic silica aerogel particles are preferably silylated silica aerogel particles (INCI name : SILICA SILYLATE), especially particles of hydrophobic silica aerogels surface modified by trimethylsilyl groups (triméthylsiloxylated silica).

According to a preferred embodiment, the hydrophobic silica aerogel particles can be chosen (s) from:

- Aerogel marketed under the trademark VM-2260 (INCI name Silica silylate), by Dow Corning, whose particles have an average size of about 1000 microns and a surface area per unit mass of from 600 to 800 $m^2/g$,
- Aerogels marketed by the company Cabot Aerogel TLD under the references 201, 201 and EMT Aerogel, Aerogel TLD 203, Enova Aerogel MT 1100, Aerogel Enova MT 1200.

In a preferred embodiment, the hydrophobic silica aerogel particles will be selected from Aerogel marketed under the trademark VM-2270 (INCI name Silica silylate), by Dow Corning, whose particles have a mean size ranging from 5-15 microns and a surface area per unit mass of from 600 to 800 $m^2/g$.

As spherical inorganic filler, there may also be mentioned as silicas Sunsil 130 sold by Sunjin Chemical (INCI name: SILICA) AND (POLY) METAL OXIDES such as (poly) bismuth oxides.

Lamellar charges

As indicated above, the platy fillers are fillers of parallelepipedal shape (rectangular or square surface), discoidal (circular surface) or ellipsoidal (oval area), characterized by three dimensions: length, width and height. When the shape is circular, the length and width are identical and correspond to the diameter of a disc, while the height corresponds to the thickness of the disc. When the surface is oval, the length and width respectively correspond to the major axis and the minor axis of an ellipse and the height corresponds to the thickness of the elliptical disc formed by the wafer. When it is a parallelepiped, the length and width can be of identical or different dimensions when they are of the same size, the shape of the surface of the parallelepiped is square; otherwise, the shape is rectangular. As for the height, it is the thickness of the parallelepiped.
The lamellar fillers used according to the invention have a length ranging from 0.01 to 100 $\mu$m, preferably from 0.1 to 50 microns and preferably from 1 to 50 $\mu$m. Platelets have a width ranging from 0.01 to 100 $\mu$m, preferably from 0.1 to 50 $\mu$m and preferably 1 to 10 $\mu$m. Platelets have a height (thickness) of from 0.1 nm to 1 micron, preferably 1 to 600 nm and preferably from 1 to 500 nm.
As lamellar fillers used in the composition of the invention include phyllosilicates, such as talcs, micas, perlite and mixtures thereof.
Talcs are hydrous magnesium silicates comprising mostly aluminum silicate. The crystalline structure of talc consists in repeated layers of a brucite sandwich between the layers of silica. As talc, there may be mentioned the product sold under the name Micro Ace P3 by Nippon Talc (INCI name: talc), that sold under the name Luzenac 00 Imerys (INCI name: TALC), or the product sold under the name Luzenac Pharma M by Imerys (INCI name: TALC).
The micas are aluminum silicates optionally comprising iron and / or alkali metals. They have the property that it can be divided into thin layers (about 1 micron). They usually have a size of from 5 to 150 $\mu$m, preferably from 10 to 100 $\mu$m and more preferably from 10 to 60 $\mu$m for the largest dimension (length) and a height (thickness) of 0.1 to 0.5 $\mu$m. Among the micas, it may be mentioned phlogopite, muscovite, fluorophlogopite, vermiculite, and mixtures thereof. As mica, there may be mentioned the product sold under the name S-sericite-152 BC by Miyoshi Kasei (INCI name: mica), Mearlmica Treated SVA sold by BASF Personal Care Ingredients (INCI name: MICA (AND) LAUROYL LYSINE).
Among the phyllosilicates, mention may also be perlite and preferably the perlite.
The perlite used according to the invention are generally aluminosilicate volcanic origin and composition as :

- 70.0 to 75.0% by weight of silica $SiO_2$ 12.0 to 15.0% by weight of aluminum oxide $Al_2O_3$ oxide
- 3.0-5.0% sodium oxide $Na_2O$
- 3.0-5.0% of potassium oxide $K_2O$- 0.5-2% of iron oxide $Fe_2O_3$
- 0.2-0.7% of magnesium oxide MgO
- 0.5-1.5% calcium oxide CaO
- 0.05 to 0.15% titanium oxide $TiO_2$

Perlite is milled, dried and then calibrated in a first step. The product obtained is said Perlite Ore gray in color and size of the order of 100 microns. Perlite Ore is then expanded (1000 °C/2 seconds) to give particles more or less white. When the temperature reaches 850-900 ° C, the water trapped in the structure of the material vaporizes and causes the material to expand over its original volume. The expanded perlite particles according to the invention can be obtained by the expansion process described in US Patent 5,002,698.
Preferably the perlite particles used will be crushed; in this case they are called Expanded Perlite Milled (EMP). They preferably have a particle size defined by a median diameter D50 of from 0.5 to 50 microns and preferably from 0.5 to 40 $\mu$m. Preferably the perlite particles used have a bulk density loose packed at 25 °C ranging from 10 to 400 $kg/m^3$ (DIN 53468) and preferably 10 to 300 $kg/m^3$.
Preferably, it will be used expansed perlite particles sold under the trade names OPTIMAT 1430 OR or OPTIMAT 2550 by the company WORLD MINERALS.
May be mentioned also nitride boron, sericite, barium sulfate ($BaSO_4$), alumina ($Al_2O_3$) particles.
According to a preferred embodiment of the present invention, the (the) lamellar fillers (s) is (are) chosen (s) from talcs, micas, perlite, boron nitride and mixtures thereof.
In a preferred embodiment, the filler(s) is (are) selected from polyamide powders, elastomeric organopolysiloxane powders, metal soaps, silicas, (poly) metal oxides, hydrophobic silica aerogel particles, perlite, talcs, micas, boron nitride and a mixture thereof, preferably a mixture thereof.
In a preferred embodiment, the fillers are selected from OPTIMAT 1430 OR or OPTIMAT 2550 by the company WORLD MINERALS sold by Word Minerals, VM-2270 Aerogel Fine Particles sold by Dow Corning (INCI name: SILICA SILYLATE); Micro Ace P3 sold by Nippon Talc (INCI name: TALC); Sunsil 130 sold by Sunjin Chemical (INCI name: silica); Luzenac 00 sold by Imerys (INCI name: TALC); Orgasol 2002 sold by Arkema (INCI name: Nylon-12); Boron Nitride ays sold under the trade name SOFTOUCH BORON NITRIDE POWDER CC6058 by Momentive Performance

Materials, magnesium stearate sold by Stearinerie Dubois; sericite S-152-BC sold by Miyoshi Kasei (INCI name: MICA); and mixtures thereof, preferably mixtures thereof.

The amount of filler(s) can range, for example, from 0.05 to 10% by weight and better still from 0.1 to 5% by weight, with respect to the total weight of the composition.

## NON-EMULSIFYING ORGANOPOLYSILOXANE ELASTOMER

According to a particular mode of the invention, the composition contains at least one non-emulsifying organopolysiloxane elastomer.

The organopolysiloxane elastomer, usable as a lipophilic gelling agent, has the advantage of giving the composition according to the invention good application properties. It provides a very soft matting and after application, particularly advantageous for application to the skin. It may also allow effective hiding of pores present on the keratin materials. The compositions of the invention have a good wearing of the pores masking .

By "organopolysiloxane elastomer" or "silicone elastomer" refers to a flexible organopolysiloxane deformable having viscoelastic properties, and especially the consistency of a sponge or of a flexible sphere. Its modulus of elasticity is such that this material is resistant to deformation and has a limited capacity for expansion and contraction. This material is capable of regaining its original shape after stretching.

This is particularly a crosslinked organopolysiloxane elastomer.

Thus, the organopolysiloxane elastomer can be obtained by addition reaction of diorganopolysiloxane containing at least one hydrogen linked to silicon and of diorganopolysiloxane containing ethylenically unsaturated groups linked to silicon, especially in the presence of a platinum catalyst; or by dehydrogenation crosslinking condensation reaction between a hydroxyl terminated diorganopolysiloxane and a diorganopolysiloxane containing at least one hydrogen bonded to silicon, particularly in the presence of an organotin compound; or by crosslinking condensation reaction of a diorganopolysiloxane with hydroxyl end groups and of a hydrolysable organopolysilane; or by thermal crosslinking of organopolysiloxane, especially in the presence of an organoperoxide catalyst; or by crosslinking of organopolysiloxane by high-energy radiation such as gamma rays, ultraviolet rays or an electron beam.

Preferably, the organopolysiloxane elastomer is obtained by crosslinking addition reaction (A) of diorganopolysiloxane containing at least two hydrogens each bonded to a silicon, and (B) of diorganopolysiloxane containing at least two ethylenically unsaturated groups bonded to silicon especially in the presence (C) platinum catalyst, as described for example in EP-A-295886.

In particular, the organopolysiloxane elastomer can be obtained by reaction of dimethylpolysiloxane containing dimethylvinylsiloxy end groups and of a methylhydropolysiloxane comprising trimethylsiloxy endings in the presence of a platinum catalyst.

The compound (A) is the base reagent for the formation of elastomeric organopolysiloxane and the crosslinking is carried out by an addition reaction of compound (A) with the compound (B) in the presence of catalyst (C).

The compound (A) is an organopolysiloxane having at least two hydrogen atoms bonded to different silicon atoms in each molecule.

The compound (A) may have any molecular structure, in particular a straight chain or branched chain structure or a cyclic structure. The compound (A) may have a viscosity at 25 ° C ranging from 1 to 50,000 centistokes, especially have good miscibility with compound (B). The organic groups bonded to silicon atoms of the compound (A) may be alkyl groups such as methyl, ethyl, propyl, butyl, octyl; substituted alkyl groups such as 2-phenylethyl, 2-phenylpropyl, 3,3,3-trifluoropropyl; aryl groups such as phenyl, tolyl, xylyl; substituted aryl groups such as phenylethyl; and substituted monovalent hydrocarbon groups such as an epoxy group, a carboxylate ester group or a mercapto group.

Compound (A) can be selected from trimethylsiloxy-terminated methylhydrogenpolysiloxanes, copolymers terminated dimethylsiloxane-methylhydrogensiloxane containing trimethylsiloxy, cyclic dimethylsiloxane-methylhydrogensiloxane copolymers.

The compound (B) is preferably a diorganopolysiloxane having at least two lower alkenyl groups (e.g. C2-C4); the lower alkenyl group may be selected from vinyl, allyl, and propenyl. These lower alkenyl groups can be located in any position of the organopolysiloxane molecule but are preferably located at the ends of the organopolysiloxane molecule. The organopolysiloxane (B) may have a branched chain structure, a linear, cyclic or network structure but the linear chain structure is preferred. The compound (B) may have a viscosity ranging from the liquid state to the gum state. Preferably, compound (B) has a viscosity of at least 100 centistokes at 25 ° C.

In addition to the aforementioned alkenyl groups, other organic groups bonded to silicon atoms in the compound (B) may be alkyl groups such as methyl, ethyl, propyl, butyl or octyl; substituted alkyl groups such as 2-phenylethyl, 2-phenylpropyl or 3,3,3-trifluoropropyl; aryl groups such as phenyl, tolyl or xylyl; substituted aryl groups such as phenylethyl; and substituted monovalent hydrocarbon groups such as an epoxy group, a carboxylate ester group or a mercapto group.

The organopolysiloxane (B) can be chosen from methylvinylpolysiloxanes, methylvinylsiloxane-dimethylsiloxane copolymers them, dimethylpolysiloxanes comprising dimethylvinylsiloxy endings, dimethylsiloxane-methylphenylsiloxane copolymers containing dimethylvinylsiloxy end groups, copolymers terminated dimethylsiloxane-diphenylsiloxane-methylvinylsiloxane dimethylsiloxane copolymers, dimethylsiloxane-methylvinylsiloxane trimethylsiloxy end groups, dimethylsiloxane-methylphenylsiloxane-methylvinylsiloxane containing trimethylsiloxy end groups, methyl (3,3,3-trifluoropropyl) - polysiloxane dimethylvinylsiloxy endings and dimethylsiloxane-methyl (3,3,3-trifluoropropyl) siloxane-terminated dimethylpolysiloxane .

In particular, the organopolysiloxane elastomer can be obtained by reaction of dimethylpolysiloxane containing dimethylvinylsiloxy end groups and of methylhydrogen polysiloxane containing trimethylsiloxy end groups, in the presence of a platinum catalyst.

Advantageously, the sum of the number of ethylenic groups per molecule of the compound (B) and the number of hydrogen atoms bonded to silicon atoms per molecule of the compound (A) is at least 5.

It is advantageous that the compound (A) is added in an amount such that the molar ratio between the total amount of hydrogen atoms bonded to silicon atoms in the compound (A) and the total amount of all such groups ethylenically unsaturated compound (B) is in the range of 1.5 / 1 to 20/1.

The compound (C) is a catalyst for the crosslinking reaction, and is especially chloroplatinic acid, chloroplatinic acid-olefin complexes, chloroplatinic acid-alkenylsiloxane complexes, chloroplatinic acid-diketone, platinum black, and platinum on support.

The catalyst (C) is preferably added from 0.1 to 1000 parts by weight, more preferably from 1 to 100 parts by weight, as clean platinum metal per 1000 parts by weight of the total amount of compounds (A) and (B). The elastomer is preferably a non-emulsifying elastomer.

The term "non-emulsifying" defines organopolysiloxane elastomers not containing a hydrophilic chain, in particular containing no polyoxyalkylene units (in particular polyoxyethylene or polyoxypropylene) or polyglyceryl unit. Thus, in one particular embodiment of the invention, the composition comprises an elastomeric organopolysiloxane devoid of polyoxyalkylene units and polyglyceryl pattern.

In particular, the silicone elastomer used in the present invention is selected from Dimethicone Crosspolymer (INCI name), Vinyl Dimethicone Crosspolymer (INCI name), Dimethicone / Vinyl Dimethicone Crosspolymer (INCI name), Dimethicone Crosspolymer-3 (INCI name).

Organopolysiloxane elastomer particles can be conveyed in the form of a gel consisting of an elastomeric organopolysiloxane included in at least one hydrocarbon oil and / or a silicone oil. In these gels, the organopolysiloxane particles are often non-spherical particles.

Non-emulsifying elastomers are described in patents EP 242 219, EP 285 886, EP 765 656 and in JP-A-61-194009. The silicone elastomer is generally in the form of a gel, a paste or a powder but preferably as a gel in which the silicone elastomer is dispersed in a linear silicone oil (dimethicone) or cyclic (eg cyclopentasiloxane), preferably in a linear silicone oil.

As non-emulsifying elastomers that may be used more particularly those sold under the names "KSG-6", "KSG-15", "KSG-16", "KSG-18", "KSG-41", "KSG-42" "KSG-43", "KSG-44" by the company Shin Etsu, "DC9040", "DC9041" by Dow Corning, "SFE 839" by the company General Electric.

In one particular embodiment, one uses a silicone elastomer gel dispersed in a silicone oil selected from a non-exhaustive list including cyclopentadimethylsiloxane, dimethicones, the dimethylsiloxane the methyl trimethicone, phenylmethicone, phenyldimethicone, phenyltrimethicone and cyclomethicone, of preferably a linear silicone oil selected from the polydimethylsiloxanes (PDMS) or viscosity dimethicones at 25 ° C ranging from 1 to 500 cSt at 25 ° C, optionally modified by aliphatic groups, optionally fluorinated, or with functional groups such as groups hydroxyl, thiol and / or amine. These include in particular the following compounds having the INCI name:

- Dimethicone / Vinyl Dimethicone Crosspolymer, such as "USG-105" and "USG-107A" of Shin-Etsu; "DC9506" and "DC9701" by Dow Corning,
- Dimethicone / Vinyl Dimethicone Crosspolymer (and) Dimethicone, such as "KSG-6" and "KSG-16" by the company Shin Etsu;
- Dimethicone / Vinyl Dimethicone Crosspolymer (and) Cyclopentasiloxane, such as "KSG-15";
- Cyclopentasiloxane (and) Dimethicone Crosspolymer, such as "DC9040", "DC9045" and "DC5930" from Dow Corning;
- Dimethicone (and) Dimethicone Crosspolymer, such as "DC9041" from Dow Corning;
- Dimethicone (and) Dimethicone Crosspolymer, such as "Dow Corning silicone elastomer EL-9240® Blend" from Dow Corning (mixture of polydimethylsiloxane with Reticulated hexadiene / polydimethylsiloxane (2 cSt));
- C4-24 Alkyl Dimethicone / DivinylDimethicone Crosspolymer, such as Silk NuLastic Alzo AM by the company.

Examples of silicone elastomer dispersed in a linear silicone oil advantageously used in the invention, we may notably mention the following references:

- Dimethicone / Vinyl Dimethicone Crosspolymer (and) Dimethicone, such as "KSG-6" and "KSG-16" by the company Shin Etsu;
- Dimethicone (and) Dimethicone Crosspolymer, such as "DC9041" from Dow Corning; and
- Dimethicone (and) Dimethicone Crosspolymer, such as "Dow Corning silicone elastomer EL-9240® Blend" from Dow Corning (mixture of polydimethylsiloxane Reticulated by hexadiene / polydimethylsiloxane (2 cSt));
- Diphenylsiloxy PHENYL trimethicone (and) Dimethicone (and) PHENYL VINYL DIMETHICONE CROSSPOLYMER (INCI name) such as KSG 18A marketed by Shin Etsu).

The organopolysiloxane elastomer particles can also be used in powder form, mention may be made of the powders sold under the name "Dow Corning 9505 Powder", "Dow Corning 9506 Powder" by Dow Corning, these powders have the INCI name: dimethicone / vinyl dimethicone crosspolymer. The organopolysiloxane powder may also be coated with silsesquioxane resin, as described for example in US Patent 5,538,793. Such elastomeric powders are sold under the names "KSP-100", "KSP-101", "KSP-102", "KSP-103", "KSP-104", "KSP-105" by the company Shin Etsu, and the INCI name: vinyl dimethicone / methicone silsesquioxane crosspolymer.

Examples of organopolysiloxane powders coated with silsesquioxane resin used advantageously according to the invention include in particular organopolysiloxane elastomers INCI name VINYL dimethicone / methicone SILSESQUIOANE CROSSPOLYMER as those sold under the brand name "KSP-100" of the Shin Etsu. As a preferred lipophilic gelling agent type organopolysiloxane elastomer that may especially be mentioned crosslinked organopolysiloxane elastomers chosen from Dimethicone Crosspolymer (INCI name), Dimethicone (and) Dimethicone Crosspolymer (INCI name), Vinyl Dimethicone Crosspolymer (INCI name), Dimethicone / Vinyl Dimethicone Crosspolymer (INCI name), Dimethicone Crosspolymer-3 (INCI name), VINYL dimethicone / methicone SILSESQUIOANE CROSSPOLYMER, diphenylsiloxy PHENYL trimethicone (and) Dimethicone (and) PHENYL VINYL DIMETHICONE CROSSPOLYMER (INCI name) and especially Dimethicone Crosspolymer (INCI name).

The organopolysiloxane is preferably present in a concentration of 1 to 5%% by weight relative to the total weight of the composition.

**DISPERSANT**

Advantageously, a composition according to the invention may also comprise a dispersant.

Such a dispersant may be a surfactant, an oligomer, a polymer or a mixture of several thereof.

According to one particular embodiment, a dispersant in accordance with the invention is a surfactant.

**ACTIVE AGENT**

For a particular care application, a composition according to the invention may comprise at least one moisturizer (also known as a humectant).

Preferably, such moisturizer is glycerol.

The moisturizer(s) could be present in the composition in a content ranging from 0.1% to 15% by weight, especially from 0.5% to 10% by weight or even from 1% to 6% by weight, relative to the total weight of the said composition.

As other active agents that may be used in the composition of the invention, examples that may be mentioned include vitamins, such as vitamins A, C, E, B3, B5, K and their derivatives, in particular their esters, hyaluronic acid, sunscreens, urea and its hydroxylated derivatives, such as the N-(2-hydroxyethyl)urea sold under the name Hydrovance by National Starch; salicylic acid, 5-n-octanoyl salicylic acid or CAPRYLOYL SALICYLIC ACID sold under the trade name MEXORYL SAB®; C-BETA-D-XYLOPYRANOSIDE-2-HYDROXY-PROPANE in particular in solution at 30% in a mixture water/1,2-propanediol under the trade name MEXORYL BB®; sequestering agents, such as EDTA, and mixtures thereof.

Preferably, a composition of the invention comprises at least one active agent.

It is a matter of routine for those skilled in the art to adjust the nature and amount of the additives present in the compositions in accordance with the invention such that the desired cosmetic properties thereof are not thereby affected.

According to another embodiment, a composition of the invention may advantageously be in the form of a composition for caring for the skin in particular body, legs or the face, as anti-aging products, self-tanning products, suncare products, compositions for slimming, compositions for modulating the pigmentation.

According to one embodiment, a composition of the invention may advantageously be in the form of a composition for making up the skin and especially face, eyeslids or around the eyes. It may thus be a face lighter, a tinted BB ("Blamish Balm") gel, an eye lighter.

According to another embodiment, a composition of the invention may advantageously be in the form of a lipcare product, gloss.

Such compositions are especially prepared according to the general knowledge of a person skilled in the art.
Throughout the description, including the claims, the term "comprising a" should be understood as being synonymous with "comprising at least one", unless otherwise specified.
The expressions "between ... and ..." and "ranging from ... to ..." should be understood as meaning limits included, unless otherwise specified.
The invention is illustrated in greater detail by the examples. Unless otherwise mentioned, the amounts indicated are expressed as weight percentages.

**EXEMPLES**

Reference is now made to the following examples, which together with the above descriptions illustrate some embodiments of the invention in a non limiting fashion.

**MATERIALS AND METHODS**

- Ethyl acetate was obtained from Gadot, Israel.
- Magnesium stearate was obtained from FACI ASIA PACIFIC PTE Ltd.
- Titanium oxide, which is also referred to herein throughout as titanium dioxide or $TiO_2$ RC402, was obtained from Sachtleben Chemie GmbH.
- Bismuth oxychloride pre-dispersed 2-ethylhexyl hydroxystearate (marketed as Timiron® Liquid Silver) was obtained from Merck KGaA, Darmstadt, Germany.
- Polyvinyl alcohol (PVA) as used was Mowiol 4-88, KSE solution 4 %; Kuraray America, Inc., USA.
- Cellulose acetate 398-10NF was obtained from Eastman, USA.
- (Poly(ethyl acrylate-co-methyl methacrylate-co-trimethylammonium ethyl methacrylate chloride), EUDRAGIT® RS PO, was obtained from Evonik industries, Germany).
- Size distribution of the microcapsules was determined using HORIBA LA300. Loose Bulk Density of the microcapsules was determined using USP-NF <616>.

**EXAMPLE 1** : **Preparation of PMMA microcapsules containing bismuth oxychloride predispersed 2-ethylhexyl hydroxystearate**

**1.1 Preparation of organic phase/master batch (MB)**

An organic phase (herein referred to interchangeably as "master batch" (MB)) was prepared by gradually adding 10 grams of the wall-forming polymer poly(methyl methacrylate) (PMMA) under stirring (10 minutes), into 300 grams of ethyl acetate, heating the obtained mixture to 50 °C and stirring well until the mixture was homogeneous and transparent (about 20 minutes). The obtained polymer solution was cooled to 25 °C. One gram of Magnesium Stearate (MgSt) was added to the solution under stirring for about 5 minutes. Ten grams of Titanium dioxide (TiO2) were then added to the solution under stirring for about 5 minutes and then the mixture was homogenized for about 8 minutes.
A mixture of bismuth oxychloride predispersed 2-ethylhexyl hydroxystearate (79 grams) was added to the previous suspension under stirring for about 5 minutes.
A list of the components included in the prepared MB is presented in Table 1.

Table 1. Master batch constituents

| Material | Loading for 100 grams MB |
|---|---|
| Poly(methyl methacrylate) | 10.0 |
| $TiO_2$ RC402 | 10.0 |
| Magnesium stearate | 1.0 |
| Bismuth oxychloride dispersed in 2-ethylhexyl hydroxystearate | 79.0 |
| Ethyl acetate | 300.0 |

### 1.2 Preparation of the emulsion

An aqueous solution of 0.25 % polyvinyl alcohol (PVA) was prepared by mixing water (1013 grams) with PVA 4 % solution (68 grams). Ethyl acetate (120 grams) was added to the aqueous solution, and the master batch of step 1.1 above was thereafter gradually added into the ethyl acetate/water emulsion under stirring at about 400 RPM for 2 minutes. The ratio between the master batch and the emulsion (w/w) was 1/3. A list of the components included in the prepared emulsion is presented in Table 2.

Table 2. Emulsion constituents

| Material | Loading (grams) |
|---|---|
| Water | 1013 |
| PVA (4 % solution) | 68 |
| Ethyl Acetate | 120 |
| MB | 400 |

### 1.3 Extraction of the organic solvent

The extraction solution was composed of a mixture of 8775 grams water and 225 grams of PVA solution 4 % (final concentration of PVA in the extraction solution was 0.10 % PVA). The emulsion of step 1.2 above (1600 grams) was gradually added into the extraction solution in a 15 L pail under stirring at 150 RPM using a manual pump, and the obtained mixture was further stirred for additional 15 minutes. The resulting mixture was left to sediment for about 24 hours at 25 °C. A list of the components included in extraction medium is presented in Table 3.

Table 3. Extraction medium constituents

| Material | Loading (grams) |
|---|---|
| Emulsion | 1600 |
| Water | 8775 |
| 4 % PVA solution | 225 |

### 1.4 Washing, Drying and Sifting of the microcapsules

The microcapsules obtained in step 1.3 above were separated either by sedimentation or vacuum filtration, and then dried and sifted.

In the sedimentation procedure, the upper liquid phase from the pail was decanted and the remaining suspension was shaken and transferred to a drying vessel.

In the filtration procedure, the upper phase liquid was decanted from the pail, the remaining suspension was shaken and then filtered, and the sediment was rinsed on the filter with 400 ml water. The suspension was transferred to a drying vessel. In the drying stage, the microcapsules were freeze dried (lyophilized) for 48 hours.

In the sifting stage, the dried microcapsules were sifted using automatic sifter "Ari j-Levy", Sifter MIC. 100. The sifted microcapsules were stored in an appropriate container at room temperature or in a refrigerator.

## EXAMPLE 2 : Preparation of cellulose acetate microcapsules containing bismuth oxychloride predispersed 2-ethylhexyl hydroxystearate

### 2.1 Preparation of organic phase/master batch (MB) stage

An organic phase (herein referred to interchangeably as "master batch" (MB)) was prepared by gradually adding 5 grams of the wall-forming polymer Cellulose Acetate 398-10NF (CA) under stirring (10 minutes), into 300 grams of ethyl acetate, and stirring the obtained mixture until the mixture was homogeneous and transparent

(about 20 minutes). Thirty grams of Titanium dioxide (TiO2) were then added to the obtained solution under stirring for about 5 minutes and then the mixture was homogenized for about 8 minutes. A mixture of bismuth oxychloride predispersed in 2-ethylhexyl hydroxystearate (60 grams) was thereafter added to the suspension under stirring for about 5 minutes.

A list of the components included in the prepared MB is presented in Table 4.

Table 4. Master batch constituents

| **Material** | **Loading for 100 grams MB** |
|---|---|
| Cellulose acetate 398-10NF | 5.0 |
| $TiO_2$ RC402 | 35.0 |
| Bismuth oxychloride dispersed 2-ethylhexyl hydroxystearate | 60.0 |
| Ethyl acetate | 300.0 |

**2.2 Preparation of the emulsion**

An aqueous solution of 0.4 % polyvinyl alcohol (PVA) was prepared by mixing water (972 grams) with PVA 4 % solution (108 grams). Ethyl acetate (120 grams) was added to the aqueous phase, and the master batch of step 3.1 above was thereafter gradually added into the ethyl acetate/water emulsion under stirring at about 400 RPM for 2 minutes. The ratio between the master batch and the emulsion (w/w) was 1/3. A list of the components included in the prepared emulsion is presented in Table 5.

Table 5. Emulsion constituents

| **Material** | **Loading (grams)** |
|---|---|
| Water | 972 |
| PVA (4 % solution) | 108 |
| Ethyl Acetate | 120 |
| MB | 400 |

**2.3 Extraction of the organic solvent**

The extraction solution was composed of a mixture of 8550 grams water and 450 grams of PVA solution 4 % (final concentration of PVA in the extraction fluid 0.20 % PVA). The emulsion of step 3.2 above (1600 grams) was gradually added to the extraction solution in a 15 L pail under stirring at 150 RPM using a manual pump, and the obtained mixture was further stirred for additional 15 minutes. The resulting mixture was left to sediment for about 24 hours at 25 °C. A list of the components included in the prepared extraction medium is presented in Table 6.

Table 6. Extraction medium constituents

| Material | Loading (grams) |
|---|---|
| Emulsion | 1600 |
| Water | 8550 |
| 4 % PVA solution | 450 |

**2.4 Washing, Drying and Sifting of the microcapsules**

The microcapsules obtained in step 3.3 above were separated either by sedimentation or vacuum filtration, dried and sifted, as described hereinabove, for Example 1.

**EXAMPLE 3** : **Preparation of EUDRAGIT® microcapsules containing bismuth oxychloride predispersed 2-ethylhexyl hydroxystearate**

**3.1 Preparation of organic phase/master batch (MB) stage**

An organic phase (herein referred to interchangeably as "master batch" (MB)) was prepared by gradually adding 10 grams of the wall-forming Poly(ethyl acrylate-co-methyl methacrylate-co-trimethylammonioethyl methacrylate chloride) (EUDRAGIT® RS PO) under stirring (10 minutes), into 300.0 grams of ethyl acetate, heating to 50 °C and stirring well until the mixture was homogeneous and transparent (about 20 minutes). The obtained polymer solution was cooled to 25 °C. One gram of Magnesium Stearate (MgSt) was added to the solution under stirring for about 5 minutes. Then, bismuth oxychloride predispersed 2-ethylhexyl hydroxystearate (79 grams) was added to the suspension under stirring for about 5 minutes. The components of the MB are presented in Table 7.

Table 7. Master batch constituents

| **Material** | **Loading for 100 grams MB** |
|---|---|
| EUDRAGIT® RS PO (Poly(ethyl acrylate-co-methyl methacrylate-co-trimethylammonioethyl methacrylate chloride) | 10.0 |
| Magnesium Stearate | 1.0 |
| Bismuth oxychloride dispersed in 2-ethylhexyl hydroxystearate | 79.0 |
| Ethyl acetate | 233 |

### 3.2 Preparation of the emulsion

An aqueous solution of 0.25 % polyvinyl alcohol (PVA) was prepared by mixing water (844 grams) with PVA 4 % solution (56 grams). Ethyl acetate (100 grams) was added to the water phase. Ten grams of Titanium dioxide (TiO2) was added to previous step under stirring for about 5 minutes and then the mixture was homogenized for about 8 minutes and then the master batch of step 4.1 above was gradually added into the ethyl acetate/water emulsion under stirring at about 400 RPM for 2 minutes. The ratio between the master batch and the emulsion (w/w) was 1/3. The components of the emulsion are presented in Table 8.

Table 8. Emulsion constituents

| **Material** | **Loading (grams)** |
|---|---|
| Water | 844 |
| PVA (4 % solution) | 56 |
| Ethyl Acetate | 100 |
| $TiO_2$ RC402 | 10 |
| MB | 323 |

### 3.3 Extraction of the organic solvent

The extraction fluid was composed of a mixture of 6923 grams water and 178 grams of PVA solution 4 % (final concentration of PVA in the extraction fluid 0.10 % PVA). The emulsion of step 4.2 above (1333 grams) was gradually added into the extraction fluid in a 15 L pail under stirring at 150 RPM using a manual pump, and was further stirred for additional 15 minutes. The resulting mixture was left to sediment for about 24 hours at 25 °C. The components of the extraction medium are presented in Table 9.

Table 9. Extraction medium constituents

| **Material** | **Loading (grams)** |
|---|---|
| Emulsion | 1333 |
| Water | 6923 |
| 4 % PVA solution | 178 |

### 3.4 Washing, Drying and Sifting of the microcapsules

The microcapsules obtained in step 3.3 above were separated either by sedimentation or vacuum filtration, dried and sifted, as described hereinabove, for Example 1.

## EXAMPLE 4 : Preparation of PMMA/MA microcapsules containing bismuth oxychloride predispersed 2-ethylhexyl hydroxystearate

### 4.1 Preparation of organic phase/master batch (MB) stage

An organic phase (herein referred to interchangeably as "master batch" (MB)) was prepared by gradually adding 10 grams of the wall-forming polymer Poly(methacrylic acid-co-methyl methacrylate) (PMMA/MA) under stirring (10 minutes), into 300.0 grams of ethyl acetate, heating to 50 °C and stirring well until the mixture was homogeneous and transparent (about 20 minutes). The obtained polymer solution was cooled to 25 °C. One gram of Magnesium Stearate (MgSt) was added to the solution under stirring for about 5 minutes. Ten grams of Titanium dioxide (TiO2) were thereafter added under stirring for about 5 minutes and then the mixture was homogenized for about 8 minutes. Thereafter, bismuth oxychloride predispersed 2-ethylhexyl hydroxystearate (79 grams) was added to the suspension under stirring for about 5 minutes. The components of the MB are presented in Table 10.

Table 10. Master batch constituents

| Material | Loading for 100 grams MB |
|---|---|
| Poly(methacrylic acid-co-methyl methacrylate) (PMMA/MA) | 10.0 |
| $TiO_2$ RC402 | 10.0 |
| Magnesium stearate | 1.0 |
| Bismuth oxychloride dispersed in 2-ethylhexyl hydroxystearate | 79.0 |
| Ethyl acetate | 300.0 |

### 4.2 Preparation of the emulsion

An aqueous solution of 0.25 % polyvinyl alcohol (PVA) was prepared by mixing water (1013 grams) with PVA 4 % solution (68 grams). Ethyl acetate (120 grams) was added to the water phase, and then the master batch of step 1.1 above was gradually added into the ethyl acetate/water emulsion under stirring at about 400 RPM for 2 minutes. The ratio between the master batch and the emulsion (w/w) was 1/3. The components of the emulsion are presented in Table 11.

Table 11. Emulsion constituents

| Material | Loading (grams) |
|---|---|
| Water | 1013 |
| PVA (KSE 4 % solution) | 68 |
| Ethyl Acetate | 120 |
| MB | 400 |

### 4.3 Extraction of the organic solvent

The extraction fluid was composed of a mixture of 8775 grams water and 225 grams of PVA solution 4 % (final concentration of PVA in the extraction fluid 0.10 % PVA). The emulsion of step 1.2 above (1600 grams) was gradually added into the extraction fluid in a 15 L pail under stirring at 150 RPM using a manual pump, and was further stirred for additional 15 minutes. The resulting mixture was left to sediment for about 24 hours at 25 °C. The components of the extraction medium are presented in Table 12.

Table 12. Extraction medium constituents

| Material | Loading (grams) |
|---|---|
| Emulsion | 1600 |
| Water | 8775 |
| 4 % PVA solution | 225 |

### 4.4 Washing, Drying and Sifting of the microcapsules

The microcapsules obtained in step 4.3 above were separated either by sedimentation or vacuum filtration, dried and sifted, as described hereinabove, for Example 1.

**EXAMPLE 5** : Preparation of EUDRAGIT® microcapsules containing bismuth oxychloride predispersed 2-ethylhexyl hydroxystearate

### 5.1 Preparation of organic phase/master batch (MB) stage

An organic phase (herein referred to interchangeably as "master batch" (MB)) was prepared by gradually adding 10 grams of the wall-forming polymer Poly(ethyl acrylate-co-methyl methacrylate-co-trimethylammonioethyl methacrylate chloride) (EUDRAGIT® RS PO) under stirring (10 minutes), into 185.7 grams of ethyl acetate, heating to 50 °C and stirring well until the mixture was homogeneous and transparent (about 20 minutes). The obtained polymer solution was cooled to 25 °C. Ten grams of Triethyl Citrate were added to the solution under stirring for about 5 minutes. Eighty grams of bismuth oxychloride (BiClO) were thereafter added to the mixture under stirring for about 5 minutes and then the mixture was homogenized for about 8 minutes. The components of the MB are presented in Table 13.

Table 13. Master batch constituents

| **Material** | **Loading for 100 grams MB** |
|---|---|
| EUDRAGIT® RS PO (Poly(ethyl acrylate-co-methyl methacrylate-co-trimethyl ammonioethyl methacrylate chloride) | 10.0 |
| Triethyl Citrate | 10.0 |
| Bismuth oxychloride dispersed in 2-ethylhexyl hydroxystearate | 80.0 |
| Ethyl acetate | 185.7 |

### 5.2 Preparation of the emulsion

An aqueous solution of 0.25 % polyvinyl alcohol (PVA) was prepared by mixing water (723.2 grams) with PVA 4 % solution (48.2 grams). Ethyl acetate (85.7 grams) was added to the water phase, and then the master batch of step 6.1 above was gradually added into the ethyl acetate/water emulsion under stirring at about 400 RPM for 10 minutes. The ratio between the master batch and the emulsion (w/w) was 1:3. The components of the emulsion are presented in Table 14.

Table 14. Emulsion constituents

| Material | Loading (grams) |
|---|---|
| Water | 723.2 |
| PVA (4 % solution) | 48.2 |
| Ethyl Acetate | 85.7 |
| MB | 285.7 |

### 5.3 Extraction of the organic solvent

The extraction fluid was composed of a mixture of 5599 grams water and 144 grams of PVA solution 4 % (final concentration of PVA in the extraction fluid 0.10 % PVA). The emulsion of step 6.2 above (1449.2 grams) was gradually added into the extraction fluid in a 15 L pail under stirring at 150 RPM using a manual pump, and was further stirred for additional 15 minutes. The resulting mixture was left to sediment for about 24 hours at 25 °C. The components of the extraction medium are presented in Table 15.

Table 15. Extraction medium constituents

| Material | Loading (grams) |
|---|---|
| Emulsion | 1449.2 |
| Water | 5599 |
| 4 % PVA solution | 144 |

### 5.4 Washing, Drying and Sifting of the microcapsules

The microcapsules obtained in step 5.3 above were separated either by sedimentation or vacuum filtration, dried and sifted, as described hereinabove, for Example 1.

### EXAMPLE 6 : Characterization

**Size Distribution:**

The size distribution of the microcapsules obtained in Examples 1-5 was measured and the obtained data are indicated below.

Herein throughout, a "mean" diameter means an average size of the microcapsules. The size of the microcapsules may be measured by a Laser distribution size method and particularly by measuring the values D[50] and D[90]. D50 means the size of which 50 % of the microcapsules do not exceed, and D90 means the size of which 90 % of the microcapsules do not exceed.

The diameter of the microcapsules obtains as described in Example 1 is in the range of from about 3 microns to about 600 microns, with the mean diameter being about 175 microns, the D50 of the microcapsules being about 155 microns, and the D90 of the microcapsules being about 320 microns.

The diameter of the microcapsules obtains as described in Example 3 is in the range of from about 3 microns to about 500 microns, with the mean diameter being about 120 microns, the D50 of the microcapsules being about 96 microns, and the D90 of the microcapsules being about 237 microns.

The diameter of the microcapsules obtains as described in Example 4 is in the range of from about 3 microns to about 400 microns, with the mean diameter being about 120 microns, the D50 of the microcapsules being about 106 microns, and the D90 of the microcapsules being about 195 microns.

The diameter of the microcapsules obtains as described in Example 5 is in the range of from about 3 microns to about 250 microns, with the mean diameter being about 120 microns, the D50 of the microcapsules being about 96 microns, and the D90 of the microcapsules being about 237 microns.

**Loose Bulk Density:**

The loose bulk density of the microcapsules obtained in Example 1 was determined as ranging from about 300 to about 450 grams/liter (from about 0.30 to about 0.45 gram/$cm^3$), or from about 300 to about 380 grams/liter (from about 0.30 to about 0.38 gram/$cm^3$) or from about 300 to about 340 grams/liter (from about 0.30 to about 0.4 gram/$cm^3$).

The loose bulk density of the microcapsules obtained in Example 2 was determined as ranging from about 360 to about 460 grams/liter (from about 0.36 to about 0.46 gram/$cm^3$), or from about 380 to 440 grams/liter (from about 0.38 to about 0.44 gram/$cm^3$), or from about 400 to 420 grams/liter (from about 0.40 to about 0.42 gram/$cm^3$).

The loose bulk density of the microcapsules obtained in Example 3 was determined as ranging from about 140 to about 360 grams/liter (from about 0.14 to about 0.36 gram/$cm^3$), or from about 200 to 300 grams/liter (from about 0.20 to about 0.30 gram/$cm^3$), or from about 240 to about 260 grams/liter (from about 0.24 to about 0.26 gram/$cm^3$).

The loose bulk density of the microcapsules obtained in Example 5 was determined as ranging from about 420 to about 560 grams/liter (from about 0.42 to about 0.56 gram/$cm^3$), or from about 450 to about 530 grams/liter (from about 0.45 to about 0.53 gram/$cm^3$), or from about 480 to about 500 grams/liter (from about 0.48 to about 0.50 gram/$cm^3$).

**Masking:**

Quantitative measurements of the masking effect provided by encapsulating bismuth oxychloride, the X-Rite measurement technique using the CIE Color Systems (based on the CIE L*a*b* color scale, wherein L* defines lightness, a* denotes the red/green value and b* the yellow/blue value) was used. The standard illuminant applied for these measurements was daylight.

Quantitative values were obtained by integrating values/data measured for three visual elements of color: hue (namely, how we perceive an object's color), chroma (the vividness or dullness of a color namely, how close the color is to either gray or the pure hue), and degree of lightness (namely classifying whether a color is light or dark).

Table 16 below presents the shift in lightness on the lightness scale L* of the present microcapsules relative to the bismuth oxychloride-containing raw material Timiron® Liquid Silver (DL*). The positive DL* values presented in Table XXX denote a shift on the lightness scale in the direction of substantially lighter, brighter color for the microcapsules of the invention compared to the raw material, which is indicative of the masking effect.

Table 16

| Example No. | DL* relative to Timiron® Liquid Silver Raw material |
|---|---|
| 1 | 8.45 |
| 2 | 5.71 |
| 3 | 9.96 |
| 5 | 13.47 |

**Light Reflectance:**

Light reflectance is measured using polarized goniophotometer system with a halogen lamp. Both input and detected light are polarized. The incident light angle is 45° and a convergent angle ranges over 20-75°, with a moving detector. The detection polarizer can be rotated to collect either parallel or perpendicular polarized light. Each quantity of light can be calculated from the quantity of parallel filtered and vertically filtered light.

The quantity of internally reflected light:

$$Iinternally\ reflected\ light = 2 \times Ivertical$$

The quantity of surface-reflected light:

$$Isurface\text{-}reflected\ light = Iparallel - Ivertical$$

The quantity of totally reflected light:

$$Itotal = Iinternally + Isurface = Iparallel + Ivertical$$

Icrossed: the quantity of light passing through the crossed polarized filters.

Iparallel: the quantity of light passing through the parallel polarized filters.

**Exemples 6A and 6B : Aqueous gels**

| Phase | Ingredients | Example 6A (comparative) | Example 6B (invention) |
|---|---|---|---|
| A | WATER | qsp 100 | qsp 100 |
| | GLYCERIN | 7 | 7 |
| | PHENOXYETHANOL | 1,2 | 1,2 |

(continued)

| Phase | Ingredients | Example 6A (comparative) | Example 6B (invention) |
|---|---|---|---|
| B | MYRETH-3 MYRISTATE | 2 | 2 |
| | OCTYLDODECANOL | 1,5 | 1,5 |
| | GLYCERYL STEARATE (and) PEG-100 STEARATE | 1,7 | 1,7 |
| | ISOPROPYL PALMITATE | 5 | 5 |
| | SYNTHETIC WAX | 1 | 1 |
| | CETYL ALCOHOL | 2 | 2 |
| | STEARYL ALCOHOL | 0,5 | 0,5 |
| C | WATER | qs | qs |
| D | CARBOMER | 0,5 | 0,5 |
| | DIMETHICONE | 1,5 | 1,5 |
| E | WATER | 0,7 | 0,7 |
| | SODIUM HYDROXIDE | 0,085 | 0,085 |
| F | DIMETHICONE (and) DIMETHICONE/VINYL DIMETHICONE CROSSPOLYMER KSG-16" by the company Shin Etsu | 2 | 2 |
| G | ALCOHOL DENAT. | 3 | 3 |
| | BISMUTH OXYCHLORIDE DISPERSION IN ETHYLHEXYL HYDROXYSTEARATE (TIMIRON LIQUID SILVER®-MERCK) | 6 | 0 |
| | ENCAPSULATED BISMUTH OXYCHLORIDE OILY DISPERSION OF EXAMPLE 1 | | 6 |

**PROCESS OF PREPARATION :**

The components of the phase B were weighed into a 500ml beaker and heated to 80°C. Meanwhile, the phase A was brought to the boil then added in the phase B. The resulting mixture was stirred with a Morritz to be cooled to 65°C.. In a 1 liter beaker, the phase D was weighed and stirred (Rayneri)., then added into the previous preparation. In a capsule, the phase E was weighed then added in the previous preparation in order to reach a pH closed to 5,3. The phase C was added in order to cool down under stirring until 55°C. Then added successively F and G were previously dispersed in a separated capsule. The resulting mixture was stirried and maintained for 5 minutes (Raynerie). The quantities of the ingredients are expressed in percentages by weight relative to the total weight of the composition.

**EVALUATION :**

The example 6A, contrarily to the example 6B according to the invention, presented a non homogeneous visual aspect. There was a lot of grey spots. the products presented a lot of volutes and the color homogeneity was not sufficient. It is not acceptable compared to the example 6B which show a good stability, good color transformation and homogeneity.

## Claims

**1.** Composition under the form of aqueous gel for caring for and/or making up keratin materials comprising, in a physiologically acceptable medium,

a) one aqueous phase; and
b) at least one hydrophilic gelling agent, and
c) at least microcapsules having a mean size from 10 μm to 400 μm and comprising:

- an inner core comprising at least a dispersion of bismuth oxychloride in at least one oil chosen from 2-ethylhexyl hydroxystearate, ethylhexyl ethylhexanoate, castor oil, or any combination thereof, and more particularly is 2-ethylhexyl hydroxystearate and
- at least one outer shell formed of a wall-forming polymeric material surrounding the said core, the said outer shell comprising

i) at least one wall-forming polymer, and
ii) optionally at least one plasticizer and/or at least one opaque substance and/:or at least one fatty acid salt.

**2.** Composition according to claims 1, wherein the amount of bismuth oxychloride ranges from 50 % to 90 % by weight, more preferably from 60 % to 80 %, more particularly from 65 % to 75 % by weight of the total weight of the -dispersion.

**3.** Composition according to any one of claims 1 or 2, wherein the weight ratio of the bismuth oxychloride particles to the oil(s) ranges from 1.5/1 to 5/1, more preferably from 1.5/1 to 3/1, particularly from 2/1 to 4/1, and more particularly from 2/1 to 3/1.

**4.** Composition according to any one of claims 1 to 3, wherein the oily dispersion dispersion of bismuth oxychloride is a dispersion of bismuth oxychloride in ethylhexyl hydroxystearate, more particularly a dispersion containing from 68% to 72% by weight of bismuth oxychloride in 28% to 32% by weight of 2-ethylhexyl hydroxystearate relative to the total weight of the dispersion,

**5.** Composition according to any one of claims 1 to 4, wherein the wall-forming polymer forming the outer shell(s) is selected from a polyacrylate, a polymethacrylate, a cellulose ether or ester, or any combination thereof.

**6.** Composition according to claim 5, wherein the wall-forming polymer is selected from poly(methyl methacrylate (PMMA), poly(methyl methacrylate)-co-(methacrylic acid) (PMMA/MA), an acrylate/ammonium methacrylate copolymer, cellulose acetate.

**7.** Composition according to any one of claims 1 to 6, wherein the opaque substance is selected from $TiO_2$, zinc oxide, alumina, boron nitride, talc, mica and any combination thereof and preferably is $TiO_2$.

**8.** Composition according to any one of claims 1 to 7, wherein the fatty acid salt is magnesium stearate.

**9.** Composition according to any one of claims 1 to 8, wherein the microcapsules comprise

- the inner core constituted by the oily dispersion of bismuth oxychloride in an amount within a range of from 20 % to 90 %%, by weight., more preferably from 30 % to 90 % by weight, in particular from 40 % to 90 % by weight, more particularly from 50 % to 90 % by weight, more better from 60 % to 90%% by weight, more advantageously from 70 % to 90 %, by weight., more advantageously from 70 % to 80 %, by weight, more particularly advantageously from 60 % to 80 %, by weight relative to the total weight of the microcapsule.
- the wall-forming polymer(s) of the outer shell is within a range of from 5 % to 30 %, preferably from 5 % to 20 % by weight, more preferably from 5 % to 15 % by weight, particularly from 5 % to 10 % by weight, by weight relative to the total microcapsule weight; and
- optionally, the amount of opaque substance(s) in the outer shell is within a range of from 1 % to 50 % by weight, more preferably from 1 % to 40 % by weight, more particularly from 10 % to 40% by weight, relative to the total weight of the microcapsule and/or
- optionally the fatty acid salt in an amount within a range of from 0.05 % 5 %, by weight, more preferably from 0.1 % to 4.5 % by weight, particularly from 0.2 % to 4 % by weight, more particularly from 0.5 % to 4 % by weight, advantageously from 0.5 % to 3.0 % by weight, more advantageously from 0.75 % to 3.0 % by weight, particularly more advantageously from 1.0 % to 3.0 % by weight, more better from 1.0 % to 2.0 % by weight, and in particular is 1.0 %, by weight, relative to the total microcapsule's weight; and/or
- optionally the plasticizer(s) is within a range of from 0.5 % to about 30 % by weight, preferably from 0.5 % to 20 % by weight, more preferably from 1.0 % to 20 % by weight, particularly from 5 % to 15 % by weight, more particularly from 5 % to 10 % by weight, advantageously is 10 % by weight relative to the total weight of the microcapsule.

**10.** Composition according to any one of claims 1 to 9, wherein the microcapsules are single-layer microcapsules.

**11.** Composition according to any one of claims 1 to 10, wherein the microcapsules are single-layer microcapsules comprising the inner core constituted by bismuth oxychloride dispersed in 2-ethylhexyl hydroxystearate in an amount from 60 to 80% by weight, the outer shell comprises magnesium stearate in an amount within a range of from 1.0 % to 2.0 % by weight, $TiO_2$ in an amount within a range of from 1 % to 20 % by weight, more preferably from 5 % to 15 % by weight, more particularly in an amount of 10 %, by weight, and, as a wall-forming polymer, PMMA, in an amount within a range of from 5 % to 20 % by weight, more preferably in an amount of 10 %, by weight relative to the total weight of the microcapsule.

**12.** Composition according to any one of claims 1 to 10, wherein the microcapsules are single-layer microcapsules comprising the inner core constituted by bismuth oxychloride dispersed in 2-ethylhexyl hydroxystearate in an amount from 60 to 80% by weight, the outer shell does not comprise magnesium stearate, and comprises $TiO_2$ in an amount within a range of 10 % to 50 % by weight, more preferably from 10 % to 40 % by weight, more particularly from 20 % to 40 % by weight, advantageously from 30 % to 40 % by weight, more advantageously in an amount of 25 %, by weight, and, as a wall-forming polymer, ethyl cellulose, in an amount within a range of 1 % to 10 %, more preferably in an amount of 5 %, by weight relative to the total weight of the microcapsule.

**13.** Composition according to any one of claims 1 to 10, wherein the microcapsules are single-layer microcapsules comprising the inner core constituted by bismuth oxychloride dispersed in 2-ethylhexyl hydroxystearate in an amount from 60 to 80% by weight, the outer shell comprises magnesium stearate in an amount within a range of from 1.0 % to 2.0 % by weight, $TiO_2$ in an amount within a range of 1 % to 20 % by weight, preferably from 5 % to 15 % by weight, more preferably in an amount of 10 %, by weight, and, as a wall-forming polymer, poly(ethyl acrylate-co-methyl methacrylate-co-trimethyl ammonium ethyl methacrylate chloride, in an amount within a range of 5 % to 20 % by weight, more preferably in an amount of 10 %, by weight relative to the total weight of the microcapsule.

**14.** Composition according to any one of claims 1 to 10, wherein the microcapsules are single-layer microcapsules comprising the inner core constituted by bismuth oxychloride dispersed in 2-ethylhexyl hydroxystearate n an amount of from 60 to 80% by weight, the outer shell comprises magnesium stearate in an amount within a range of from 1.0 % to 2.0 % by weight, $TiO_2$ in an amount within a range of 1 % to 20 % by weight, more preferably from 5 % to 15 % by weight, more particularly in an amount of 10 %, by weight, and, as a wall-forming polymer, PMMA/MA, in an amount within a range of 5 % to 20 % by weight, more preferably in an amount of 10 %, by weight relative to the total weight of the microcapsule.

**15.** Composition according to any one of claims 1 to 10, wherein the microcapsules are single-layer microcapsules comprising the inner core constituted by bismuth oxychloride dispersed in 2-ethylhexyl hydroxystearate in an amount of from 60 to 80% by weight, the outer shell does not comprise magnesium stearate nor $TiO_2$, and comprises a plasticizer, in an amount within a range of 1 % to 20 % by weight, preferably from 5 % to 15 % by weight, more preferably in an amount of 10 %, by weight, and, as a wall-forming material, poly(ethyl acrylate-co-methyl methacrylate-co-trimethyl ammonium ethyl methacrylate chloride, in an amount within a range of 5 % to 20 %, more preferably in an amount of 10 % by weight relative to the total weight of the microcapsule.

**16.** Composition according to one of the preceding claims, wherein the hydrophilic gelling agent is chosen from synthetic polymeric gelling agents, natural or polymeric gelling agents of natural origin, mixed silicates and pyrogenic silicas, and mixtures thereof.

**17.** Composition according to any one of the preceding claims, which contains at least one coloring agent.

**18.** Composition according to any one of the preceding claims, which contains at least one mono-alcohol comprising from 2 to 8 carbon atoms, preferably ethanol.

**19.** Composition according to any one of the preceding claims, which contains at least one filler.

**20.** Composition according to any one of the preceding claims, which contains at least one non-emulsifying organopolysiloxane elastomer.

**21.** Cosmetic process for caring for and/or making up keratinic materials, comprising application on said keratinic materials in particular on the skin of a composition as defined in any claims 1 to20.

## Patentansprüche

1. Zusammensetzung in Form von wässrigem Gel zum Pflegen und/oder Schminken von Keratinmaterialien, umfassend in einem physiologisch unbedenklichen Medium:

   a) eine wässrige Phase und
   b) mindestens ein hydrophiles Geliermittel und
   c) mindestens Mikrokapseln, die eine mittlere Größe von 10 μm bis 400 μm aufweisen und

      - einen inneren Kern, der mindestens eine Dispersion von Bismutoxidchlorid in mindestens einem Öl, das aus Hydroxystearinsäure-2-ethyl-hexylester, Ethylhexansäureethylhexylester, Rizinusöl oder einer Kombination davon ausgewählt ist und spezieller Hydroxystearinsäure-2-ethyl-hexylester ist, umfasst, und
      - mindestens eine äußere Schale aus einem wandbildenden Polymermaterial, die den Kern umgibt, wobei die äußere Schale

         i) mindestens ein wandbildendes Polymer und
         ii) gegebenenfalls mindestens einen Weichmacher und/oder mindestens eine opake Substanz und/oder mindestens ein Fettsäuresalz
         umfasst, umfassen.

2. Zusammensetzung nach Anspruch 1, wobei die Menge an Bismutoxidchlorid im Bereich von 50 bis 90 Gew.-%, weiter bevorzugt von 60 bis 80 Gew.-%, spezieller von 65 bis 75 Gew.-%, bezogen auf das Gesamtgewicht der Dispersion, liegt.

3. Zusammensetzung nach einem der Ansprüche 1 oder 2, wobei das Gewichtsverhältnis der Bismutoxidchlorid-Teilchen zu Öl(en) im Bereich von 1,5/1 bis 5/1, weiter bevorzugt von 1,5/1 bis 3/1, speziell von 2/1 bis 4/1 und spezieller von 2/1 bis 3/1 liegt.

4. Zusammensetzung nach einem der Ansprüche 1 bis 3, wobei es sich bei der öligen Dispersion von Bismutoxidchlorid um eine Dispersion von Bismutoxidchlorid in Hydroxystearinsäureethylhexylester, spezieller eine Dispersion, die 68 bis 72 Gew.-% Bismutoxidchlorid und 28 bis 32 Gew.-% Hydroxystearinsäure-2-ethylhexylester, bezogen auf das Gesamtgewicht der Dispersion, enthält, handelt.

5. Zusammensetzung nach einem der Ansprüche 1 bis 4, wobei das die äußere Schale bzw. die äußeren Schalen bildende wandbildende Polymer aus einem Polyacrylat, einem Polymethacrylat, einem Celluloseether oder -ester oder einer Kombination davon ausgewählt ist.

6. Zusammensetzung nach Anspruch 5, wobei das wandbildende Polymer aus Poly(methylmethacrylat) (PMMA), Poly(methylmethacrylat)-co-(methacrylsäure) (PMMA/MA), einem Acrylat/Ammoniummethacrylat-Copolymer und Celluloseacetat ausgewählt ist.

7. Zusammensetzung nach einem der Ansprüche 1 bis 6, wobei die opake Substanz aus $TiO_2$, Zinkoxid, Aluminiumoxid, Bornitrid, Talk, Glimmer und einer Kombination davon ausgewählt ist und vorzugsweise $TiO_2$ ist.

8. Zusammensetzung nach einem der Ansprüche 1 bis 7, wobei es sich bei dem Fettsäuresalz um Magnesiumstearat handelt.

9. Zusammensetzung nach einem der Ansprüche 1 bis 8, wobei die Mikrokapseln

   - den inneren Kern, der durch die ölige Dispersion von Bismutoxidchlorid gebildet wird, in einer Menge im Bereich von 20 bis 90 Gew.-%, weiter bevorzugt von 30 bis 90 Gew.-%, speziell von 40 bis 90 Gew.-%, spezieller von 50 bis 90 Gew.-%, besser von 60 bis 90 Gew.-%, vorteilhafter von 70 bis 90 Gew.-%, vorteilhafter von 70 bis 80 Gew.-%, noch vorteilhafter von 60 bis 80 Gew.-%, bezogen auf das Gesamtgewicht der Mikrokapsel, umfassen,
   - wobei das wandbildende Polymer bzw. die wandbildenden Polymere der äußeren Schale in einem Bereich von 5 bis 30 Gew.-%, vorzugsweise von 5 bis 20 Gew.-%, weiter bevorzugt von 5 bis 15 Gew.-%, speziell von 5 bis 10 Gew.-%, bezogen auf das Gesamtgewicht der Mikrokapsel, vorliegt bzw. vorliegen; und
   - gegebenenfalls die Menge an opaker Substanz bzw. opaken Substanzen in der äußeren Schale im Bereich

von 1 bis 50 Gew.-%, weiter bevorzugt von 1 bis 40 Gew.-%, weiter bevorzugt von 10 bis 40 Gew.-%, bezogen auf das Gesamtgewicht der Mikrokapsel, liegt; und/oder

- gegebenenfalls das Fettsäuresalz in einer Menge im Bereich von 0,05 bis 5 Gew.-%, weiter bevorzugt von 0,1 bis 4,5 Gew.-%, speziell von 0,2 bis 4 Gew.-%, spezieller von 0,5 bis 4 Gew.-%, vorteilhaft von 0,5 bis 3 Gew.-%, vorteilhafter von 0,75 bis 3,0 Gew.-%, noch vorteilhafter von 1,0 bis 3,0 Gew.-%, noch besser von 1,0 bis 2,0 Gew.-% und insbesondere von 1,0 Gew.-%, bezogen auf das Gesamtgewicht der Mikrokapsel, vorliegt; und/oder
- gegebenenfalls der Weichmacher bzw. die Weichmacher in einem Bereich von 0,5 bis etwa 30 Gew.-%, vorzugsweise von 0,5 bis 20 Gew.-%, weiter bevorzugt von 1,0 bis 20 Gew.-%, speziell von 5 bis 15 Gew.-%, spezieller von 5 bis 10 Gew.-%, vorteilhaft von 10 Gew.-%, bezogen auf das Gesamtgewicht der Mikrokapsel, vorliegt bzw. vorliegen.

**10.** Zusammensetzung nach einem der Ansprüche 1 bis 9, wobei es sich bei den Mikrokapseln um einschichtige Mikrokapseln handelt.

**11.** Zusammensetzung nach einem der Ansprüche 1 bis 10, wobei es sich bei den Mikrokapseln um einschichtige Mikrokapseln handelt, die den inneren Kern aus in Hydroxystearinsäure-2-ethylhexylester dispergiertem Bismutoxidchlorid in einer Menge von 60 bis 80 Gew.-% umfassen, und die äußere Schale Magnesiumstearat in einer Menge im Bereich von 1,0 bis 2,0 Gew.-%, $TiO_2$ in einer Menge im Bereich von 1 bis 20 Gew.-%, weiter bevorzugt von 5 bis 15 Gew.-%, spezieller in einer Menge von 10 Gew.-%, und als wandbildendes Polymer PMMA in einer Menge im Bereich von 5 bis 20 Gew.-%, weiter bevorzugt in einer Menge von 10 Gew.-%, bezogen auf das Gesamtgewicht der Mikrokapsel, umfasst.

**12.** Zusammensetzung nach einem der Ansprüche 1 bis 10, wobei es sich bei den Mikrokapseln um einschichtige Mikrokapseln handelt, die den inneren Kern aus in Hydroxystearinsäure-2-ethylhexylester dispergiertem Bismutoxidchlorid in einer Menge von 60 bis 80 Gew.-% umfassen, und die äußere Schale kein Magnesiumstearat umfasst und $TiO_2$ in einer Menge im Bereich von 10 bis 50 Gew.-%, weiter bevorzugt von 10 bis 40 Gew.-%, spezieller in einer Menge von 20 bis 40 Gew.-%, vorteilhaft von 30 bis 40 Gew.-%, vorteilhafter in einer Menge von 25 Gew.-%, und als wandbildendes Polymer Methylcellulose in einer Menge im Bereich von 1 bis 10 Gew.-%, weiter bevorzugt in einer Menge von 5 Gew.-%, bezogen auf das Gesamtgewicht der Mikrokapsel, umfasst.

**13.** Zusammensetzung nach einem der Ansprüche 1 bis 10, wobei es sich bei den Mikrokapseln um einschichtige Mikrokapseln handelt, die den inneren Kern aus in Hydroxystearinsäure-2-ethylhexylester dispergiertem Bismutoxidchlorid in einer Menge von 60 bis 80 Gew.-% umfassen, und die äußere Schale Magnesiumstearat in einer Menge im Bereich von 1,0 bis 2,0 Gew.-%, $TiO_2$ in einer Menge im Bereich von 1 bis 20 Gew.-%, bevorzugt von 5 bis 15 Gew.-%, weiter bevorzugt in einer Menge von 10 Gew.-%, und als wandbildendes Polymer Poly(ethylacrylat-co-methylmethacrylat-co-trimethylammoniumethylmethacrylatchlorid) in einer Menge im Bereich von 5 bis 20 Gew.-%, weiter bevorzugt in einer Menge von 10 Gew.-%, bezogen auf das Gesamtgewicht der Mikrokapsel, umfasst.

**14.** Zusammensetzung nach einem der Ansprüche 1 bis 10, wobei es sich bei den Mikrokapseln um einschichtige Mikrokapseln handelt, die den inneren Kern aus in Hydroxystearinsäure-2-ethylhexylester dispergiertem Bismutoxidchlorid in einer Menge von 60 bis 80 Gew.-% umfassen, und die äußere Schale Magnesiumstearat in einer Menge im Bereich von 1,0 bis 2,0 Gew.-%, $TiO_2$ in einer Menge im Bereich von 1 bis 20 Gew.-%, weiter bevorzugt von 5 bis 15 Gew.-%, spezieller in einer Menge von 10 Gew.-%, und als wandbildendes Polymer PMMA/MA in einer Menge im Bereich von 5 bis 20 Gew.-%, weiter bevorzugt in einer Menge von 10 Gew.-%, bezogen auf das Gesamtgewicht der Mikrokapsel, umfasst.

**15.** Zusammensetzung nach einem der Ansprüche 1 bis 10, wobei es sich bei den Mikrokapseln um einschichtige Mikrokapseln handelt, die den inneren Kern aus in Hydroxystearinsäure-2-ethylhexylester dispergiertem Bismutoxidchlorid in einer Menge von 60 bis 80 Gew.-% umfassen, und die äußere Schale weder Magnesiumstearat noch $TiO_2$ umfasst und einen Weichmacher in einer Menge im Bereich von 1 bis 20 Gew.-%, vorzugsweise von 5 bis 15 Gew.-%, weiter bevorzugt in einer Menge von 10 Gew.-%, und als wandbildendes Material Poly(ethylacrylat-co-methylmethacrylat-co-trimethylammoniumethylmethacrylatchlorid) in einer Menge im Bereich von 5 bis 20 Gew.-%, weiter bevorzugt in einer Menge von 10 Gew.-%, bezogen auf das Gesamtgewicht der Mikrokapsel, umfasst.

**16.** Zusammensetzung nach einem der vorhergehenden Ansprüche, wobei das hydrophile Geliermittel aus synthetischen polymeren Geliermitteln, natürlichen oder polymeren Geliermitteln natürlichen Ursprungs, gemischten Silikaten und pyrogenen Kieselsäuren und Mischungen davon ausgewählt ist.

**17.** Zusammensetzung nach einem der vorhergehenden Ansprüche, die mindestens ein Farbmittel enthält.

**18.** Zusammensetzung nach einem der vorhergehenden Ansprüche, die mindestens einen Monoalkohol mit 2 bis 8 Kohlenstoffatomen, vorzugsweise Ethanol, enthält.

**19.** Zusammensetzung nach einem der vorhergehenden Ansprüche, die mindestens einen Füllstoff enthält.

**20.** Zusammensetzung nach einem der vorhergehenden Ansprüche, die mindestens ein nichtemulgierendes Organopolysiloxanelastomer enthält.

**21.** Kosmetisches Verfahren zum Pflegen und/oder Schminken von Keratinmaterialien, bei dem man auf die Keratinmaterialien, insbesondere auf die Haut, eine Zusammensetzung gemäß einem der Ansprüche 1 bis 20 aufbringt.

## Revendications

**1.** Composition sous forme de gel aqueux pour le soin et/ou le maquillage de matériaux de kératine comprenant, dans un milieu physiologiquement acceptable,

a) une phase aqueuse ; et
b) au moins un agent gélifiant hydrophile, et
c) au moins des microcapsules possédant une grosseur moyenne de 10 μm à 400 μm et comprenant :

- un noyau interne comprenant au moins une dispersion d'oxychlorure de bismuth dans au moins une huile choisie parmi l'hydroxystéarate de 2-éthylhexyle, l'éthylhexanoate d'éthylhexyle, l'huile de ricin, ou une quelconque combinaison correspondante, et plus particulièrement étant l'hydroxystéarate de 2-éthylhexyle et
- au moins une enveloppe externe formée d'un matériau polymérique formant paroi entourant ledit noyau, ladite enveloppe externe comprenant

i) au moins un polymère formant paroi, et
ii) éventuellement au moins un plastifiant et/ou au moins une substance opaque et/ou au moins un sel d'acide gras.

**2.** Composition selon la revendication 1, la quantité d'oxychlorure de bismuth se situant dans la plage de 50 % à 90 % en poids, plus préférablement de 60 % à 80 %, plus particulièrement de 65 % à 75 % en poids du poids total de la dispersion.

**3.** Composition selon l'une quelconque des revendications 1 et 2, le rapport pondéral des particules d'oxychlorure de bismuth à l'huile/aux huiles se situant dans la plage de 1,5/1 à 5/1, plus préférablement de 1,5/1 à 3/1, particulièrement de 2/1 à 4/1, et plus particulièrement de 2/1 à 3/1.

**4.** Composition selon l'une quelconque des revendications 1 à 3, la dispersion huileuse d'oxychlorure de bismuth étant une dispersion d'oxychlorure de bismuth dans de l'hydroxystéarate d'éthylhexyle, plus particulièrement une dispersion contenant de 68 % à 72 % en poids d'oxychlorure de bismuth dans 28 % à 32 % en poids d'hydroxystéarate de 2-éthylhexyle par rapport au poids total de la dispersion.

**5.** Composition selon l'une quelconque des revendications 1 à 4, le polymère formant paroi formant l'enveloppe/les enveloppes externe(s) étant choisi parmi un polyacrylate, un polyméthacrylate, un éther ou un ester de cellulose, ou une quelconque combinaison correspondante.

**6.** Composition selon la revendication 5, le polymère formant paroi étant choisi parmi le poly(méthacrylate de méthyle) (PMMA), le poly(méthacrylate de méthyle)-co(acide méthacrylique) (PMMA/MA), un copolymère acrylate/méthacrylate d'ammonium, l'acétate de cellulose.

**7.** Composition selon l'une quelconque des revendications 1 à 6, la substance opaque étant choisie parmi $TiO_2$, l'oxyde de zinc, l'alumine, le nitrure de bore, le talc, le mica et une quelconque combinaison correspondante et préférablement étant $TiO_2$.

**8.** Composition selon l'une quelconque des revendications 1 à 7, le sel d'acide gras étant le stéarate de magnésium.

**9.** Composition selon l'une quelconque des revendications 1 à 8, les microcapsules comprenant

- le noyau interne constitué par la dispersion huileuse d'oxychlorure de bismuth en une quantité dans une plage allant de 20 % à 90 % en poids, plus préférablement de 30 % à 90 % en poids, en particulier de 40 % à 90 % en poids, plus particulièrement de 50 % à 90 % en poids, encore mieux de 60 à 90 % en poids, plus avantageusement de 70 % à 90 % en poids, plus avantageusement de 70 % à 80 % en poids, plus particulièrement avantageusement de 60 % à 80 % en poids, par rapport au poids total de la microcapsule,
- le(s) polymère(s) formant paroi de l'enveloppe externe étant dans une plage allant de 5 % à 30 %, préférablement de 5 % à 20 % en poids, plus préférablement de 5 % à 15 % en poids, particulièrement de 5 % à 10 % en poids, par rapport au poids total de la microcapsule ; et
- éventuellement, la quantité de substance(s) opaque(s) dans l'enveloppe externe étant dans une plage allant de 1 % à 50 % en poids, plus préférablement de 1 % à 40 % en poids, plus particulièrement de 10 % à 40 % en poids, par rapport au poids total de la microcapsule et/ou
- éventuellement le sel d'acide gras en une quantité dans une plage allant de 0,05 à 5 % en poids, plus préférablement de 0,1 % à 4,5 % en poids, particulièrement de 0,2 % à 4 % en poids, plus particulièrement de 0,5 % à 4 % en poids, avantageusement de 0,5 % à 3,0 % en poids, plus avantageusement de 0,75 % à 3,0 % en poids, particulièrement plus avantageusement de 1,0 % à 3,0 % en poids, encore mieux de 1,0 % à 2,0 % en poids, et en particulier étant de 1,0 % en poids, par rapport au poids total de la microcapsule ; et/ou
- éventuellement le(s) plastifiant(s) étant dans une plage allant de 0,5 % à environ 30 % en poids, préférablement de 0,5 % à 20 % en poids, plus préférablement de 1,0 à 20 % en poids, particulièrement de 5 % à 15 % en poids, plus particulièrement de 5 % à 10 % en poids, avantageusement étant de 10 % en poids par rapport au poids total de la microcapsule.

**10.** Composition selon l'une quelconque des revendications 1 à 9, les microcapsules étant des microcapsules monocouches.

**11.** Composition selon l'une quelconque des revendications 1 à 10, les microcapsules étant des microcapsules monocouches comprenant le noyau interne constitué par de l'oxychlorure de bismuth dispersé dans de l'hydroxystéarate de 2-éthylhexyle en une quantité de 60 à 80 % en poids, l'enveloppe externe comprenant du stéarate de magnésium en une quantité dans une plage allant de 1,0 à 2,0 % en poids, du $TiO_2$ en une quantité dans une plage allant de 1 % à 20 % en poids, plus préférablement de 5 % à 15 % en poids, plus particulièrement en une quantité de 10 % en poids, et, en tant que polymère formant paroi, du PMMA, en une quantité dans une plage allant de 5 % à 20 % en poids, plus préférablement en une quantité de 10 % en poids par rapport au poids total de la microcapsule.

**12.** Composition selon l'une quelconque des revendications 1 à 10, les microcapsules étant des microcapsules monocouches comprenant le noyau interne constitué par de l'oxychlorure de bismuth dispersé dans de l'hydroxystéarate de 2-éthylhexyle en une quantité de 60 à 80 % en poids, l'enveloppe externe ne comprenant pas de stéarate de magnésium, et comprenant du $TiO_2$ en une quantité dans une plage de 10 % à 50 % en poids, plus préférablement de 10 % à 40 % en poids, plus particulièrement de 20 % à 40 % en poids, avantageusement de 30 % à 40 % en poids, plus avantageusement en une quantité de 25 % en poids, et, en tant que polymère formant paroi, de l'éthylcellulose, en une quantité dans une plage de 1 % à 10 %, plus préférablement en une quantité de 5 % en poids par rapport au poids total de la microcapsule.

**13.** Composition selon l'une quelconque des revendications 1 à 10, les microcapsules étant des microcapsules monocouches comprenant le noyau interne constitué par de l'oxychlorure de bismuth dispersé dans de l'hydroxystéarate de 2-éthylhexyle en une quantité de 60 à 80 % en poids, l'enveloppe externe comprenant du stéarate de magnésium en une quantité dans une plage allant de 1,0 à 2,0 % en poids, du $TiO_2$ en une quantité dans une plage allant de 1 % à 20 % en poids, préférablement de 5 % à 15 % en poids, plus préférablement en une quantité de 10 % en poids, et, en tant que polymère formant paroi, du poly(acrylate d'éthyle-co-méthacrylate de méthyle-co-chlorure de méthacrylate d'éthyle-triméthylammonium), en une quantité dans une plage de 5 % à 20 % en poids, plus préférablement en une quantité de 10 % en poids par rapport au poids total de la microcapsule.

**14.** Composition selon l'une quelconque des revendications 1 à 10, les microcapsules étant des microcapsules monocouches comprenant le noyau interne constitué par de l'oxychlorure de bismuth dispersé dans de l'hydroxystéarate de 2-éthylhexyle en une quantité de 60 à 80 % en poids, l'enveloppe externe comprenant du stéarate de magnésium en une quantité dans une plage allant de 1,0 à 2,0 % en poids, du $TiO_2$ en une quantité dans une plage allant de

1 % à 20 % en poids, plus préférablement de 5 % à 15 % en poids, plus particulièrement en une quantité de 10 % en poids, et, en tant que polymère formant paroi, du PMMA/MA, en une quantité dans une plage de 5 % à 20 % en poids, plus préférablement en une quantité de 10 % en poids par rapport au poids total de la microcapsule.

**15.** Composition selon l'une quelconque des revendications 1 à 10, les microcapsules étant des microcapsules monocouches comprenant le noyau interne constitué par de l'oxychlorure de bismuth dispersé dans de l'hydroxystéarate de 2-éthylhexyle en une quantité de 60 à 80 % en poids, l'enveloppe externe ne comprenant ni du stéarate de magnésium ni du $TiO_2$, et comprenant un plastifiant, en une quantité dans une plage de 1 % à 20 % en poids, préférablement de 5 % à 15 % en poids, plus préférablement en une quantité de 10 % en poids, et, en tant que matériau formant paroi, du poly(acrylate d'éthyle-co-méthacrylate de méthyle-co-chlorure de méthacrylate d'éthyle-triméthylammonium), en une quantité dans une plage de 5 % à 20 %, plus préférablement en une quantité de 10 % en poids par rapport au poids total de la microcapsule.

**16.** Composition selon l'une des revendications précédentes, l'agent gélifiant hydrophile étant choisi parmi des agents gélifiants polymériques synthétiques, des agents gélifiants naturels ou polymériques d'origine naturelle, des silicates mixtes et des silices pyrogéniques, et des mélanges correspondants.

**17.** Composition selon l'une quelconque des revendications précédentes, qui contient au moins un agent colorant.

**18.** Composition selon l'une quelconque des revendications précédentes, qui contient au moins un monoalcool comprenant de 2 à 8 atomes de carbone, préférablement de l'éthanol.

**19.** Composition selon l'une quelconque des revendications précédentes, qui contient au moins une charge.

**20.** Composition selon l'une quelconque des revendications précédentes, qui contient au moins un élastomère d'organopolysiloxane non émulsifiant.

**21.** Procédé cosmétique pour le soin et/ou le maquillage de matériaux kératiniques, comprenant l'application sur lesdits matériaux kératiniques en particulier sur la peau d'une composition telle que définie selon l'une quelconque des revendications 1 à 20.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004041234 A
- US 703361 A
- US 2014356403 A
- US 20120269752 A
- US 2010095868 A
- US 7622132 B
- WO 09079135 A
- EP 1518903 B1
- US 5320835 A
- US 5382433 A
- WO 985002 A
- US 5380485 A
- US 20050031558 A
- US 20050276774 A
- US 4756906 A
- WO 2004075679 A
- US 6932984 B
- US 7838037 B
- WO 2009138978 A
- EP 0955039 A
- US 2004175338 A
- WO 2012156965 A
- US 3301848 A
- US 3589578 A
- US 4031307 A
- EP 0216479 A
- US 3915921 A
- US 4509949 A
- WO 8781121 B
- WO 9492103 A
- WO 9844012 A
- EP 0815928 B1
- JP 09188830 A
- JP 10158450 A
- JP 10158541 A
- JP 07258460 A
- JP 05017710 A
- EP 1086683 A
- US 5002698 A
- EP 295886 A
- EP 242219 A
- EP 285886 A
- EP 765656 A
- JP 61194009 A
- US 5538793 A


### Non-patent literature cited in the description


- **C.M. HANSEN.** The three dimensional solubility parameters. *J. Paint Technol.,* 1967, vol. 39, 105
- Kirk-Othmer Encyclopedia of Chemical TECHNOLOGY. Wiley Interscience, 1983, vol. 21, 492-507
- **G. FONNUM ; J. BAKKE ; FK. HANSEN.** *Colloid Polym. Sci,* 1993, vol. 271, 380-389
- *The Journal of the American Chemical Society,* February 1938, vol. 60, 309